# EUROPEAN PATENT SPECIFICATION

(11) **EP 2 049 681 B1**
(45) Date of publication and mention of the grant of the patent: **21.09.2016**
(21) Application number: 07765247.7
(22) Date of filing: 23.07.2007
(51) Int. Cl.: C12Q 1/68

(54) **METHODS FOR ANALYSES OF CELLULAR PROLIFERATIVE DISORDERS**
VERFAHREN FÜR DIE ANALYSE VON ZELLULÄREN PROLIFERATIVEN KRANKHEITEN
PROCÉDÉS POUR ANALYSER DES TROUBLES DE PROLIFÉRATION CELLULAIRE

(30) Priority: 21.07.2006 US 832509 P; 20.10.2006 US 853097 P; 24.10.2006 US 854324 P; 10.11.2006 EP 06123802
(43) Date of publication of application: 22.04.2009
(73) Proprietor: Epigenomics AG, 10829 Berlin (DE)
(72) Inventor: TETZNER, Reimo, 10439 Berlin (DE); SLEDZIEWSKI, Andrew, Shoreline, WA 98177 (US); LOFTON-DAY, Catherine, Seattle, WA 98103 (US); DISTLER, Jürgen, 12163 Berlin (DE); MODEL, Fabian, 12683 Berlin (DE); PAYNE, Shannon, Seattle, WA 98117 (US)
(74) Representative: Zwicker, Jörk
(86) International application number: PCT/EP2007/006538
(87) International publication number: WO 2008/009478

(56) References cited:
- CHUNG WOONBOK ET AL: "Identification of novel hypermethylated genes in primary prostate cancer and colon" PROCEEDINGS OF THE AMERICAN ASSOCIATION FOR CANCER RESEARCH ANNUAL MEETING, vol. 47, April 2006 (2006-04), page 14, XP001536665 & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- CHUNG WOONBOK ET AL: "Identification of differentiatly methylated sequences in prostate cancer." PROCEEDINGS OF THE AMERICAN ASSOCIATION FOR CANCER RESEARCH ANNUAL MEETING, vol. 46, April 2005 (2005-04), page 214, XP001536666 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- TUMMALA R ET AL: "Molecular cloning and characterization of AP-2epsilon, a fifth member of the AP-2 family" GENE: AN INTERNATIONAL JOURNAL ON GENES AND GENOMES, ELSEVIER, AMSTERDAM, NL, vol. 321, 4 December 2003 (2003-12-04), pages 93-102, XP004474274 ISSN: 0378-1119
- ECKERT DAWID ET AL: "The AP-2 family of transcription factors." GENOME BIOLOGY 2005, vol. 6, no. 13, 2005, page 246, XP009089197 ISSN: 1465-6914

### Sequence Listing

<110> EPIGENOMICS AG
<120> METHODS AND NUCLEIC ACIDS FOR ANALYSES OF CELLULAR PROLIFERATIVE DISORDERS
<130> E30940PCT
<160> 102
<210> 1
   <211> 24959
   <212> DNA
   <213> Homo Sapiens
   <400> 1
<210> 2
   <211> 6096
   <212> DNA
   <213> Homo Sapiens
   <400> 2
   taccagtgta agattcaaaa ttcccttttt gcactgcaca gtgagatgcc cagggctcca 60
   gctcagtgcc tggacatagc gattcctggg cctgcccgtc gccgccccaa gcgaagctgg     120
   tgcgccttgg gcggagcaga cagagaccct gggtggcagg ggcttgggaa gacatgggcg     180
   gctagggctt tatgcgccct caccgctgcc ctctgctatt tgcaggcaat ggacgagccg     240
   ggaatgagcc tcctagacca gtccgtgatc aagaaaggta aggaatggtc tgtcagggca     300
   gagcccggcg agatggtgca ggcccttggt gcacagatcc attttcttca ccggccgtgc     360
   ctcctgtgtg tcgccaggct gggtgtccac caggcactct tcctggccca gccagatgtt     420
   aggcagacgt gcgggcttgg tgagtttgcc cagcaccctg tggcctgggg tgggcctcag     480
   cggatcagca ttcactgggc tgcagcactg ggagcctggc ctctccccgc cgagggggag     540
   ggcactcttg tggatctgga gttgatttgc agaacgagtt aaaccacttc cctgtttccc     600
   taagagatgg gaatggaagt gctgttccca cggagttggg gaaatgattt tcactttaca     660
   gtgccttagc atttcggtgc ctggcgggca ctttcttcct cttccttcca ggcagggcct     720
   tggaggcctc tgggggaatt ttctttctgt gggagtctct tgcggcattt agacttaggg     780
   gagcttgtgt gtgagtactg tgtgttaggc tgtgtgcacc tgagtcaggg cccacctgct     840
   cctgggtgtc tgtgtccatg tgagttcagg gtcctgtgca tgtctgaaat gttcccttca     900
   tgggtgtctt agtatttctt ggagtgtgag tgtgtctgtt tctgtgaatg tgtttgtgag     960
   gtgtgtctct gtatgttggt gtgcatttct ctgcatttgg gggatgtaca catttctcaa    1020
   tatgtacagt atctctgttg tgtcctgcac tttgttcttt ggtatctgag gatttccaag    1080
   catgcgcggg ccctctctgt gtatatatag gagtatttat gtgactcctg gcattagtaa    1140
   aatccaggga cacgggatcc accttttctg gcctgaggac caagtactgg ccatgacagg    1200
   ggaaggtgag agacgacaaa aacagagaga cagccagaga ggagcagaga gtcagagggg    1260
   cccaggcatt gggtagcagc ctctttacat ttggggcagg tgcccgaaag aattcagagg    1320
   tgcacatgag cctgaggtgc cccaggcagg cactgctccc acagggtttg gcttgagttg    1380
   tttttcaaac gagtgaattc aagcctgggc tctatttgcc ctccacttgt tctcagggga    1440
   ggccaaggtg gaagtggtgg tagcagggct ggggctggac ttccaggagc tggggctgag    1500
   ttaccaggag ctgggggttg ggtggatgac ttggagtgtg tagcagggaa gatgaggcaa    1560
   cagggcagga agtgggtggg gggaggtgga attggggctg tgtcctgtgt cgcttggaac    1620
   tgggagtgtg ggaaagacac taggaacctg gttgcagcgc agctctgctg gtggggcttg    1680
   gttggcttac tgtacagagc ctttcttgac ccctgaagaa agagatccgt ctgcagtggg    1740
   caaaagcctg cctggacttc ctggccacca gaaatatgag catggtggtg gtccccagtt    1800
   ccctattcat gcttgggctc aagagactgg gagtctaggt tcactgactc cctgagaaag    1860
   actaagaccc tgcattttag aaagaggttt ggggatctct gccctgcgca agggtagaag    1920
   gatcagctgt tcctctgagc accttaaccc ggaaccccgg tccgaagccg agacaggaga    1980
   ctggatgcga ggccctccca gagctggttt ctctcaaaca acttccaaaa ctcctagatc    2040
   ctaggggtac gccgaaatcc cccaaagcag tccaaagaac acaacgagag tcctaacatc    2100
   ccaggtggcg gcgcgctggc tccctggagc ggggcgggac gcggccgcgc ggactcacgt    2160
   gcacaaccgc gcgggacggg gccacgcgga ctcacgtgca caaccgcggg accccagcgc    2220
   cagcgggacc ccagcgccag cgggacccca gcgccagcgg gaccccagcg ccagcgggac    2280
   cccagcgcca gcgggacccc agcgccagcg ggaccccagc gccagcgggt ctgtggccca    2340
   gtggagcgag tggagcgctg gcgacctgag cggagactgc gccctggacg ccccagccta    2400
   gacgtcaagt tacagcccgc gcagcagcag caaaggggaa ggggcaggag ccgggcacag    2460
   ttggatccgg aggtcgtgac ccaggggaaa gcgtgggcgg tcgacccagg gcagctgcgg    2520
   cggcgaggca ggtgggctcc ttgctccctg gagccgcccc tccccacacc tgccctcggc    2580
   gcccccagca gttttcacct tggccctccg cggtcactgc gggattcggc gttgccgcca    2640
   gcccagtggg gagtgaatta gcgccctcct tcgtcctcgg cccttccgac ggcacgagga    2700
   actcctgtcc tgccccacag accttcggcc tccgccgagt gcggtactgg agcctgcccc    2760
   gccagggccc tggaatcaga gaaagtcgct ctttggccac ctgaagcgtc ggatccctac    2820
   agtgcctccc agcctgggcg ggagcggcgg ctgcgtcgct gaaggttggg gtccttggtg    2880
   cgaaagggag gcagctgcag cctcagcccc accccagaag cggccttcgc atcgctgcgg    2940
   tgggcgttct cgggcttcga cttcgccagc gccgcggggc agaggcacct ggagctcgca    3000
   gggcccagac ctgggttgga aaagcttcgc tgactgcagg caagcgtccg ggaggggcgg    3060
   ccaggcgaag ccccggcgct ttaccacaca cttccgggtc ccatgccagt tgcatccgcg    3120
   gtattgggca ggaaatggca gggctgaggc cgaccctagg agtataaggg agccctccat    3180
   ttcctgccca catttgtcac ctccagtttt gcaacctatc ccagacacac agaaagcaag    3240
   caggactggt ggggagacgg agcttaacag gaatattttc cagcagtgag caggggctgt    3300
   atgggacgcg ggaggagctc agaggaggcg cggagagtgc ccgaggttgg gtgagtgcct    3360
   agaggggaga tagttgaacc gggttcaaga ggtgcttagt gggtgtttgt tgaatgaatg    3420
   agtgatgggc tttgaagtct gagtgcattg aaagaggggg tgtgtaaaaa gggctccttt    3480
   catcacacag gacacagcat atgcaaatcc tctccctgtg gaaaagccag acaggttaaa    3540
   aaggttacaa acaaattagc cgggcatggt ggtgcgcgtc tgtagtccca gctactaggg    3600
   aggctgagcc aggggaatcg cttgaacccg ggaggcggag attgcagtga gccaagatcg    3660
   cgccactgca ctccagcctg gaaacagagc gagactccgt ctcggaaaaa aaaaaaaaaa    3720
   gttacaaacc gtgtgtgggt ttcaggttat acaatcagag ctggagggga gtggtcaagg    3780
   atgagaactg agatggatcc ctcgttccct ctggaggaga gtgggtggtt gcctacttgg    3840
   gggtggggaa tccctctcca cgggctcagc tgtccaatct caggggatct ctaggacagg    3900
   agctgatgta aacagtcgcc ctattccttg ctgtctttgg ccctggagaa ggaggaggga    3960
   gctggggagg gtctccactt cccagacaat ctctaagcag ccaggacatg ggtgagatga    4020
   gtgagatact gacttctggg acagaatttg agagggtgcc aaaaaactca gtaatcaaga    4080
   taaataggcc gggcgcagtg gctcacgtct gtaatcccag cactttggga ggccggatca    4140
   cttgaggtca agagttcgag accagcctgg ccaagatggt gaaaccccat ctctactaaa    4200
   aatacaaaaa ttagcccagt gtggtggcgc tagcctgtaa tcccagccac tcaggaggct    4260
   gaggcaagag aattgcttga cccaggaggc agaggttgca gtgagccgag atcatgccac    4320
   tgtactccag cctggacaac agagggagac tatctcaaaa aaaaaaaaaa aaaaaaaaaa    4380
   aaaaaagagg ccgggcggcg gtggctcaca ccatgtgatc ccagcacttt gggaggccga    4440
   ggcgggtgga tcacctgagg tctggagttc gagaccagcc tggccaatat ggtgaaaccc    4500
   cgtttctact aaaaatacaa aaattagctg ggtggggtgg caggcacctg taatcccagc    4560
   tactccggag gctgaggcag gagaatccct tgaacctgcg gggcggaggt tgcagtgaac     4620
   caagatcaca ccattgcact ccagcctgga caacaacagc aaaactctgt ctcaaaaaaa    4680
   aaaaaaatct tttttttcga gacacagttt tactctctcg cccaggttgg ggtgcagcac    4740
   cacgatctca gctcactgca acctctgcct ctcagattct cgtacctcag cctcccaagt    4800
   agctgggatt acaggtacct gtcaccacgc ccagctaatt tttgtatttt tagtaggggc    4860
   gtggtttcac catgttggcc aggctggtct tgaactcctg acctcaagtg acctgcccgc    4920
   ttcagccacc caaagtgctg ggattacagg cgtgagccac cacgcttggc ctttttaaat    4980
   gaaaatagtg caaaaatcca cgataaacaa aatatcaaaa atttactgaa cttgcacttc    5040
   cacaaccctt tctcacctgc ctcccaggct actctctgcc ccagaaagca acttaaaaaa    5100
   tgtgcagatg gagtttggac tttacctgaa aatggtggga gctatggaaa accttggagc    5160
   aggggagtga aggatagaaa ttatatgtaa aagaaaccct gggccgggcg cagtggctta    5220
   tgcctgtaat cccagcactt tgggaggccg aggcaggtgg attacctgag gtcaggagat    5280
   tgagaccagc ctgaccaaca tggtgaaatg tcatctctac taaaaataca aaaaaaatta    5340
   gccaggcatg gtggtgcacg cctgtagtcc cagctactcc ggaggctgag acaggaaaat    5400
   cgcttgaacc cgggaggcgg aggttgcagt gagccaagat tgtgccattg cactccagcc    5460
   tgggcaacaa gagcaaaact ccatcttaaa aaaaaagaaa gaaagaaacc ctctggcagt    5520
   tgatgagaag gaaacttaat cggcaggtcc cagcagggga gatgaggaga ctctagggag    5580
   ggcatttgca catgctgtgc cccagtgtgg gccagggagc aggtcactac tcctcccgtc    5640
   taccttcctc ttgctccaac cccttcaagc tttggaccag tggtacccta agtgtagtcc    5700
   aaggaaccac atgcatcagg acccccaggg ggtgcttgtt aaaaatgcaa attttggcca    5760
   ggtgcagtgg ctcacacctg taatcccagc actttgggag gccgaggcgg gtggatcacg    5820
   aggtcaggag atcgagacca tcctggcaaa cacggtgaaa ccccatctct actaaaaaaa    5880
   caaaaacaaa ccaaaaaaaa cattagctgg gcgtggtggc gggcgcctgt agtcccagct    5940
   actcgggagg ctgaggcagg agaatggcgt gaacccggga ggcggagctt gcagtgagct    6000
   gagattgcgc cactgcaccc cagcctgggc gacagagcga gactctgcct caaaaaaaaa    6060
   aaagcaaatt tcttgggcac caccccacat tgactg                              6096
<210> 3
   <211> 24959
   <212> DNA
   <213> Artificial Sequence
<220>
   <223> Synthetic construct: chemically treated genomic DNA (Homo sapiens)
   <400> 3
   ttgagagtta ttgatttatg agtaagagta aggtagttta aggtgttgag atagggtgtt      60
   atgtgttttt tgggatattt gttttaattt tttattatat attttttgtg ggtggatgtt     120
   taagggttag aaatggtaat attttataag gattagtttg tgggggttaa ggaaaggatt     180
   tacgtttttt tgggatgatt agtaagtttt gaatttgaaa aagggtggtg ttatttatga     240
   aattatagag taaaggagaa gtaggttggg gttggtgttg gggatagagg agatgttgag     300
   tttagtgttg gatttggaaa attttagata gttgtaatga gatgtttgga gaaaatacgt     360
   agttggtagt tgtgtttatg ggtttggatt ttttattagt gatttaggtt agagataata     420
   tagttgggag ataatagtag ataggtagtt attaaagatt tgggtcgggc gtggtgattt     480
   atgtttgtaa ttttagtatt ttgggaggtt gaggcgagtg tttatttgaa gttaggagtt     540
   cgagattagt ttggttgata tgatgaaatt ttgtttttag taaaaatata aaagttagtc     600
   gggcgtggtg gtgggttttt gtaattttag ttatttagga aattgagata tgagaatcgt     660
   ttgaattcgg gaggtggagg ttgtagtgag tcgagattgt gttattgtat tttagtttaa     720
   gtaatagagt gagattttgt tataaaaaaa aaaaaaaaaa agatttgaga ggggatgagt     780
   ttatttatga agagtgtagg aagaaaaaga agtttttttg ttttgagaaa gaattatcga     840
   tattaagtat gtaggtgaaa agagaaatag aaaagaagat agaaatggtc ggagtaggat 900
   aatgaggtaa aaaggggtta ttaaaggtgt aggaagtaga ttgagaaaat ttaaggaagt 960
   tatagatagg tagtatattt cgttattttg gtttttttaa gttgttggag agagtagggg 1020
   tattttcgta gttgggtata ggtattattt tttttgcggt ttttagattt tttttagagg 1080
   agtttattaa tttttattta cgtattgata tttgttttgg agtttgattt ttgatgtttt 1140
   gtggttgggg ttgagggagg gaggaatagg gtattgttat agattttttt atatatagat 1200
   atatataaat ataattttga tttttgattt atttcgagta ggaaagttgt aaagtttttt 1260
   aatttcgttt tattttttgg aatttcgttg gcggttttag attttagaaa gtttttaatg 1320
   atgaattttt ggtttttagt tttttttttt ttttattatt ttttaggttg agattattta 1380
   gttttgagtt ttgtatttgt tatgtgtttt ttgggatatt tatttggaat tatatatttt 1440
   atgagttaat ggtgttatta ttattttttt ttttatatta ataattttat tttaattttt 1500
   ttatttatta ttttattaaa aagggattta aaaaaataat tagttttttt tgtttttttt 1560
   tattttttta ttggtgttgt cgaagttgta gaaattgaga ttatagagga aacgggatgt 1620
   agttaggatt atatagttag gtagagttag gattaaattt aagtttttga gtttgaggta 1680
   gtagagaata gtgattttga gtagagaggg gttaggggta gattatttta gttttgttat 1740
   ttagttgttt gattttgggt aagttattta atttttttat gttttatttt ttttttgaaa 1800
   atggggatga tatgggtatt ttttttatag agttgttgtg agggttaaag aatttaatat 1860
   tcgtgaagta ttttgtatag aggttggtat attttgtaat aagtttgttg tgattatttt 1920
   tattttgttt tggtttgtat attttgtttt taaaaaatat tttttttttg tttttttaat 1980
   ttttgttttt tttagtattt tttttatttg tttattttta atttttgttt ttgtttgtta 2040
   ggaggggagg gttgggtttg gggagaaaga gagagtgatt atagagattg gaatttgttt 2100
   gatagttagg gagtgtttgt tatggtaata taggaatatg tagtttgcgt atgttagtgt 2160
   aataattata cggtatttat gttagttagt tatatatagt tatttaatag tttttgggta 2220
   ttttatgttt atatttaatt agtttaggat tttgtggttt tagaaatatt ttggtttttt 2280
   gttaggtgga gtttatattt aaatttattt taggtaagtt tttttttttg tttggttagt 2340
   ttttgttttt aattttttta gagtttggtt tggtttggaa gtttttagtt taggaagttt 2400
   gagagcgaag tgggtatttt cgagggattt tgatttagaa ttatttagga aagattgtga 2460
   tggtagattt tttaaatata cgtattgata ttgtgtgcgt atgagtagat aggttggagt 2520
   ttttagttta taaatttttt ttacgttttc gattatttat tttatttaaa gttttttgag 2580
   gtgtttagag aaaggattga tatttatttt atcgaggaag ttgaggttag cgaagaatag 2640
   tgttttgttt aaaggtaagg atgtgataga gttaggtaaa tgtagggttt ttgcgtttta 2700
   gtttcgaata tttttgtttt attgtagttg cgagttgcga gtttagaagg cggttttgat 2760
   taatgtgggt tgaatttacg ttttttattt attttattcg atttcgttaa agttattgtt 2820
   ttttttgtgg tagtcgttgg ggaaaaggga gagaattgtt atagtttgtt tttttaatgc 2880
   gcgttttcgg aggttcggcg cgttttcgtt attataattg gagtgtatgg agtaggttac 2940
   gtttagagga ggaagggcgg gcgttgggta ttcgttgatc gattttttta agtgcgatta 3000
   gtgttcgttc gttttgtttt tatggattcg ttcgggaacg gttatcgttg aggattttac 3060
   gtttattagg atttcggttg ggtcgtattt agttatcgta tcgtgatttt cgcgggttgt 3120
   gtcggttttc ggcgtttttg ttcgtagcgt tcgtcgtcgg gttagggttt cgtcgtcgtt 3180
   acgtttcgcg ttcggtattt atcgttttat gttggtgtat atttatttcg ttatggtgag 3240
   tagtttcggg ttcgggatat attcggtcgg gggatcgggg cgtttgagtg ttggattttt 3300
   taattttttt gtttcgcgtt aacgaaattt cggagggagg gggtcgtagg gatttcgtta 3360
   gttgagaacg cgatgcgtaa gtgatcgttg tttttagttt gaggtttttg cgttcgcggg 3420
   tggttcggaa ataaatttcg ggttttaaga aacgggaggg attgtagttg aattttttcg 3480
   agttgaggag gattttttta ggttggggtt tttttagttg ttagtgggtt atttaaggta 3540
   tttttttttt tttttaggag cgtttcgacg ggttgggagt agttgtcggc ggggttcgtt 3600
   tgtcgttttt gttttaggcg gtttacgggt cggcgttttc gttttgttat acgtcggtcg 3660
   ttatagttgt cgtcgaattt tagtcgtttt atttttcgtc gttttattcg tagttatcgt 3720
   tgttttacgg ttaggcgttc gacgtcgtcg tagttttttt ttatttggta ggggatttat 3780
   atggcggttt ggcgtttttg gcgtagtcgt agttttttta ggtcgtttgg gtcgcgtttc 3840
   gcgtagtcgt tcgcgtttac gaggagtttt tcggtttgtt ggtatcgttc gttcgcgttt 3900
   tgggttttga ttcgcgtcgt gattatgtta ttgtcgtgtt tcggtttttg tacggtttgg 3960
   tcgacggcgc gtacggtttg gtagacgtat ttttcggttt ttcggggttg gcggcggttt 4020
   tcggtttgga ggatttgtag gtgagattcg agggattcgg gatgggtcgg gattggtcgc 4080
   ggtggtttat tattatggtt ggaggtagaa ggtgataaat gtaggaagtc gatttttttt 4140
   ttagttcgtt atattttatt ggtgatttcg aggatgtgtt ttattttttg ggttagacgt 4200
   tgtttggtta ttaaggttgt ttggtattga gttcgttagt agtgcgtggg tggtagtaat 4260
   tttcggtagg gatcgaatta tttttttttt tttcgttgtt acgtgtggta tttatggggt 4320
   agtttgtatt tggggcgaag agaagtataa tagttattag tgtaagattt aaaatttttt 4380
   ttttgtattg tatagtgaga tgtttagggt tttagtttag tgtttggata tagcgatttt 4440
   tgggtttgtt cgtcgtcgtt ttaagcgaag ttggtgcgtt ttgggcggag tagatagaga 4500
   ttttgggtgg taggggtttg ggaagatatg ggcggttagg gttttatgcg tttttatcgt 4560
   tgttttttgt tatttgtagg taatggacga gtcgggaatg agttttttag attagttcgt 4620
   gattaagaaa ggtaaggaat ggtttgttag ggtagagttc ggcgagatgg tgtaggtttt 4680
   tggtgtatag atttattttt tttatcggtc gtgttttttg tgtgtcgtta ggttgggtgt 4740
   ttattaggta ttttttttgg tttagttaga tgttaggtag acgtgcgggt ttggtgagtt 4800
   tgtttagtat tttgtggttt ggggtgggtt ttagcggatt agtatttatt gggttgtagt 4860
   attgggagtt tggttttttt tcgtcgaggg ggagggtatt tttgtggatt tggagttgat 4920
   ttgtagaacg agttaaatta tttttttgtt tttttaagag atgggaatgg aagtgttgtt 4980
   tttacggagt tggggaaatg atttttattt tatagtgttt tagtatttcg gtgtttggcg 5040
   ggtatttttt tttttttttt tttaggtagg gttttggagg tttttggggg aatttttttt 5100
   ttgtgggagt tttttgcggt atttagattt aggggagttt gtgtgtgagt attgtgtgtt 5160
   aggttgtgtg tatttgagtt agggtttatt tgtttttggg tgtttgtgtt tatgtgagtt 5220
   tagggttttg tgtatgtttg aaatgttttt tttatgggtg ttttagtatt ttttggagtg 5280
   tgagtgtgtt tgtttttgtg aatgtgtttg tgaggtgtgt ttttgtatgt tggtgtgtat 5340
   ttttttgtat ttgggggatg tatatatttt ttaatatgta tagtattttt gttgtgtttt 5400
   gtattttgtt ttttggtatt tgaggatttt taagtatgcg cgggtttttt ttgtgtatat 5460
   ataggagtat ttatgtgatt tttggtatta gtaaaattta gggatacggg atttattttt 5520
   tttggtttga ggattaagta ttggttatga taggggaagg tgagagacga taaaaataga 5580
   gagatagtta gagaggagta gagagttaga ggggtttagg tattgggtag tagttttttt 5640
   atatttgggg taggtgttcg aaagaattta gaggtgtata tgagtttgag gtgttttagg 5700
   taggtattgt ttttataggg tttggtttga gttgtttttt aaacgagtga atttaagttt 5760
   gggttttatt tgttttttat ttgtttttag gggaggttaa ggtggaagtg gtggtagtag 5820
   ggttggggtt ggatttttag gagttggggt tgagttatta ggagttgggg gttgggtgga 5880
   tgatttggag tgtgtagtag ggaagatgag gtaatagggt aggaagtggg tggggggagg 5940
   tggaattggg gttgtgtttt gtgtcgtttg gaattgggag tgtgggaaag atattaggaa 6000
   tttggttgta gcgtagtttt gttggtgggg tttggttggt ttattgtata gagttttttt 6060
   tgatttttga agaaagagat tcgtttgtag tgggtaaaag tttgtttgga ttttttggtt 6120
   attagaaata tgagtatggt ggtggttttt agttttttat ttatgtttgg gtttaagaga 6180
   ttgggagttt aggtttattg attttttgag aaagattaag attttgtatt ttagaaagag 6240
   gtttggggat ttttgttttg cgtaagggta gaaggattag ttgttttttt gagtatttta 6300
   attcggaatt tcggttcgaa gtcgagatag gagattggat gcgaggtttt tttagagttg 6360
   gtttttttta aataattttt aaaattttta gattttaggg gtacgtcgaa attttttaaa 6420
   gtagtttaaa gaatataacg agagttttaa tattttaggt ggcggcgcgt tggttttttg 6480
   gagcggggcg ggacgcggtc gcgcggattt acgtgtataa tcgcgcggga cggggttacg 6540
   cggatttacg tgtataatcg cgggatttta gcgttagcgg gattttagcg ttagcgggat 6600
   tttagcgtta gcgggatttt agcgttagcg ggattttagc gttagcggga ttttagcgtt 6660
   agcgggattt tagcgttagc gggtttgtgg tttagtggag cgagtggagc gttggcgatt 6720
   tgagcggaga ttgcgttttg gacgttttag tttagacgtt aagttatagt tcgcgtagta 6780
   gtagtaaagg ggaaggggta ggagtcgggt atagttggat tcggaggtcg tgatttaggg 6840
   gaaagcgtgg gcggtcgatt tagggtagtt gcggcggcga ggtaggtggg ttttttgttt 6900
   tttggagtcg ttttttttta tatttgtttt cggcgttttt agtagttttt attttggttt 6960
   ttcgcggtta ttgcgggatt cggcgttgtc gttagtttag tggggagtga attagcgttt 7020
   tttttcgttt tcggtttttt cgacggtacg aggaattttt gttttgtttt atagattttc 7080
   ggttttcgtc gagtgcggta ttggagtttg tttcgttagg gttttggaat tagagaaagt 7140
   cgttttttgg ttatttgaag cgtcggattt ttatagtgtt ttttagtttg ggcgggagcg 7200
   gcggttgcgt cgttgaaggt tggggttttt ggtgcgaaag ggaggtagtt gtagttttag 7260
   ttttatttta gaagcggttt tcgtatcgtt gcggtgggcg ttttcgggtt tcgatttcgt 7320
   tagcgtcgcg gggtagaggt atttggagtt cgtagggttt agatttgggt tggaaaagtt 7380
   tcgttgattg taggtaagcg ttcgggaggg gcggttaggc gaagtttcgg cgttttatta 7440
   tatattttcg ggttttatgt tagttgtatt cgcggtattg ggtaggaaat ggtagggttg 7500
   aggtcgattt taggagtata agggagtttt ttattttttg tttatatttg ttatttttag 7560
   ttttgtaatt tattttagat atatagaaag taagtaggat tggtggggag acggagttta 7620
   ataggaatat tttttagtag tgagtagggg ttgtatggga cgcgggagga gtttagagga 7680
   ggcgcggaga gtgttcgagg ttgggtgagt gtttagaggg gagatagttg aatcgggttt 7740
   aagaggtgtt tagtgggtgt ttgttgaatg aatgagtgat gggttttgaa gtttgagtgt 7800
   attgaaagag gggtgtgtta aaaagggttt tttttattat ataggatata gtatatgtaa 7860
   attttttttt tgtggaaaag ttagataggt taaaaaggtt ataaataaat tagtcgggta 7920
   tggtggtgcg cgtttgtagt tttagttatt agggaggttg agttagggga atcgtttgaa 7980
   ttcgggaggc ggagattgta gtgagttaag atcgcgttat tgtattttag tttggaaata 8040
   gagcgagatt tcgtttcgga aaaaaaaaaa aaaagttata aatcgtgtgt gggttttagg 8100
   ttatataatt agagttggag gggagtggtt aaggatgaga attgagatgg atttttcgtt 8160
   ttttttggag gagagtgggt ggttgtttat ttgggggtgg ggaatttttt tttacgggtt 8220
   tagttgttta attttagggg atttttagga taggagttga tgtaaatagt cgttttattt 8280
   tttgttgttt ttggttttgg agaaggagga gggagttggg gagggttttt attttttaga 8340
   taatttttaa gtagttagga tatgggtgag atgagtgaga tattgatttt tgggatagaa 8400
   tttgagaggg tgttaaaaaa tttagtaatt aagataaata ggtcgggcgt agtggtttac     8460
   gtttgtaatt ttagtatttt gggaggtcgg attatttgag gttaagagtt cgagattagt     8520
   ttggttaaga tggtgaaatt ttatttttat taaaaatata aaaattagtt tagtgtggtg     8580
   gcgttagttt gtaattttag ttatttagga ggttgaggta agagaattgt ttgatttagg     8640
   aggtagaggt tgtagtgagt cgagattatg ttattgtatt ttagtttgga taatagaggg     8700
   agattatttt aaaaaaaaaa aaaaaaaaaa aaaaaaaaaa gaggtcgggc ggcggtggtt     8760
   tatattatgt gattttagta ttttgggagg tcgaggcggg tggattattt gaggtttgga     8820
   gttcgagatt agtttggtta atatggtgaa atttcgtttt tattaaaaat ataaaaatta     8880
   gttgggtggg gtggtaggta tttgtaattt tagttatttc ggaggttgag gtaggagaat     8940
   tttttgaatt tgcggggcgg aggttgtagt gaattaagat tatattattg tattttagtt     9000
   tggataataa tagtaaaatt ttgttttaaa aaaaaaaaaa attttttttt tcgagatata     9060
   gttttatttt ttcgtttagg ttggggtgta gtattacgat tttagtttat tgtaattttt     9120
   gttttttaga ttttcgtatt ttagtttttt aagtagttgg gattataggt atttgttatt     9180
   acgtttagtt aatttttgta tttttagtag gggcgtggtt ttattatgtt ggttaggttg     9240
   gttttgaatt tttgatttta agtgatttgt tcgttttagt tatttaaagt gttgggatta     9300
   taggcgtgag ttattacgtt tggttttttt aaatgaaaat agtgtaaaaa tttacgataa     9360
   ataaaatatt aaaaatttat tgaatttgta tttttataat ttttttttat ttgtttttta     9420
   ggttattttt tgttttagaa agtaatttaa aaaatgtgta gatggagttt ggattttatt     9480
   tgaaaatggt gggagttatg gaaaattttg gagtagggga gtgaaggata gaaattatat     9540
   gtaaaagaaa ttttgggtcg ggcgtagtgg tttatgtttg taattttagt attttgggag     9600
   gtcgaggtag gtggattatt tgaggttagg agattgagat tagtttgatt aatatggtga     9660
   aatgttattt ttattaaaaa tataaaaaaa attagttagg tatggtggtg tacgtttgta     9720
   gttttagtta tttcggaggt tgagatagga aaatcgtttg aattcgggag gcggaggttg     9780
   tagtgagtta agattgtgtt attgtatttt agtttgggta ataagagtaa aattttattt     9840
   taaaaaaaaa gaaagaaaga aattttttgg tagttgatga gaaggaaatt taatcggtag     9900
   gttttagtag gggagatgag gagattttag ggagggtatt tgtatatgtt gtgttttagt     9960
   gtgggttagg gagtaggtta ttattttttt cgtttatttt ttttttgttt taattttttt    10020
   aagttttgga ttagtggtat tttaagtgta gtttaaggaa ttatatgtat taggattttt    10080
   agggggtgtt tgttaaaaat gtaaattttg gttaggtgta gtggtttata tttgtaattt    10140
   tagtattttg ggaggtcgag gcgggtggat tacgaggtta ggagatcgag attattttgg    10200
   taaatacggt gaaattttat ttttattaaa aaaataaaaa taaattaaaa aaaatattag    10260
   ttgggcgtgg tggcgggcgt ttgtagtttt agttattcgg gaggttgagg taggagaatg    10320
   gcgtgaattc gggaggcgga gtttgtagtg agttgagatt gcgttattgt attttagttt    10380
   gggcgataga gcgagatttt gttttaaaaa aaaaaaagta aattttttgg gtattatttt    10440
   atattgattg tatgagaatt tttggaagta tggttttgga atttgtattt tttaacgatt    10500
   ttttgaggta aaatttatat atttaaaatt tatatattgt aagaatataa tttgatgatt    10560
   tttagtaaat gtagagttgt gtaattatta ttataaaaaa atttattttg aggtattttt    10620
   gttattttta gaagttttta tttgtagtta ttttttattt ttattttaag ttttaggtta    10680
   ttattgattt gttttttgtt tataaatttg tatattttgg atatgtttta taaatagaat    10740
   atataaatat gtagattttt gtattaagcg ttttttgttt agtataatat ttttgaggtt    10800
   cgttttaaat attatgttaa gtaaaagaaa tagtaataaa aagaaataaa ttattattag    10860
   tagtttgttt ttttttattg ttttatagta tgttattgta cgggtgtaat atattttgtt    10920
   tatttattgg ttagttgacg ggtatttggg ttgtttttat tttttagttg ttttaaataa    10980
   tgttgttatg aatatttata tatatgtttt attatagaaa tatattttta gcgtttttga    11040
   gtaaattttt aggagtgtaa ttgttggatt atatggtcgg tttaggttta atttttaaga    11100
   aattgttaag ttatttttta aagtggttgt attattttgt atttttatta gtaatgtgtg    11160
   agggttttat ttttttatat tttatattta gaattgtttt tttttttttt tttttttttt    11220
   gagatggagt tttatttttg ttgtttaggt tggagtgtaa tgaggtgatt tcggtttatc    11280
   gtaatttttg ttttttgggt ttaagtgatt tttttgtttt agttttttga gtagttggga    11340
   ttataggtgc gtgatattac gttcggttaa ttttgttttt ttagtagaga cggggttttt    11400
   ttatgttagt taggttggtt ttgaattttc gattttaggt gatttattcg ttttggtttt    11460
   ttaaaatgtt gggtttatag gtatgagtta ttatgtttgg ttttttttat ttatttattg    11520
   ttttgagatg gattgttttt ttgttattta ggttggagtg tagtggtata atttcggttt    11580
   attgtaattt ttgttttttg ggtttaagta atttttttgt tttagttttt ttagtagtta    11640
   ggattatagg tacgtattat tatatttagt taatttttgt atttttagtt gagacggggt    11700
   tttattacgt tggataggtt ggttggtttc gaatttttga ttttatgatt tatttatttt    11760
   agttttttaa attgttggga ttataggtat gagttatcgt gtttggtttt tgtttttttt    11820
   attataatta ttaagtggta ttttagtata gttttaattt gtattttttt aatgattaat    11880
   aatgttaaga atttttttat gtatttattt aattatttat tttttatttt tagtgaaatg    11940
   tttatttata tatttggatt attttttatt taggttgtta ttttattgtt gagttataat    12000
   agttttttat ataatatatt ttgaatatag ttttttatta gatatatgat ttgaaaatat    12060
   ttttttttag tttgtggttt atcgtttttt tttttatttt taaaatttta attttttttt    12120
   tgtattttta aaaattattt tttttttttt ttttatagaa atagggtttc gttatgttgt    12180
   ttaggttggt tttaaatttt tggtttttag tgattttttt gttttggttt ttttaagtgt 12240
   tagggttata ggagtgagta attgtttttt tttttttttt taattaaaaa attttttaga 12300
   gataggtttt attaagttgt ttaagttgga gtttggtggt tatttatagg taagattata 12360
   gtatattgta ttttcgaatt tttgaattta agtgattttt ttgtttgagt ttttcgagta 12420
   attgggatta tatttttatg ttattatatt atgttatttt tttaataata ttttttgagg 12480
   tgtaaaagtt ttagattttg atgaagttta gtttattatt attttttttt tttttttttt 12540
   tttttttttt ttgagattag ttttattttg tcgtttaggt tggagtgtag tggcgttatt 12600
   ttagtttatt gtaattttcg tttttcgggt ttaagcgatt tttttgtttt agttttttaa 12660
   gtagttggga ttataggtga gtgttattat gttgggttaa tttttgtatt tttagtagag 12720
   atagttttat tatgttggtt aagttgtttt taaatttttg attttaggtg atttgttcgt 12780
   tttagttttt taaagtgttg ggattttagg agttagttat tatatttggt ttattatttt 12840
   tttttatggg ttttgttttt ggtgttgtat ttaggaattt tttgtttaat ttgaggttac 12900
   gaagattttt tttttaagtt ttttgttgtt gttgttgttg ttatttgtat ttttagtaga 12960
   gatgggtttt ttttatgttg tttaggttgg ttttaaattt ttgattttaa atgatttatt 13020
   cgttttgttt tttaaagtgg taggattata ggtgtaagtt aggacgtttg gttttttatg 13080
   ttttttttta gaaaaaattt gtaattttaa taaggttttt tggagagttt gttgtatatt 13140
   tgtttaggtt tagaagttag attatattta ttatttgcgg atagagtgtt ttgtttgata 13200
   ggttgaataa tcgggagttt ttgtgtttgt aggagtgtgt gtgtgtgtgt gtgtgtgtgt 13260
   gtgtgtgtgt gtgaatggtt tttgtatatg tgattagtag gatattatta gattatttta 13320
   gttttaggag ttattatagg gaattttgtt ttgtgtgtgt gggagataaa ttttagtgtt 13380
   tttgttttgt tttgtgtgtt agtttaattt ttatttttat tattgttttt ttgtgatcga 13440
   tttgtttttt taattatgga ttttattttt tttgttattt ttaagggaag aagttttgag 13500
   aaaaaaaggg ataggtgggg gaagtagtaa ggttagttat ttatatatat aaatatatga 13560
   gtaatgttat ttttgttttt atatttagta gtatttatgt ttgtataaat ttggtattaa 13620
   taggagaaag tatttgtaaa tatttatatt taggttatta tattttttta tattttagtt 13680
   tatttttatt gttgtattta ttataagggt atatagtttt tgtatatagt tatttttttt 13740
   tatttttttt gagatggagt ttcgttttgt cgtttaggtt ggagtgtagt ggtgtaatta 13800
   tagtttattg tagttttaaa ttgggtttag ttattttgtg gttttagttt ttcgagtagt 13860
   tgggattatg ggtatatgtt attatgtttg gttaatttaa aaaaattttt ttttgtagag 13920
   atgaggtttt attatgttgt ttaggttggt tttaaatttt tgggtttaag tgattttttt 13980
   gttttagttt tttaaagtgt tgggattata ggtgtgagtt attatgttta gttagtttgg 14040
   gttttaggtt ttaatagtag ttggatggag aaggttgttt attttttttt tttaattttt 14100
   atttttggtg aggggtgatg ggagggaaga tagttatttt gttttttttt ttatttttaa 14160
   tttattaatt ttttgttttt gtttttgttt ttgtttttga gatagggttt tattgtgtta 14220
   tttaggttgg tatggttagt ttattgtaat ttttgttttt tgggtttaag taattttttt 14280
   attttagttt ttagagtagt tgggattgta ggtatgtgta attacgtcgg gttaattttt 14340
   gtattttttg tagagataag gttttattgt attgtttagg ttggttttga atttttgggt 14400
   ttaatttttt tatttcggtt ttttaaagtg ttgggattat aggtgtgagt tatcgtgttt 14460
   ggttttattt gtagttttat ttgtttgaag ttaagttcga ggtataattt ttttatatat 14520
   atagtatagt ttagttcggg tttttagagt ttttgtatta ttagtttatt ttatatttat 14580
   ttatggttat ttgtgttaag attttgtttt ttatatattt atgtttggta gggaagatat 14640
   ttatggaaat agattaggaa ggtttagtgc ggtaaggtta gggatggggg tgagagggag 14700
   ttggggggta ggggagagaa gttttgtata ggggttggtg tttaaattta gtagagtgga 14760
   gtaggatttt tgagaatcga aggagtattg tggggtttaa agagtttata ttttgggagt 14820
   tattatattt taggaggtat tttatatttg ttttatttgg tttgtgttgg gtttttaaat 14880
   ttttgaatta ttgttaatgt ttaaaatttt agagatttta tagaaaaatt tagaatttta 14940
   gttttaaaaa ttagtaaaat aaaataaaaa tataaataaa ttaggagttt tgtgttttgt 15000
   ttggcgggat tttgtataag gtgttttttt agtttgttaa ttttttttat agtttattta 15060
   ttatgttatt tgttgagttt tttttttttt gaagtttgga gttttgttgt ttattaagtg 15120
   gttattatag ttacgagtgg tttttgagta tttaaaatgt gattagtttg aattgagatg 15180
   cgtttaagta taaaatgttt attggatttt gaaggttaat tttaaataat gtaaaatatt 15240
   ttattaataa tttttaaaat attgattata tgttgaaatg attttatttt taatatattg 15300
   ggttaaataa aatatattaa atagtaatat attatttatt attatattga ttaaaattaa 15360
   ttttatatgt ttttttttat tgtttaaaat gtgggttagg tttggtggtt tattttttta 15420
   attttagtat tttgggaggt taaggtaggt aggtggatag tttgagttta ggagtttaag 15480
   attagtttgg gtaatatggt gaaattttat ttttatgaaa tatataaaaa ttatttaggt 15540
   atggtggtac gtatttgtag ttttagtaat ttgggaggtt gaggtgggag gattgtttga 15600
   gtttgggagg tggtggttgt agtgagttgt gattgtatta ttgtatttta gtttgggtaa 15660
   tagagtaaga ttttgtttta aaaaaaaaaa tggtggatat taggaaatgt aaatgtatat 15720
   atgtggttta tattatattt tttttggtta gtttgggttt agagtttaat ggataaatgg 15780
   ggatttagta agaggggtag gtagagttag aatataaggg agggtgaagg aggttattgt 15840
   aatatgatgg ttaaagttgt gaattttgga gttagtgttt agagttgaag gtttattttt 15900
   gttttttatt tgttgtttta ttttaatttt tttgaatttt tagtatttgg tatttattga 15960
   taggtgaaat tatagttggt atttattgat aggtgatatt attttgttta gttttggagt   16020
   tgggatttga tttttaggga attgatcggg ttttgatgag ttttaggttg agtttttggt   16080
   tgtagaatga tgtatttaga tggaaagatt tttttggatt ggggtagggt gattagagta   16140
   aggtttgtgg agaaatttat atgggaaggg aatttgtgtt tgtgtgtatg tagaggagtg   16200
   gttatatata aggtatataa agaaatgaaa gtcgtaggat gggtaggggt gggagaaata   16260
   aaaaaagaaa gttgtaggat ttagtgatgg atgggatgtg gtgatggatg gggtgtggtg   16320
   atggatggga tgtgttgggg gagagggttg tagagggtat tatagaggtt tttggttggg   16380
   ttgttgggtg gaggtgggga ggtttattta gagagggaga gattagacgt tttgttaggt   16440
   tacggattag gttagttttt tttatttttg tattttttag ttttggtatt atttttggta   16500
   tgaaagtgtt tagttaatat tgttaagtgg ttggaaggtt aatagtatcg tttttgagat   16560
   tgtagttttg ggtttgttat cggtttttcg tttattagtt gtgtgatttt aggtatatta   16620
   ttttattttt ttggatatta gtttttttat ttgtaaaatg ggaatagtga taagtgtagt   16680
   tttttgtggg ttgttttaag gattgagtgg gtttatatac gggagtatat agtagtgagt   16740
   gtttagtagt tgttagtttt agattttata ttcgagtttt atttttgatg tatatacgag   16800
   gttttgttta ttaaggttgt tattttttta aaggaagaaa aggttaaagg gtttcgttaa   16860
   agaagtaagt gtttcgatat tttttttttt tttgaggtta agtaggagga tggaggttag   16920
   attgtaggaa gaattttttt ttttttatag ttaagaatgt ttaagtgaat tttagaaaat   16980
   taatttttgg tcgggtacgg tggtttatgt ttgtaatttt agtattttgg gaggtcgagg   17040
   cgggtggatt atgaggttag gagtttgaga ttagtttggt taatatggtg aaattttatt   17100
   tttattaaaa atatagaaat tagttaggcg tggtggtggg tatttgtagt tttagttatt   17160
   taggaggttg aggtagaaga atcgtttgaa ttcgggaggt agaggttgcg gtgagtcgag   17220
   atggtattat tgtattttag tttggtgata gagcgagatt ttattttaaa aaaaaaaaaa   17280
   aaaaaaagaa agaaagaaag aaaattaatt ttttagattt tttttttggt ttttagataa   17340
   ttagtagata attagggagt gtttggggag ggggtatagg gtttattttt tggttttttt   17400
   tagtattcgt agagtgattt ggtaggaaga gagggttagg gttgaaggta ttaatagggt   17460
   aataattttt ttgtttttag gatgtggagt ttatagtttt taggtttttt atcgatgtag   17520
   gggtgaggtg gggtgggagg gtagagagga aaaacgtggg aattacgaat gggaagaggg   17580
   gatttgggga ttttttgtga ttgtttagtt ttggtttgat tttgttgtag ggttttaggg   17640
   gttttgtttt tagtttatat ttatattttt atatttaggt ttttttttga tggtattcgt   17700
   taaaatatta agatttcgtt ggagttgttt gagtttaaga taggattagt ttggtttagt   17760
   gagggggata tagatttaag agttagataa ttcggagttt agtttttgag tttatattta   17820
   ttagttgagg gaatttgggc gagttatttt tattttattg tggtttagtt ttttttttta   17880
   taaagtaagg atattagatt tttttttgag tggggttgtg tgcggtagtt attggtttag   17940
   cgtttttttt ttttttatag tgtttatttt ttttaaagtt agtagttttt tagttttttt   18000
   tttggttaaa gatagtttgg tgggcggtat tataaatttt ggtgaggttt tttgtttcgt   18060
   gttcggtcgg tttttattgt ttagtttaac gtttaagtat aaggtgacgg tgggggaggt   18120
   gtagcggcga ttttcgtttt tcgagtgttt taacgttttt tttttggggg gtgtttttcg   18180
   taggtaggaa ggttagttta taatttttcg tttgattgga tttttggttt ttttaggttt   18240
   ttttaaggta ttagagagga ggttagtttt atttaggttt tttgttttag tttttttggg   18300
   aggggaggtt tcggggattt tggattgtgt atgttgtggg ggtcggttta ggtttttggt   18360
   ggttgttata tgtttgtggg tttggggtag ggaggtgggt agagaaagaa gtttgggtat   18420
   agagaaggga tggagaattt atttatattg gttaggacga gattgggttt ggggaagaga   18480
   gagggttatt tttatttttt tttttaggaa atgaatttta ggattcgagg tttattttgc   18540
   gcggtagtat aggttttgtg tgttttgggg tgtggagtat ggtagtttat ggtttatttt   18600
   tttagatttg tgtttttttt tttttgtgta tgtttttgtg tttgggaagg gtatttttgt   18660
   tttagggtta tgagtttgga tgagtttgag gatggtgtgg atatgtgtgt gcgtgtgtat   18720
   gtgtttatag tagcggatgg ttaggagtat gtgtttttat gagtgtgagt attttgtgtt   18780
   ttttttagtg ggatgtgtgg tttagggtgt gttttgtttt gtgtgtgtgt gtgtgtgtgt   18840
   gtgtgtgtgt gtgtgtgtgt gtgtgtgatt gtttgtgtat ttggagttag ttatatggat   18900
   gtttatatat ttgtgttatt gtgtgggagt ggtatgtgtg tagttatagg ggttattttt   18960
   ttatttttag cgttttggag gatttttgtg agtttttgag tgtttgtata tgtgtttttg   19020
   ggattttgat ttggtgtgat tttgtgtttt tgtgtttttg ggtgtttgag tttgtgtttt   19080
   tgtatgtttg tttttattgg ttttatgtgt ttttattttt ttacgtgtgt gtgtgtgtgt   19140
   gtgtgagaga gttattaggt ggttgtgtgt gtatttgtgt ttttttatgt gttttttttt   19200
   ttgtatgtta ttgcgtgttt ttatatatag gtttgtgtat tttttggtag gtgtgtgtgg   19260
   ttttttggtg tgttaaggtt tttaggatgt tttattttgg ttgtttttgt tgttgagagg   19320
   attagagtga attgaggggt ttttggagtt gggtgttggt ttttttgttt ttgttttggt   19380
   tgtaaaatta tggagattag ttgcgttttt gttgaaagag attttagagt atttttagga   19440
   ttaattttaa attttaatag gggttggtgg aggtaataag gttttttggt agttatttag   19500
   tttttatgcg ttttttttgt ttttatagtt gtatgtttgt ttgtttttgt gtatgttaag   19560
   ggttaagttt aaaaatgggg gtcggtgttt gcgggaacgg ttagagaaga ttgggtttaa   19620
   tttgttagtt ggtcgtcgta aggtcgttaa tgtgacgttg ttgatttcgt tagtggaagg   19680
   tgagtgaggt ttgaaggtgg gtatgggagt ggatgtgagg gtaagtgagt tgtttggtgt   19740
   gattgttttt aatgtaggag ggtgtgttta gtggtgtgtg tgtattcgtg taagtgttgt 19800
   agtatttgtg tgcgtatttt ttgttatgta taaggtgtgt ttgtgatttt tgtgtgtagt 19860
   ttttgtatat gtgtgtatgt atgtgtaatg gagatttatt tgtagtgtgc ggatgtgtat 19920
   gtgggtgtgg gtgtgggtgt gtgggtgtgt gtgttggcgg ggagtggtta gtgtttggat 19980
   gtggtaaagg gatgaagggg gtgatagttt ttttttttgg aggttggtgt ttttggaata 20040
   tatggtgggt ttagttttta tttttgagtt tagagtttaa ttttaatggt tttaaggtgg 20100
   ggtaatggaa tgggggttgg agttgggttg ggaaggaata ttagaggggg tatttatata 20160
   ggtaggatgg ggtaggatat ttttgattag ggggttagag gtttaaagtt gtgttttagg 20220
   cgtagaggta tattttgtag tagtgatagt tttttttttt ttaggagagg tcgtgtattt 20280
   ggttcgagat ttcggttacg tttgtgagac ggagttttta gttaaggtag ttgtcgagta 20340
   tttgtgtcga tagtacgttg attcggggga gttgtatagt cgtaagagta tgttgttggt 20400
   tgttaagtga gtgagggtat tttgtatagg tatacgtggg tgttatgtat agatagatat 20460
   tatgtatggg tataatggat attattgtgt atatgagtag tgggtatata tgcgtatttg 20520
   cgtattattg tgtatacgag tagtgggtat atatatatac gtgcgtatta ttgtgtatat 20580
   tagtagtgag tatatacgta tgtgtgcgtt attgtgtacg tgagtagtgg gtatatatac 20640
   gtacgtgcgc gttattgtgt atacgagtag tgagtatata tacgtatttg cggtttatgt 20700
   ggtattatat atgggtatat ttaggttttt tgtttaggtt tttttgggta tatgtgtgta 20760
   tgttatttgg gtatatttgg gtgttatata tgggtatatt tgggtattat gtttattatt 20820
   tagatatata tgaatatttt ttataagttt ttggagtata tatagatatt atatgtgaat 20880
   atatttgggt ttttgtatta ggaatagtga tgttatatat tggtttatat aggtttacgt 20940
   atgggtttta tatttatata tatttttagg atattattag ttttattttt tatcgtaggt 21000
   ttgaggtttt tttttttagt tttgtttatt aattttttgt tttagtgacg tttattaggt 21060
   tattaagtta tatttgattg ttttgtaaat tttattgtgt tttttttatt atcgtttata 21120
   tgttgttatt tttatttagg tttttatttt atttttggat ttttatttta gagcgttttt 21180
   gtttttaatt tttttgtacg tgtattcgtt ttattgtata agaattttta atggttttcg 21240
   ttgtattgat ttttaaatta ggtttgcgag ggtattttag ggtttgttta ataaattagt 21300
   attttattaa gatttagttt tgtttttgtt ttttgagatg gagtttcgtt tttgtttttt 21360
   agattggagt gtagtggcgt aattttagtt tattgtaatt tttatttttt gggtttaagt 21420
   aatttttttg ttttagtttt tcgagtagtt gggattatag gcgtttgtta ttaggtttag 21480
   ttaatttttg gtattttaag tagagatggg gttttattat gttggttagg ttggtttcga 21540
   atttttggtt ttaggtgatt tattcgtttt ggatttttaa agttttggaa ttataggtgt 21600
   gaattattat tttcggtttt aagatttagt tttttaggtt aggtaaggtg gtttatattt 21660
   gtaattttag tatttttgag aggttgaggt tggtggatcg tttgagttta ggaattggag 21720
   attagtttgg gtaatatggt gaaattcggt ttttagtaaa aatataatag ttaggtgtgg 21780
   tggtgtattt ttgtagtttt agttatttgg gaggttgagg tgggaggata atttgagttt 21840
   gggaggttaa ggttgtagta agttatgatt gtatcgttgt attttagttt gcgtgatata 21900
   gtgagatttt attttaaaaa aaaaaaaagg cgaaagattt agttttttta aaagagtttt 21960
   tgttttagtt tatttgggtt gttataataa aatattataa attgggtagt ttataaataa 22020
   tagaaatttt tatttattta tttattgaga tagaattttg ttttgttgtt taggtcggag 22080
   tgtagtggtg taattttagt gtattgtaat ttttagtttt tgggtttaag taattttttt 22140
   gttttagttt tttgagtagt tgggattata ggtgtttgtt attatatttg gttaattttt 22200
   gtatttttag taaaggtttt attatgttgg ttaggttggt tttaaatttt cgattttata 22260
   tgatttattt gttttgggtt ttttaagtgt ttggattata ggtgtgagtt attacgttta 22320
   gttaataaga gatatttatt ttttatagtg ttggaggttg ggaagtttaa ggttaaggta 22380
   gatttcgtgt taggtgaggg tttgtttttt ggtttataga tagtattttg ttgtgtgttt 22440
   ttatattgta gaagggagaa ggggttattt tttggttttt tttataataa gggtattaat 22500
   tttatttatg acggttttat ttttatgatt taattatttt ttaaagtttt tattttttaa 22560
   tattattatt tagtggggta gaattttaat atacgaattt tggggaatat aagtatttat 22620
   attatagtag ttttattgta agaaaagata gagagaggag ggggatagag aaagagagaa 22680
   agaaagagag agagattgag tgtagtggtt tatgtttgta attttagtat tttgggaagt 22740
   tgaggtgatt agatcgtttg agtttaggag tttaaggtta gtttgggtaa tttggcgaaa 22800
   ttttatttgt attaaaaaaa aattagttag gagtgatggt gcgtatttgt agttttagtt 22860
   attcgggagg ttgaggttga gattgtagtg agttgggatt atattattgt attttagttt 22920
   gggtgataga gtaagatttt gtttcggaaa aaaaagaaaa gagagagata aatttgagaa 22980
   ttattagttg gtaggttaaa gtttaaattt ttttgagggt tttatagttt ggtttatata 23040
   aattttattt atttttgtta attttgtttt tatatttttg ggtttgtttt cggtttagaa 23100
   tatttagttt ttttatttaa tatagttttt taaattgata attttatttt tttgtttttg 23160
   aggttttttt taagtgttta aggtagaaga ggaagtatag atttttggga ttgttaaatg 23220
   gagagatttt tgaggttgtt tatattgtgg ttggagaaat ttagttagga cggggtttat 23280
   ttcgtttatt tattagttta tttattaagt attttgtggg ttttagttat gtgtaagata 23340
   ttggttggtt tttatggtga tataaagatg aatgatgagt gatttttttg tttgagttat 23400
   atataggacg tataaaggaa tgaataatga atggttgtaa attagagtta ggtttaaatt 23460
   ttagttcggt tattttgata ggttatttaa tttgttcgaa attagttttt ttgttcgtat 23520
   gatggggata ataatagtat agatttgtta tgaagattaa gatgatgtag atagtgtggt   23580
   tggtatagta tttagttttt aattatattt attgttagtt attattttcg gtagattttg   23640
   gattgttttt tgatagtttt aaagataaag ttttgtgaag tattgagtgt ttggggtggg   23700
   agagggaatt taggaaagtt ttttatataa ggtagtaagt agttgagttg tattttgaat   23760
   gaatatgggt tagatattta aaaaagaaat attatagggt gatcgatatg tgttgtttat   23820
   tagttgtgtg atattgggtt agttaaatga tttttttgaa ttttagtttt tttttttgta   23880
   aaaaatagta tggttattat ttgtagattg ttgggagggt tagtaattgt gtagttaatg   23940
   tttgtaaaat gtttagtatg tagtaggttt ttttttgggt ttagattttt gttttttaat   24000
   ttttgatttt ttttttcgta ttaggtagat ttgtaaggag tttgtagatt tgatggttta   24060
   ggatcgttta tcgttgggta atagtcgttt agtatttatt ttggagttcg gagtatagag   24120
   ttgtttgata tattttagtt ttattattta tggtttcggt gggtttgtta tttgtgttgt   24180
   ttttattgtt ttttagaatt atttgttgga gttatttaag gggttggata agatgttttt   24240
   aagtagtgtg ggtagtgggt atggtgaaat taaggtttcg gagaaggatg ttaagtatcg   24300
   gaaataattg ttttttttat tttattttta aggggttttt aggttttgaa atagggattt   24360
   agtttttggg ggtgggtttg gaaggattga aaggtgggat tagagttagg ttagaaagag   24420
   aatatttatt tagagatttt agagttgggg atttggtttg gagtaaggga gggtggtttt   24480
   tttgtggtgg tgtgttggta agttaagggt ttaggtattt gttttatgtg tgtaattttt   24540
   tgatttttga tggttgagaa gggtttggat agaaaattga tatgaaaaga tttggtttat   24600
   ggggtagagt tttttttatt agcgtggttg ggtggtcgtg ggtgttttta gaggttaaag   24660
   tttttgtgtt ttttattggt ggtaggagga aaattgataa attagaggtg ttattgagga   24720
   gttggtgttt tttattttag aattttttat ttttagaaaa ggggtgttgg aaggaggttt   24780
   tagtggattt tttgtatttt ttttattttt agagagaagt gggtaggagg ggtttttaag   24840
   gaataaagaa gataaagtat aaatcgtaga atttgaattt aggttgttgt ttattatagt   24900
   tttgttgttg tttgttttat ttatttatgt atgttgtaat taaatttgaa attttaaaa    24959
<210> 4
   <211> 24959
   <212> DNA
   <213> Artificial Sequence
<220>
   <223> Synthetic construct: chemically treated genomic DNA (Homo sapiens)
   <400> 4
   ttttaaaatt ttaaatttaa ttataatata tataaataaa taggatagat agtaatagga      60
   ttatgatgag tagtagtttg gatttaagtt ttgcgatttg tgttttattt tttttgtttt     120
   ttgaggattt tttttgttta tttttttttg agagtaggaa gggtataaag agtttattgg     180
   ggtttttttt tagtattttt tttttggggg taggagattt tggaatgagg ggtattaatt     240
   ttttagtagt atttttgatt tattaatttt ttttttgtta ttagtgaaaa atataaaggt     300
   tttggttttt agaagtattt acggttattt agttacgtta atggaaaggg ttttgtttta     360
   tgagttagat ttttttatgt taattttttg tttaaatttt ttttaattat taaaggttag     420
   aaaattgtat atatgagata aatatttggg tttttaattt attaatatat tattatagag     480
   aggttatttt tttttatttt aagttagatt tttaattttg ggatttttgg atgaatgttt     540
   tttttttggt ttgattttaa ttttattttt tagttttttt aggtttattt ttaagagtta     600
   agtttttatt ttagggtttg ggagtttttt agggatgggg tgggagaagt agttattttc     660
   gatgtttggt attttttttc gaagttttgg ttttattatg tttattgttt atattgttta     720
   gaaatatttt gtttagtttt ttgagtgatt ttagtaaata gttttggaag gtagtgaggg     780
   tagtatagat ggtaggttta tcgaagttat gggtgatgag gttaaagtgt gttaagtagt     840
   tttgtatttc gggttttagg atgagtgttg ggcggttgtt gtttagcggt gagcggtttt     900
   gagttattaa gtttgtaaat tttttgtaga tttgtttggt gcgagaagga gggttagagg     960
   ttggaggata aaagtttgag tttagagaag gatttgttat atgttaggta ttttatagat    1020
   attagttata taattgttga tttttttaat agtttgtaaa tgatgattat attgtttttt    1080
   atagaaaagg aaattaaggt ttagagaagt tatttgatta gtttagtatt atatagttaa    1140
   taaatagtat atgtcgatta ttttgtaatg tttttttttt ggatgtttgg tttatattta    1200
   tttaaagtgt agtttagtta tttattattt tatataggaa atttttttgg attttttttt    1260
   ttattttaaa tatttaatgt tttatagaat tttgtttttg aagttattag gaaataattt    1320
   agaatttgtc ggaaataata attagtagtg aatataattg aaggttggat gttgtgttaa    1380
   ttatattgtt tgtattattt taatttttat aataagttta tattattatt atttttatta    1440
   tacggataag aaaattggtt tcggataggt tggatgattt gttaaggtga tcgagttggg    1500
   atttgaattt agttttagtt tgtagttatt tattatttat ttttttgtgc gttttgtgtg    1560
   tggtttaggt aagagaatta tttattattt atttttgtgt tattatgaaa gttagttagt    1620
   attttatata tgattgagat ttataaagtg tttgatgaat gagttgataa gtgaacgagg    1680
   tgagtttcgt tttggttagg tttttttagt tatagtatga gtagttttag aggttttttt 1740
   atttggtagt tttaggagtt tgtgtttttt tttttgtttt gggtatttga ggaaagtttt 1800
   aaaggtagga gggtggagtt gttagtttga aggattgtgt taggtgggag gattggatgt 1860
   tttaagtcgg aaataaattt aaggatgtga ggatagggtt ggtagaagtg ggtggagttt 1920
   gtgtgggtta gattgtgggg tttttaaaag agtttgaatt ttagtttatt aattggtagt 1980
   ttttagattt attttttttt tttttttttt tttcgagata aggttttgtt ttgttattta 2040
   ggttggagtg tagtggtgtg attttagttt attgtagttt taattttagt ttttcgagta 2100
   gttgggatta taggtgcgta ttattatttt tggttaattt ttttttagta tagatggggt 2160
   ttcgttaagt tgtttaggtt ggttttgaat ttttgggttt aagcgatttg attattttag 2220
   ttttttaaag tgttgggatt ataggtataa gttattgtat ttagtttttt tttttttttt 2280
   tttttttttt tttatttttt tttttttttt gttttttttt gtagtgggat tgttatggtg 2340
   tgaatgttta tgttttttaa aattcgtatg ttgaaatttt attttattag gtgatagtat 2400
   taggaggtga ggattttggg aggtgattag gttatggagg tggagtcgtt atgaatggga 2460
   ttaatgtttt tgttataaaa gaagttagag agtaattttt tttttttttt ataatgtgag 2520
   gatatatagt aaggtgttgt ttatgagtta gaaagtaggt ttttatttga tacggaattt 2580
   gttttgattt tgaatttttt agtttttagt attatgagaa ataaatgttt tttgttggtt 2640
   gggcgtggtg gtttatattt ataatttaag tatttaggga gtttaaggta ggtggattat 2700
   gtgaggtcgg gagtttgaga ttagtttggt taatatggtg aaatttttat taaaaatata 2760
   aaaattaatt agatgtggtg gtaggtattt gtaattttag ttatttagga ggttgaggta 2820
   ggagaattgt ttgaatttag gagttggagg ttgtagtgta ttgagattgt attattgtat 2880
   ttcggtttgg gtaatagaat aagattttgt tttaataaat aaataaataa aaatttttgt 2940
   tgtttataag ttatttagtt tatggtattt tgttatagta gtttaaatgg attaagataa 3000
   gaattttttt ggaagaatta aatttttcgt tttttttttt ttttgagatg gggttttatt 3060
   atgttacgta ggttagagta tagcgatgta attatggttt attgtagttt tgatttttta 3120
   ggtttaggtt atttttttat tttagttttt taagtagttg ggattatagg agtgtattat 3180
   tatatttaat tgttgtattt ttattagaga tcgggtttta ttatgttgtt taggttagtt 3240
   tttaattttt gggtttaagc gatttattag ttttagtttt ttaaaagtgt tgggattata 3300
   gatgtgagtt attttgtttg gtttgaaaaa ttaaattttg gggtcgggag tggtggttta 3360
   tatttgtaat tttagaattt tgggagttta aggcgggtgg attatttgag gttaggagtt 3420
   cgagattagt ttggttaata tggtgaaatt ttatttttat ttaaaatatt aaaaattagt 3480
   tgggtttggt ggtaggcgtt tgtgatttta gttattcggg aggttgagat aggagaattg 3540
   tttgaattta ggaggtggag gttgtagtga gttgagattg cgttattgta ttttagtttg 3600
   ggaaataaga gcgaaatttt attttaaaaa ataaaaataa aattaaattt taatagaata 3660
   ttagtttgtt aaataggttt tgaggtgttt tcgtagattt agtttgaaaa ttaatgtagc 3720
   gggaattatt gaagattttt atgtagtggg gcgggtgtac gtgtagggag gttggaaata 3780
   gggacgtttt ggggtgaagg tttaggagtg agatgaagat ttgagtagag gtggtagtat 3840
   gtgaacggta gtgagggagg tataatggga tttgtagaat agttaggtgt gatttggtgg 3900
   tttgatggac gttattggag tagagggttg atgggtaagg ttagaagagg gagttttaga 3960
   tttgcgatgg ggagtagggt tggtggtgtt ttgggagtgt atgtgagtgt aggatttatg 4020
   cgtgagtttg tgtgagttag tatgtgatat tattgttttt gatgtaggag tttagatatg 4080
   tttatatgta gtgtttatgt gtgttttagg agtttgtgaa gagtgtttat gtatgtttga 4140
   gtgataaata tgatgtttaa atatatttat atgtggtatt taagtgtgtt taggtggtat 4200
   gtatatatat gtttaggaaa gtttaagtag ggagtttagg tgtgtttatg tgtggtgtta 4260
   tatgggtcgt aggtacgtgt gtgtgtttat tgttcgtgta tatagtggcg cgtacgtacg 4320
   tgtgtgtgtt tattgtttac gtatatagtg gcgtatatat acgtgtgtgt ttattgttga 4380
   tgtatatagt ggtgcgtacg tatgtgtgtg tgtttattgt tcgtgtatat agtggtgcgt 4440
   aaatacgtat gtgtgtttat tgtttatgta tatagtggtg tttattgtgt ttatatatga 4500
   tgtttgtttg tgtatggtat ttacgtgtgt ttgtgtaggg tgtttttatt tatttggtag 4560
   ttagtagtat gtttttgcgg ttgtgtagtt ttttcgggtt agcgtgttgt cggtataggt 4620
   attcggtagt tgttttggtt gggaatttcg ttttatagac gtaatcgaag tttcgggtta 4680
   ggtgtacggt tttttttggg gggaggggag ttgttattat tgtagggtgt atttttgcgt 4740
   ttggaatata gttttgaatt tttgattttt tggttagggg tattttgttt tattttgttt 4800
   gtgtagatgt tttttttgat gttttttttt agtttagttt tagtttttat tttattgttt 4860
   tattttgggg ttattagggt tgggttttga gtttagagat gggagttggg tttattatgt 4920
   attttagagg tattaatttt taggggaggg ggttgttatt ttttttattt ttttattata 4980
   tttaaatatt ggttattttt cgttaatata tatatttata tatttatatt tatatttata 5040
   tatatattcg tatattgtaa gtaggttttt attgtatatg tatgtatata tgtataagga 5100
   ttatatatag aaattataaa tatattttat atatgataga gaatacgtat atagatattg 5160
   taatatttat acggatatat atatattatt aaatatattt ttttgtatta gagatagtta 5220
   tattagataa tttatttgtt tttatattta tttttatgtt tatttttagg ttttatttat 5280
   tttttattag cgaagttagt agcgttatat tggcggtttt gcgacggtta gttggtaggt 5340
   tgagtttaat tttttttaat cgttttcgta aatatcggtt tttatttttg gatttggttt 5400
   ttgatatgta tagagataga tagatatata attatgaaga taagagaaac gtatggaagt 5460
   taagtggttg ttaaagggtt ttgttgtttt tattagtttt tgttgaggtt tgggattggt    5520
   tttggggatg ttttgaggtt ttttttagta agggcgtaat taatttttat ggttttgtag    5580
   ttaagataga ggtagggggg ttagtattta gttttaggag ttttttagtt tattttaatt    5640
   tttttaatag taggggtagt tagggtgggg tattttgggg attttagtat attagagggt    5700
   tatatatatt tattagggga tatatagatt tatatgtaga gatacgtagt gatatataga    5760
   aagagagata tatggggaga tataggtata tatatagtta tttggtgatt tttttatata    5820
   tatatatata tatacgtgaa agaatagaga tatatagaat taatagagat agatatgtag    5880
   agatatagat ttagatattt agggatatag agatatagga ttatattaag ttagggtttt    5940
   aggaatatat gtatagatat ttagggattt atagagattt tttaagacgt taggagtaag    6000
   gagataattt ttatgattgt atatatgtta tttttatata gtggtataga tatgtaaata    6060
   tttatatgat tagttttaga tgtataggta attatatata tatatatata tatatatata    6120
   tatatatata tatatatata gagtaggata tattttaggt tatatatttt attagaagaa    6180
   atataggata tttatattta tggaaatata tgtttttggt tattcgttat tatagatata    6240
   tgtatacgta tatatatgtt tatattattt ttagatttat ttaagtttat ggttttgaag    6300
   tagaaatatt ttttttaggt ataaagatat gtatagagag agagagatat aggtttagga    6360
   agatgaatta tggattatta tgttttatat tttaaaatat atagggttta tgttgtcgcg    6420
   tagaatgggt ttcgggtttt ggggtttatt ttttagaaga gggggtgagg atggtttttt    6480
   ttttttttta gatttagttt cgttttgatt agtgtggatg agttttttat tttttttttg    6540
   tgtttaggtt tttttttttg tttatttttt tattttagat ttatagatat gtggtagtta    6600
   ttagggattt gggtcggttt ttataatatg tataatttag agttttcggg gttttttttt    6660
   ttagggagat tgaggtagag ggtttaggtg agattggttt tttttttgat gttttgagaa    6720
   ggtttgaaga ggttagggat ttaattaggc ggggaattgt gggttggttt ttttatttgc    6780
   ggaggatatt ttttaggagg gaggcgttga ggtattcggg aggcgagagt cgtcgttgta    6840
   ttttttttat cgttattttg tatttggacg ttgagttgag tagtgaaagt cggtcgggta    6900
   cggagtagaa gattttatta ggatttgtga tgtcgtttat taggttgttt ttggttaagg    6960
   agagggttga gaggttgttg gttttggagg ggatgggtat tgtggagaag agggaaacgt    7020
   tgagttagtg gttgtcgtat atagttttat ttaagggagg gtttggtatt tttgttttat    7080
   gaagggagaa attgagttat agtgaggtga aagtgattcg tttaagtttt tttaattgat    7140
   ggatgtgaat ttagagattg aatttcgagt tgtttggttt ttgaatttgt gtttttttta    7200
   ttgaattagg ttggttttat tttaaattta aatagtttta gcggaatttt ggtgttttag    7260
   cggatgttat tagagaggga tttggatgta aggatatgag tgtgagttgg aggtaggatt    7320
   tttggggttt tgtagtaggg ttaggttagg gttgggtaat tatagggagt ttttaaattt    7380
   ttttttttta ttcgtgattt ttacgttttt tttttttgtt tttttatttt attttatttt    7440
   tgtatcggtg aggggtttgg agattgtggg ttttatattt tggagatagg gaggttattg    7500
   ttttgttaat gtttttagtt ttggtttttt ttttttgtta agttattttg cgaatgttaa    7560
   aggagattag gagataggtt ttatattttt tttttaaata ttttttaatt atttattaat    7620
   tgtttgaaga ttagagaagg aatttgagaa gttaattttt tttttttttt tttttttttt    7680
   tttttttttt ttgagatgga gtttcgtttt gttattaggt tggagtgtag tggtattatt    7740
   tcggtttatc gtaatttttg tttttcgggt ttaagcgatt tttttgtttt agttttttga    7800
   gtagttggga ttataggtgt ttattattac gtttggttaa tttttatatt tttagtagag    7860
   atggggtttt attatattgg ttaggttggt tttaaatttt tgattttatg atttattcgt    7920
   ttcggttttt taaagtgttg ggattatagg tatgagttat cgtgttcggt tagaagttaa    7980
   ttttttaaaa tttatttaga tatttttggt tataagagaa agaaagtttt ttttgtagtt    8040
   taatttttat ttttttgttt agttttaggg gaagggagaa tatcgaagta tttgtttttt    8100
   tagcggaatt ttttggtttt tttttttttt aaggaggtga tagttttggt agataaggtt    8160
   tcgtgtgtat attaggagtg aggttcgggt gtggagtttg ggattaatag ttattgagta    8220
   tttattgttg tgtgttttcg tatataaatt tatttagttt ttggaataat ttataagggg    8280
   ttatatttat tattattttt attttataga tgaggagatt gatgtttaga gaggtgaagt    8340
   aatatgtttg agattatata gttggtagac ggagaatcgg tagtaaattt aagattgtag    8400
   ttttaaaggc ggtgttgtta gttttttagt tatttggtaa tattggttga gtatttttat    8460
   gttaagaatg gtgttagggt tgaggaatat aaaagtagga aaggttagtt tgattcgtag    8520
   tttggtagaa cgtttagttt tttttttttt ggataaattt ttttattttt atttagtagt    8580
   ttagttagaa atttttgtga tattttttgt aatttttttt tttagtatat tttatttatt    8640
   attatatttt atttattatt atattttatt tattattaga ttttatagtt tttttttttt    8700
   gtttttttta tttttgttta ttttacgatt tttatttttt tatgtgtttt gtatgtggtt    8760
   atttttttgt atgtatatag atataggttt tttttttatg tgaatttttt tatagatttt    8820
   gttttggtta ttttgtttta gtttagaggg atttttttat ttgaatgtat tattttgtag    8880
   ttaaaaattt agtttaaaat ttattagagt tcggttagtt ttttgagaat taaattttag    8940
   ttttagagtt agataaaatg atgttatttg ttaatgagtg ttaattataa ttttatttgt    9000
   taatgagtgt taggtgttga aagtttaaag aggttaaagt gggatagtaa gtaaggggta    9060
   gaaatgaatt tttaattttg agtattgatt ttagagttta tagttttaat tattatgtta    9120
   taatggtttt ttttattttt ttttgtgttt tggttttgtt tgtttttttt gttgggtttt    9180
   tatttgttta ttaggtttta gatttaagtt gattaagaga aatataatgt gagttatatg    9240
   tatatattta tattttttag tgtttattat tttttttttt gagatagggt tttgttttgt     9300
   tgtttaggtt ggagtatagt ggtataatta tagtttattg taattattat tttttaggtt     9360
   taagtaattt ttttatttta gttttttaag ttgttgggat tataggtgcg tgttattatg      9420
   tttgggtaat ttttgtatgt tttatagaga tggggtttta ttatgttgtt tagattggtt     9480
   ttgaattttt ggatttaagt tatttatttg tttgttttgg ttttttaaag tgttggaatt      9540
   aaaggggtga gttattaggt ttggtttata ttttaaatag taaaaagaaa tatgtgaagt     9600
   taattttaat taatataata gtaaataata tattattatt tagtatattt tatttagttt     9660
   aatatattaa aaatagaatt attttaatat gtaattaata ttttaaaaat tattagtgag     9720
   atattttata ttgtttgaaa ttagttttta aaatttaatg gatattttat gtttaagcgt     9780
   attttaattt agattagtta tattttgggt gtttaagagt tattcgtggt tatagtggtt     9840
   atttggtgga tagtaaagtt ttaggtttta agggaggagg ggtttaatag gtaatatagt     9900
   aagtgggttg tgggggaggt tggtaggtta gaggggtatt ttatgtaaag tttcgttaag     9960
   taagatataa agtttttggt ttgtttgtgt ttttgttttg ttttgttgat ttttgaaatt    10020
   aggattttgg atttttttgt gaaatttttg gagttttaag tattggtaat gatttaaaga    10080
   tttaaaaatt tagtataggt taaatggaat aagtgtgaag tgttttttgg gatgtagtga   10140
   tttttagggt gtgagttttt tgagttttat agtgtttttt cggtttttag ggttttgttt   10200
   ttattttatt gagtttaggt attaattttt gtgtagagtt tttttttttt gttttttagt   10260
   ttttttttat ttttattttt ggttttatcg tattgaattt ttttggtttg tttttatggg    10320
   tgtttttttt attaagtatg agtgtgtgga gggtagggtt ttggtatagg tggttatgag   10380
   tgaatatgaa ataagttaat gatgtaaggg ttttgggaat tcgaattgaa ttatattgta    10440
   tgtataaaaa aattatattt cgagtttgat tttaaataaa taaaattata aatgaggtta    10500
   ggtacggtgg tttatatttg taattttagt attttgggag gtcgaggtgg gaggattgag    10560
   tttaggagtt taagattagt ttgggtaata taatgagatt ttgtttttat aaaaaatata    10620
   aaaattagtt cggcgtgatt gtatatgttt gtagttttag ttattttgga ggttgaggta    10680
   ggaggattgt ttgagtttaa gaggtagagg ttgtagtgag ttgattatgt tagtttgggt    10740
   ggtatagtga gattttgttt taaaaataaa aataaaaata aaaataaaaa attgataaat    10800
   taaaaataaa aagaagggta aagtaattgt tttttttttt attatttttt attagaagta    10860
   ggggttggag aaaaaggatg agtagttttt tttatttaat tgttattaaa atttagggtt    10920
   taggttggtt gagtatggta atttatattt gtaattttaa tattttggga aattgaggta    10980
   ggaggattat ttgagtttag aagtttgaga ttagtttggg taatatagtg agattttatt    11040
   tttataaaaa aaaatttttt taagttagtt aggtatggtg gtatgtgttt atagttttag    11100
   ttgttcggaa ggttgaggtt atagaatgat tgagtttagt ttgaggttgt agtgagttat    11160
   gattgtatta ttgtatttta gtttggacga tagagcgaaa ttttatttta aaaaaaataa    11220
   aaaaggatga ttgtatataa gaattgtatg tttttgtgat aaatatagta atgaggatga    11280
   gttggagtat ggaggaatgt ggtagtttag atatggatat ttgtaagtgt ttttttttgt    11340
   tgatgttaga tttatgtaaa tataggtgtt gttagatata agggtagaga tgatattgtt    11400
   tatgtgtttg tgtatatgag tgattgattt tgttgttttt tttatttgtt tttttttttt    11460
   ttagagtttt tttttttgaa aatggtaaga gggatggaat ttatggttag ggaagtaggt    11520
   cggttataga agggtagtgg tggaggtagg aattaaatta gtatataggg taaagtagag    11580
   atattaaagt ttgtttttta tatatataga ataggatttt ttgtggtgat ttttgaagtt    11640
   gaagtgattt aatggtgttt tgttggttat atatgtagaa gttatttata tatatatata    11700
   tatatatata tatatatata tatatttttg tagatataag agttttcggt tatttaattt     11760
   gttagataag gtattttgtt cgtaggtggt agatatggtt tgatttttga atttaggtaa    11820
   gtgtgtaata gattttttaa gaagttttgt taaagttgta agtttttttt agaagaaaat   11880
   atggaaggtt aggcgttttg gtttatattt gtaattttat tattttggaa ggtaaggcgg    11940
   gtggattatt tgaggttagg agtttgagat tagtttggat aatatgggaa aattttattt    12000
   ttattaaaaa tataaataat aataataata ataatagaaa atttaggaga aaaattttcg    12060
   tgattttagg ttgagtaaag agtttttaga tataatatta aaagtaaggt ttataaaaga    12120
   aaatgatagg ttaggtgtgg tggttgattt ttggaatttt agtattttgg gaggttgaag    12180
   cgggtagatt atttgaggtt aggagtttaa gagtaatttg gttaatatgg tgaaattgtt    12240
   tttattaaaa atataaaaat tagtttagta tggtggtatt tatttgtaat tttagttatt    12300
   tgggaggttg aggtaggaga atcgtttgaa ttcgggaggc ggaagttgta gtgagttgag    12360
   atagcgttat tgtattttag tttgggcgat agagtgagat tggttttaaa aaaaaaagaa    12420
   agaaagaaag aaagaaaatg atgataaatt ggattttatt aaagtttaaa atttttgtat    12480
   tttaaaagat attattaaga aaatagtatg gtatggtagt atgagaatgt agttttagtt    12540
   attcgagagg tttaggtagg gggattattt gagtttagga gttcgagggt gtagtgtgtt    12600
   atgattttgt ttgtaaatag ttattaaatt ttagtttgaa taatttagtg agatttattt    12660
   ttaaaaagtt ttttaattaa aaaaaaaaaa gagagtagtt gtttattttt gtaattttag    12720
   tatttaggga ggttaagata ggaggattat tggaagttag gagtttgaga ttagtttggg    12780
   taatatagcg ggattttgtt tttatgaaaa aaaaaaaaaa gataattttt aaaaatgtaa    12840
   aaaaaaaatt aaaattttaa aaatagaaaa gaaaacgata aattataaat tggaagaaaa    12900
   tatttttaaa ttatatattt gataaaggat tgtatttaga atatattata taaagaatta    12960
   ttataattta ataataagat agtaatttaa atagaaaatg gtttaaatat gtaaatagat    13020
   attttattaa aaatgagaga tgaatggtta aataagtata tgaaaaagtt tttaatatta 13080
   ttagttatta gggaaatgta aattaaaatt atattgagat attatttgat ggttataata 13140
   aaaaagatag gggttaggta cggtggttta tgtttgtaat tttagtaatt tgggaggttg 13200
   aggtggatgg attatgagat taggagttcg agattagtta gtttgtttaa cgtggtgaaa 13260
   tttcgtttta attaaaaata taaaaattag ttgggtgtgg tggtgcgtgt ttataatttt 13320
   agttattggg gaggttgagg taggagaatt gtttgaattt aggaggtaga agttgtagtg 13380
   agtcgagatt gtgttattgt attttagttt gggtgataga gagataattt attttaaaat 13440
   aataaataaa taaaaaaggt taggtatggt ggtttatatt tgtaagttta gtattttggg 13500
   aggttaaggc gggtggatta tttgaggtcg ggagtttagg attagtttga ttaatatgga 13560
   gaaatttcgt ttttattaaa aaaataaaat tagtcggacg tggtgttacg tatttgtaat 13620
   tttagttatt taggaggttg aggtaggaga attatttgaa tttaggaggt agaggttacg 13680
   gtgagtcgag attattttat tgtattttag tttgggtaat aagagtgaaa ttttatttta 13740
   aaaaaaaaaa aagaaaaaaa agataatttt aagtgtagga tgtggaaaag tggaattttt 13800
   atatattgtt ggtgagaatg taaaataata tagttatttt agaaaatagt ttgatagttt 13860
   tttaaaaatt aaatttaaat cgattatata atttagtaat tgtattttta ggagtttatt 13920
   taaaagcgtt aaaaatatat ttttatagta aggtatgtat gtgaatgttt atagtaatat 13980
   tatttaagat agttaaaaag tggaaataat ttaagtgttc gttaattgat taatggataa 14040
   ataaaatatg ttatattcgt ataatggtat attatgaagt aataaaaaga aataaattgt 14100
   tgataatagt ttgttttttt ttattgttgt tttttttatt tagtataatg tttggggcga 14160
   gttttaaaaa tattatgtta agtaaaagac gtttgatgta aaagtttgta tatttatata 14220
   ttttatttat agaatatgtt tagaatatgt aaatttatgg atagaaagta gattagtggt 14280
   ggtttaaggt ttggggtgag aatggagagt agttataaat gggaattttt ggggatggta 14340
   gaaatgtttt aaaatggatt tttttgtggt aatggttata taattttata tttattaaaa 14400
   attattaaat tatattttta taatgtgtaa attttaggta tgtaaatttt attttaaaaa 14460
   atcgttaaaa aatgtaagtt ttagggttat atttttaggg gtttttatgt agttaatgtg 14520
   gggtggtgtt taagaaattt gttttttttt ttttgaggta gagtttcgtt ttgtcgttta 14580
   ggttggggtg tagtggcgta attttagttt attgtaagtt tcgtttttcg ggtttacgtt 14640
   atttttttgt tttagttttt cgagtagttg ggattatagg cgttcgttat tacgtttagt 14700
   taatgttttt tttggtttgt ttttgttttt ttagtagaga tggggtttta tcgtgtttgt 14760
   taggatggtt tcgatttttt gatttcgtga tttattcgtt tcggtttttt aaagtgttgg 14820
   gattataggt gtgagttatt gtatttggtt aaaatttgta tttttaataa gtattttttg 14880
   ggggttttga tgtatgtggt tttttggatt atatttaggg tattattggt ttaaagtttg 14940
   aaggggttgg agtaagagga aggtagacgg gaggagtagt gatttgtttt ttggtttata 15000
   ttggggtata gtatgtgtaa atgttttttt tagagttttt ttattttttt tgttgggatt 15060
   tgtcgattaa gttttttttt tattaattgt tagagggttt tttttttttt ttttttttaa 15120
   gatggagttt tgtttttgtt gtttaggttg gagtgtaatg gtataatttt ggtttattgt 15180
   aattttcgtt tttcgggttt aagcgatttt tttgttttag ttttcggagt agttgggatt 15240
   ataggcgtgt attattatgt ttggttaatt ttttttgtat ttttagtaga gatgatattt 15300
   tattatgttg gttaggttgg ttttaatttt ttgattttag gtaatttatt tgtttcggtt 15360
   ttttaaagtg ttgggattat aggtataagt tattgcgttc ggtttagggt tttttttata 15420
   tataattttt attttttatt tttttgtttt aaggtttttt atagttttta ttatttttag 15480
   gtaaagttta aattttattt gtatattttt taagttgttt tttggggtag agagtagttt 15540
   gggaggtagg tgagaaaggg ttgtggaagt gtaagtttag taaatttttg atattttgtt 15600
   tatcgtggat ttttgtatta tttttattta aaaaggttaa gcgtggtggt ttacgtttgt 15660
   aattttagta ttttgggtgg ttgaagcggg taggttattt gaggttagga gtttaagatt 15720
   agtttggtta atatggtgaa attacgtttt tattaaaaat ataaaaatta gttgggcgtg 15780
   gtgataggta tttgtaattt tagttatttg ggaggttgag gtacgagaat ttgagaggta 15840
   gaggttgtag tgagttgaga tcgtggtgtt gtattttaat ttgggcgaga gagtaaaatt 15900
   gtgtttcgaa aaaaaagatt tttttttttt ttgagataga gttttgttgt tgttgtttag 15960
   gttggagtgt aatggtgtga ttttggttta ttgtaatttt cgtttcgtag gtttaaggga 16020
   tttttttgtt ttagttttcg gagtagttgg gattataggt gtttgttatt ttatttagtt 16080
   aatttttgta tttttagtag aaacggggtt ttattatatt ggttaggttg gtttcgaatt 16140
   ttagatttta ggtgatttat tcgtttcggt tttttaaagt gttgggatta tatggtgtga 16200
   gttatcgtcg ttcggttttt tttttttttt tttttttttt tttttttttg agatagtttt 16260
   tttttgttgt ttaggttgga gtatagtggt atgatttcgg tttattgtaa tttttgtttt 16320
   ttgggttaag taattttttt gttttagttt tttgagtggt tgggattata ggttagcgtt 16380
   attatattgg gttaattttt gtatttttag tagagatggg gttttattat tttggttagg 16440
   ttggtttcga atttttgatt ttaagtgatt cggtttttta aagtgttggg attatagacg 16500
   tgagttattg cgttcggttt atttattttg attattgagt tttttggtat ttttttaaat 16560
   tttgttttag aagttagtat tttatttatt ttatttatgt tttggttgtt tagagattgt 16620
   ttgggaagtg gagatttttt ttagtttttt tttttttttt tagggttaaa gatagtaagg 16680
   aatagggcga ttgtttatat tagtttttgt tttagagatt ttttgagatt ggatagttga 16740
   gttcgtggag agggattttt tatttttaag taggtaatta tttatttttt tttagaggga 16800
   acgagggatt tattttagtt tttatttttg attatttttt tttagttttg attgtataat   16860
   ttgaaattta tatacggttt gtaatttttt tttttttttt tcgagacgga gtttcgtttt   16920
   gtttttaggt tggagtgtag tggcgcgatt ttggtttatt gtaattttcg tttttcgggt   16980
   ttaagcgatt tttttggttt agttttttta gtagttggga ttatagacgc gtattattat   17040
   gttcggttaa tttgtttgta atttttttaa tttgtttggt ttttttatag ggagaggatt   17100
   tgtatatgtt gtgttttgtg tgatgaaagg agtttttttt atatattttt ttttttaatg   17160
   tatttagatt ttaaagttta ttatttattt atttaataaa tatttattaa gtattttttg   17220
   aattcggttt aattattttt tttttaggta tttatttaat ttcgggtatt tttcgcgttt   17280
   tttttgagtt tttttcgcgt tttatatagt ttttgtttat tgttggaaaa tatttttgtt   17340
   aagtttcgtt tttttattag ttttgtttgt tttttgtgtg tttgggatag gttgtaaaat   17400
   tggaggtgat aaatgtgggt aggaaatgga gggttttttt atatttttag ggtcggtttt   17460
   agttttgtta ttttttgttt aatatcgcgg atgtaattgg tatgggattc ggaagtgtgt   17520
   ggtaaagcgt cggggtttcg tttggtcgtt tttttcggac gtttgtttgt agttagcgaa   17580
   gtttttttaa tttaggtttg ggttttgcga gttttaggtg tttttgtttc gcggcgttgg   17640
   cgaagtcgaa gttcgagaac gtttatcgta gcgatgcgaa ggtcgttttt ggggtggggt   17700
   tgaggttgta gttgtttttt tttcgtatta aggattttaa tttttagcga cgtagtcgtc   17760
   gttttcgttt aggttgggag gtattgtagg gattcgacgt tttaggtggt taaagagcga   17820
   ttttttttga ttttagggtt ttggcggggt aggttttagt atcgtattcg gcggaggtcg   17880
   aaggtttgtg gggtaggata ggagtttttc gtgtcgtcgg aagggtcgag gacgaaggag   17940
   ggcgttaatt tattttttat tgggttggcg gtaacgtcga atttcgtagt gatcgcggag   18000
   ggttaaggtg aaaattgttg ggggcgtcga gggtaggtgt ggggaggggc ggttttaggg   18060
   agtaaggagt ttatttgttt cgtcgtcgta gttgttttgg gtcgatcgtt tacgtttttt   18120
   tttgggttac gattttcgga tttaattgtg ttcggttttt gttttttttt ttttgttgtt   18180
   gttgcgcggg ttgtaatttg acgtttaggt tggggcgttt agggcgtagt tttcgtttag   18240
   gtcgttagcg ttttattcgt tttattgggt tatagattcg ttggcgttgg ggtttcgttg   18300
   gcgttggggt ttcgttggcg ttggggtttc gttggcgttg gggtttcgtt ggcgttgggg   18360
   tttcgttggc gttggggttt cgttggcgtt ggggtttcgc ggttgtgtac gtgagttcgc   18420
   gtggtttcgt ttcgcgcggt tgtgtacgtg agttcgcgcg gtcgcgtttc gtttcgtttt   18480
   agggagttag cgcgtcgtta tttgggatgt taggattttc gttgtgtttt ttggattgtt   18540
   ttgggggatt tcggcgtatt tttaggattt aggagttttg gaagttgttt gagagaaatt   18600
   agttttggga gggtttcgta tttagttttt tgtttcggtt tcggatcggg gtttcgggtt   18660
   aaggtgttta gaggaatagt tgattttttt atttttgcgt agggtagaga tttttaaatt   18720
   tttttttaaa atgtagggtt ttagtttttt ttagggagtt agtgaattta gatttttagt   18780
   tttttgagtt taagtatgaa tagggaattg gggattatta ttatgtttat atttttggtg   18840
   gttaggaagt ttaggtaggt ttttgtttat tgtagacgga tttttttttt taggggttaa   18900
   gaaaggtttt gtatagtaag ttaattaagt tttattagta gagttgcgtt gtaattaggt   18960
   ttttagtgtt ttttttatat ttttagtttt aagcgatata ggatatagtt ttaattttat   19020
   ttttttttat ttattttttg ttttgttgtt ttattttttt tgttatatat tttaagttat   19080
   ttatttaatt tttagttttt ggtaatttag ttttagtttt tggaagttta gttttagttt   19140
   tgttattatt atttttattt tggttttttt tgagaataag tggagggtaa atagagttta   19200
   ggtttgaatt tattcgtttg aaaaataatt taagttaaat tttgtgggag tagtgtttgt   19260
   ttggggtatt ttaggtttat gtgtattttt gaattttttc gggtatttgt tttaaatgta   19320
   aagaggttgt tatttaatgt ttgggttttt ttgatttttt gttttttttt ggttgttttt   19380
   ttgtttttgt cgttttttat ttttttttgt tatggttagt atttggtttt taggttagaa   19440
   aaggtggatt tcgtgttttt ggattttatt aatgttagga gttatataaa tatttttata   19500
   tatatataga gagggttcgc gtatgtttgg aaatttttag atattaaaga ataaagtgta   19560
   ggatataata gagatattgt atatattgag aaatgtgtat attttttaaa tgtagagaaa   19620
   tgtatattaa tatatagaga tatattttat aaatatattt atagaaatag atatatttat   19680
   attttaagaa atattaagat atttatgaag ggaatatttt agatatgtat aggattttga   19740
   atttatatgg atatagatat ttaggagtag gtgggttttg atttaggtgt atatagttta   19800
   atatatagta tttatatata agttttttta agtttaaatg tcgtaagaga tttttataga   19860
   aagaaaattt ttttagaggt ttttaaggtt ttgtttggaa ggaagaggaa gaaagtgttc   19920
   gttaggtatc gaaatgttaa ggtattgtaa agtgaaaatt atttttttaa tttcgtggga   19980
   atagtatttt tatttttatt ttttagggaa atagggaagt ggtttaattc gttttgtaaa   20040
   ttaattttag atttataaga gtgttttttt tttcggcggg gagaggttag gtttttagtg   20100
   ttgtagttta gtgaatgttg attcgttgag gtttatttta ggttataggg tgttgggtaa   20160
   atttattaag ttcgtacgtt tgtttaatat ttggttgggt taggaagagt gtttggtgga   20220
   tatttagttt ggcgatatat aggaggtacg gtcggtgaag aaaatggatt tgtgtattaa   20280
   gggtttgtat tatttcgtcg ggttttgttt tgatagatta ttttttattt tttttgatta   20340
   cggattggtt taggaggttt attttcggtt cgtttattgt ttgtaaatag tagagggtag   20400
   cggtgagggc gtataaagtt ttagtcgttt atgttttttt aagtttttgt tatttagggt   20460
   ttttgtttgt ttcgtttaag gcgtattagt ttcgtttggg gcggcgacgg gtaggtttag   20520
   gaatcgttat gtttaggtat tgagttggag ttttgggtat tttattgtgt agtgtaaaaa   20580
   gggaattttg aattttatat tggtaattat tatgtttttt ttcgttttag gtgtaggttg 20640
   ttttatggat gttatacgtg gtagcgggga gaaaagaagt gattcggttt ttgtcgaggg 20700
   ttgttgttat ttacgtattg ttggcggatt tagtgttagg tagttttgat ggttaggtaa 20760
   cgtttaattt aggaggtggg atatattttc ggagttatta gtgagatgtg gcgagttggg 20820
   agaaaagtcg gttttttgta tttgttattt tttgttttta gttatggtag taaattatcg 20880
   cggttagttt cgatttattt cggatttttc gggttttatt tgtaggtttt ttagatcggg 20940
   ggtcgtcgtt agtttcggaa ggtcgagagg tgcgtttgtt aggtcgtgcg cgtcgtcggt 21000
   taggtcgtgt aggagtcggg gtacggtagt ggtatagtta cggcgcgggt taaggtttag 21060
   ggcgcgggcg ggcggtgtta gtaggtcggg aggtttttcg tgggcgcggg cggttgcgcg 21120
   gggcgcggtt taggcggttt gaggaggttg cggttgcgtt aggggcgtta ggtcgttata 21180
   tgggtttttt gttaggtggg gaaaggttgc ggcggcgtcg ggcgtttgat cgtagggtag 21240
   cggtggttgc gggtagggcg gcgggaagta gggcggttgg aattcggcgg tagttgtggc 21300
   ggtcggcgtg tggtagagcg ggggcgtcgg ttcgtaggtc gtttggggta gagacgatag 21360
   gcgggtttcg tcggtagttg tttttagttc gtcggggcgt ttttggggaa ggagaggggt 21420
   gttttaggtg atttattggt agttgaaagg attttagttt gaaaggattt tttttagttc 21480
   gggagggttt agttgtagtt tttttcgttt tttggggttc gaggtttatt ttcgagttat 21540
   tcgcgggcgt aggagtttta ggttggggat agcggttatt tgcgtatcgc gtttttagtt 21600
   ggcgaagttt ttgcggtttt tttttttcga gatttcgtta gcgcgggata ggagggttgg 21660
   aaagtttagt atttaggcgt ttcgattttt cggtcgaata tgtttcgggt tcgagattat 21720
   ttattatggc ggagtaggtg tgtattagta tggggcggtg agtgtcgggc gcgaggcgtg 21780
   gcggcggcgg agttttagtt cgacggcgag cgttgcgggt agaggcgtcg ggagtcggta 21840
   tagttcgcgg aggttacggt gcggtagttg ggtgcgattt agtcgggatt ttagtgggcg 21900
   tggggttttt agcggtggtc gttttcgggc gggtttatgg aggtaggacg gacgggtatt 21960
   gatcgtattt ggaaaagtcg gttaacgggt gtttagcgtt cgtttttttt tttttgagcg 22020
   tagtttgttt tatgtatttt agttatagtg gcgggggcgc gtcgggtttt cgggggcgcg 22080
   tattaaagag ataagttatg gtagtttttt tttttttttt tagcggttgt tataggggag 22140
   gtagtggttt tggcgggatc gggtagggtg ggtagggggc gtgaatttag tttatattga 22200
   ttagagtcgt tttttgggtt cgtagttcgt agttgtagtg aaatagaaat attcgaggtt 22260
   gggacgtaag ggttttatat ttgtttggtt ttattatatt tttgttttta agtaaagtat 22320
   tatttttcgt tggttttagt tttttcggta aaataggtat taattttttt tttaagtatt 22380
   ttaagagatt ttggatgagg tgggtaatcg ggaacgtgga agaagtttgt gagttgaaga 22440
   ttttagttta tttgtttatg cgtatatagt gttagtgcgt gtgtttaggg ggtttattat 22500
   tatagttttt tttgaatggt tttggattaa aatttttcga aggtgtttat ttcgttttta 22560
   ggttttttgg gttgaaaatt tttaagttaa gttaggtttt gagggagttg gagatagaag 22620
   ttggttagat agagagagag atttgtttgg gatgggtttg ggtatgagtt ttatttgata 22680
   aggggttaag atatttttga agttataggg ttttgggttg attgagtgtg ggtatgagat 22740
   atttagagat tgttgagtga ttgtgtgtaa ttgattagta tggatgtcgt gtgattgtta 22800
   tattaatatg cgtaggttat atgtttttgt gttattatgg taggtatttt ttggttgtta 22860
   agtaagtttt agtttttgta gttatttttt tttttttttt aaatttaatt tttttttttt 22920
   gataagtaga gatagggatt aaagatggat aaatgagagg aatgttaagg agaataggaa 22980
   ttaagaaagt agaagaaaga tgttttttaa aagtaagatg tgtagattag ggtaaagtag 23040
   aaataattat agtagattta ttatagaatg tgttagtttt tgtgtaaagt gttttacgaa 23100
   tattgggttt tttaattttt atagtagttt tatgaaagag atatttatat tatttttatt 23160
   tttaggggag gggtgaggta tggagaggtt aaatggtttg tttaaggtta agtagttaag 23220
   tggtagagtt ggggtgattt gtttttggtt tttttttgtt tagaattatt attttttatt 23280
   gttttagatt taggggtttg gatttagttt tgattttgtt taattgtgtg attttagtta 23340
   tatttcgttt tttttgtgat tttagttttt gtaatttcga taatattaat aaaaaagtag 23400
   gggaaggtag agaagattag ttgttttttt aggttttttt ttagtgaagt agtaaatgag 23460
   aagattaagg taaaattatt gatatagaag aggaggtgat agtgatatta ttaatttatg 23520
   gaatatatgg ttttaagtgg gtattttaga aggtatatgg taggtgtaga gtttagggtt 23580
   ggatgatttt agtttgggga gtggtgggga aagaaggagg ttagaagtta aggatttatt 23640
   attaagaatt ttttgaagtt tggaatcgtt agcgaggttt tagaggatga agcggaattg 23700
   ggaagttttg taattttttt gttcgagatg ggttagagat taggattgtg tttgtgtgta 23760
   tttatatatg agggagtttg tggtaatgtt ttgttttttt tttttttaat tttagttata 23820
   gggtattaga agttaagttt taggataggt gttagtgcgt gaatagaaat tgatgaattt 23880
   ttttgggaga agtttaggag tcgtaaagga gatgatgttt gtgtttagtt gcggggatgt 23940
   ttttgttttt tttagtagtt tgggaaggtt agagtggcgg gatgtgttgt ttgtttgtgg 24000
   tttttttggg tttttttaat ttatttttta tatttttgat agtttttttt tgttttattg 24060
   ttttatttcg attatttttg tttttttttt tgtttttttt tttatttata tgtttaatgt 24120
   cggtaatttt tttttagagt agagaagttt tttttttttt ttgtattttt tatgagtgag 24180
   tttatttttt tttaagtttt tttttttttt ttttttataa tagagtttta ttttgttgtt 24240
   tgggttggag tgtagtggta taatttcggt ttattgtaat ttttattttt cgggtttaag 24300
   cgatttttat gttttagttt tttgagtaat tgggattata ggggtttatt attacgttcg 24360
   gttaattttt gtatttttat tagagatagg gttttattat gttagttagg ttgatttcga   24420
   atttttgatt ttaagtgaat attcgtttta gttttttaaa gtgttgggat tataggtatg   24480
   agttattacg ttcggtttaa gtttttaatg attatttgtt tattgttatt ttttaattgt   24540
   gttatttttg gtttgaatta ttagtgagag atttagattt atagatatag ttgttagttg   24600
   cgtatttttt ttagatattt tattgtagtt gtttgaaatt ttttaagttt aatattgaat   24660
   ttaatatttt ttttattttt agtattaatt ttaatttgtt ttttttttgt tttgtagttt   24720
   tataaatgat attatttttt tttaagttta aaatttgtta gttattttag aagagcgtag   24780
   gttttttttt taatttttat aagttaattt ttatggagtg ttgttatttt taatttttaa   24840
   atatttattt atagaaggta tgtggtggag aattgaggta ggtattttag agggtatatg   24900
   gtattttgtt ttagtatttt aaattatttt atttttattt atagattagt ggtttttaa    24959
<210> 5
   <211> 6096
   <212> DNA
   <213> Artificial Sequence
<220>
   <223> Synthetic construct: chemically treated genomic DNA (Homo sapiens)
   <400> 5
   tattagtgta agatttaaaa tttttttttt gtattgtata gtgagatgtt tagggtttta      60
   gtttagtgtt tggatatagc gatttttggg tttgttcgtc gtcgttttaa gcgaagttgg     120
   tgcgttttgg gcggagtaga tagagatttt gggtggtagg ggtttgggaa gatatgggcg     180
   gttagggttt tatgcgtttt tatcgttgtt ttttgttatt tgtaggtaat ggacgagtcg     240
   ggaatgagtt ttttagatta gttcgtgatt aagaaaggta aggaatggtt tgttagggta     300
   gagttcggcg agatggtgta ggtttttggt gtatagattt atttttttta tcggtcgtgt     360
   tttttgtgtg tcgttaggtt gggtgtttat taggtatttt ttttggttta gttagatgtt     420
   aggtagacgt gcgggtttgg tgagtttgtt tagtattttg tggtttgggg tgggttttag     480
   cggattagta tttattgggt tgtagtattg ggagtttggt tttttttcgt cgagggggag     540
   ggtatttttg tggatttgga gttgatttgt agaacgagtt aaattatttt tttgtttttt     600
   taagagatgg gaatggaagt gttgttttta cggagttggg gaaatgattt ttattttata     660
   gtgttttagt atttcggtgt ttggcgggta tttttttttt ttttttttta ggtagggttt     720
   tggaggtttt tgggggaatt ttttttttgt gggagttttt tgcggtattt agatttaggg     780
   gagtttgtgt gtgagtattg tgtgttaggt tgtgtgtatt tgagttaggg tttatttgtt     840
   tttgggtgtt tgtgtttatg tgagtttagg gttttgtgta tgtttgaaat gtttttttta     900
   tgggtgtttt agtatttttt ggagtgtgag tgtgtttgtt tttgtgaatg tgtttgtgag     960
   gtgtgttttt gtatgttggt gtgtattttt ttgtatttgg gggatgtata tattttttaa    1020
   tatgtatagt atttttgttg tgttttgtat tttgtttttt ggtatttgag gatttttaag    1080
   tatgcgcggg ttttttttgt gtatatatag gagtatttat gtgatttttg gtattagtaa    1140
   aatttaggga tacgggattt attttttttg gtttgaggat taagtattgg ttatgatagg    1200
   ggaaggtgag agacgataaa aatagagaga tagttagaga ggagtagaga gttagagggg    1260
   tttaggtatt gggtagtagt ttttttatat ttggggtagg tgttcgaaag aatttagagg    1320
   tgtatatgag tttgaggtgt tttaggtagg tattgttttt atagggtttg gtttgagttg    1380
   ttttttaaac gagtgaattt aagtttgggt tttatttgtt ttttatttgt ttttagggga    1440
   ggttaaggtg gaagtggtgg tagtagggtt ggggttggat ttttaggagt tggggttgag    1500
   ttattaggag ttgggggttg ggtggatgat ttggagtgtg tagtagggaa gatgaggtaa    1560
   tagggtagga agtgggtggg gggaggtgga attggggttg tgttttgtgt cgtttggaat    1620
   tgggagtgtg ggaaagatat taggaatttg gttgtagcgt agttttgttg gtggggtttg    1680
   gttggtttat tgtatagagt tttttttgat ttttgaagaa agagattcgt ttgtagtggg    1740
   taaaagtttg tttggatttt ttggttatta gaaatatgag tatggtggtg gtttttagtt    1800
   ttttatttat gtttgggttt aagagattgg gagtttaggt ttattgattt tttgagaaag    1860
   attaagattt tgtattttag aaagaggttt ggggattttt gttttgcgta agggtagaag    1920
   gattagttgt ttttttgagt attttaattc ggaatttcgg ttcgaagtcg agataggaga    1980
   ttggatgcga ggttttttta gagttggttt tttttaaata atttttaaaa tttttagatt    2040
   ttaggggtac gtcgaaattt tttaaagtag tttaaagaat ataacgagag ttttaatatt    2100
   ttaggtggcg gcgcgttggt tttttggagc ggggcgggac gcggtcgcgc ggatttacgt    2160
   gtataatcgc gcgggacggg gttacgcgga tttacgtgta taatcgcggg attttagcgt    2220
   tagcgggatt ttagcgttag cgggatttta gcgttagcgg gattttagcg ttagcgggat    2280
   tttagcgtta gcgggatttt agcgttagcg ggattttagc gttagcgggt ttgtggttta    2340
   gtggagcgag tggagcgttg gcgatttgag cggagattgc gttttggacg ttttagttta    2400
   gacgttaagt tatagttcgc gtagtagtag taaaggggaa ggggtaggag tcgggtatag    2460
   ttggattcgg aggtcgtgat ttaggggaaa gcgtgggcgg tcgatttagg gtagttgcgg    2520
   cggcgaggta ggtgggtttt ttgttttttg gagtcgtttt tttttatatt tgttttcggc    2580
   gtttttagta gtttttattt tggtttttcg cggttattgc gggattcggc gttgtcgtta    2640
   gtttagtggg gagtgaatta gcgttttttt tcgttttcgg ttttttcgac ggtacgagga    2700
   atttttgttt tgttttatag attttcggtt ttcgtcgagt gcggtattgg agtttgtttc    2760
   gttagggttt tggaattaga gaaagtcgtt ttttggttat ttgaagcgtc ggatttttat    2820
   agtgtttttt agtttgggcg ggagcggcgg ttgcgtcgtt gaaggttggg gtttttggtg    2880
   cgaaagggag gtagttgtag ttttagtttt attttagaag cggttttcgt atcgttgcgg    2940
   tgggcgtttt cgggtttcga tttcgttagc gtcgcggggt agaggtattt ggagttcgta    3000
   gggtttagat ttgggttgga aaagtttcgt tgattgtagg taagcgttcg ggaggggcgg    3060
   ttaggcgaag tttcggcgtt ttattatata ttttcgggtt ttatgttagt tgtattcgcg    3120
   gtattgggta ggaaatggta gggttgaggt cgattttagg agtataaggg agttttttat    3180
   tttttgttta tatttgttat ttttagtttt gtaatttatt ttagatatat agaaagtaag    3240
   taggattggt ggggagacgg agtttaatag gaatattttt tagtagtgag taggggttgt    3300
   atgggacgcg ggaggagttt agaggaggcg cggagagtgt tcgaggttgg gtgagtgttt    3360
   agaggggaga tagttgaatc gggtttaaga ggtgtttagt gggtgtttgt tgaatgaatg    3420
   agtgatgggt tttgaagttt gagtgtattg aaagaggggg tgtgtaaaaa gggttttttt    3480
   tattatatag gatatagtat atgtaaattt tttttttgtg gaaaagttag ataggttaaa    3540
   aaggttataa ataaattagt cgggtatggt ggtgcgcgtt tgtagtttta gttattaggg    3600
   aggttgagtt aggggaatcg tttgaattcg ggaggcggag attgtagtga gttaagatcg    3660
   cgttattgta ttttagtttg gaaatagagc gagatttcgt ttcggaaaaa aaaaaaaaaa    3720
   gttataaatc gtgtgtgggt tttaggttat ataattagag ttggagggga gtggttaagg    3780
   atgagaattg agatggattt ttcgtttttt ttggaggaga gtgggtggtt gtttatttgg    3840
   gggtggggaa ttttttttta cgggtttagt tgtttaattt taggggattt ttaggatagg    3900
   agttgatgta aatagtcgtt ttattttttg ttgtttttgg ttttggagaa ggaggaggga    3960
   gttggggagg gtttttattt tttagataat ttttaagtag ttaggatatg ggtgagatga    4020
   gtgagatatt gatttttggg atagaatttg agagggtgtt aaaaaattta gtaattaaga    4080
   taaataggtc gggcgtagtg gtttacgttt gtaattttag tattttggga ggtcggatta    4140
   tttgaggtta agagttcgag attagtttgg ttaagatggt gaaattttat ttttattaaa    4200
   aatataaaaa ttagtttagt gtggtggcgt tagtttgtaa ttttagttat ttaggaggtt    4260
   gaggtaagag aattgtttga tttaggaggt agaggttgta gtgagtcgag attatgttat    4320
   tgtattttag tttggataat agagggagat tattttaaaa aaaaaaaaaa aaaaaaaaaa    4380
   aaaaaagagg tcgggcggcg gtggtttata ttatgtgatt ttagtatttt gggaggtcga    4440
   ggcgggtgga ttatttgagg tttggagttc gagattagtt tggttaatat ggtgaaattt    4500
   cgtttttatt aaaaatataa aaattagttg ggtggggtgg taggtatttg taattttagt    4560
   tatttcggag gttgaggtag gagaattttt tgaatttgcg gggcggaggt tgtagtgaat    4620
   taagattata ttattgtatt ttagtttgga taataatagt aaaattttgt tttaaaaaaa    4680
   aaaaaaattt tttttttcga gatatagttt tattttttcg tttaggttgg ggtgtagtat    4740
   tacgatttta gtttattgta atttttgttt tttagatttt cgtattttag ttttttaagt    4800
   agttgggatt ataggtattt gttattacgt ttagttaatt tttgtatttt tagtaggggc    4860
   gtggttttat tatgttggtt aggttggttt tgaatttttg attttaagtg atttgttcgt    4920
   tttagttatt taaagtgttg ggattatagg cgtgagttat tacgtttggt ttttttaaat    4980
   gaaaatagtg taaaaattta cgataaataa aatattaaaa atttattgaa tttgtatttt    5040
   tataattttt ttttatttgt tttttaggtt attttttgtt ttagaaagta atttaaaaaa    5100
   tgtgtagatg gagtttggat tttatttgaa aatggtggga gttatggaaa attttggagt    5160
   aggggagtga aggatagaaa ttatatgtaa aagaaatttt gggtcgggcg tagtggttta    5220
   tgtttgtaat tttagtattt tgggaggtcg aggtaggtgg attatttgag gttaggagat    5280
   tgagattagt ttgattaata tggtgaaatg ttatttttat taaaaatata aaaaaaatta    5340
   gttaggtatg gtggtgtacg tttgtagttt tagttatttc ggaggttgag ataggaaaat    5400
   cgtttgaatt cgggaggcgg aggttgtagt gagttaagat tgtgttattg tattttagtt    5460
   tgggtaataa gagtaaaatt ttattttaaa aaaaaagaaa gaaagaaatt ttttggtagt    5520
   tgatgagaag gaaatttaat cggtaggttt tagtagggga gatgaggaga ttttagggag    5580
   ggtatttgta tatgttgtgt tttagtgtgg gttagggagt aggttattat ttttttcgtt    5640
   tatttttttt ttgttttaat ttttttaagt tttggattag tggtatttta agtgtagttt    5700
   aaggaattat atgtattagg atttttaggg ggtgtttgtt aaaaatgtaa attttggtta    5760
   ggtgtagtgg tttatatttg taattttagt attttgggag gtcgaggcgg gtggattacg    5820
   aggttaggag atcgagatta ttttggtaaa tacggtgaaa ttttattttt attaaaaaaa    5880
   taaaaataaa ttaaaaaaaa tattagttgg gcgtggtggc gggcgtttgt agttttagtt    5940
   attcgggagg ttgaggtagg agaatggcgt gaattcggga ggcggagttt gtagtgagtt    6000
   gagattgcgt tattgtattt tagtttgggc gatagagcga gattttgttt taaaaaaaaa    6060
   aaagtaaatt ttttgggtat tattttatat tgattg                             6096
<210> 6
   <211> 6096
   <212> DNA
   <213> Artificial Sequence
<220>
   <223> Synthetic construct: chemically treated genomic DNA (Homo sapiens)
   <400> 6
   tagttaatgt ggggtggtgt ttaagaaatt tgtttttttt tttttgaggt agagtttcgt      60
   tttgtcgttt aggttggggt gtagtggcgt aattttagtt tattgtaagt ttcgtttttc     120
   gggtttacgt tatttttttg ttttagtttt tcgagtagtt gggattatag gcgttcgtta     180
   ttacgtttag ttaatgtttt ttttggtttg tttttgtttt tttagtagag atgggttttt     240
   atcgtgtttg ttaggatggt ttcgattttt tgatttcgtg atttattcgt ttcggttttt     300
   taaagtgttg ggattatagg tgtgagttat tgtatttggt taaaatttgt atttttaata     360
   agtatttttt gggggttttg atgtatgtgg ttttttggat tatatttagg gtattattgg     420
   tttaaagttt gaaggggttg gagtaagagg aaggtagacg ggaggagtag tgatttgttt     480
   tttggtttat attggggtat agtatgtgta aatgtttttt ttagagtttt tttatttttt     540
   ttgttgggat ttgtcgatta agtttttttt ttattaattg ttagagggtt tttttttttt     600
   ttttttttta agatggagtt ttgtttttgt tgtttaggtt ggagtgtaat ggtataattt     660
   tggtttattg taattttcgt ttttcgggtt taagcgattt ttttgtttta gttttcggag     720
   tagttgggat tataggcgtg tattattatg tttggttaat tttttttgta tttttagtag     780
   agatgatatt ttattatgtt ggttaggttg gttttaattt tttgatttta ggtaatttat     840
   ttgtttcggt tttttaaagt gttgggatta taggtataag ttattgcgtt cggtttaggg     900
   ttttttttat atataatttt tattttttat ttttttgttt taaggttttt tatagttttt     960
   attattttta ggtaaagttt aaattttatt tgtatatttt ttaagttgtt ttttggggta    1020
   gagagtagtt tgggaggtag gtgagaaagg gttgtggaag tgtaagttta gtaaattttt    1080
   gatattttgt ttatcgtgga tttttgtatt atttttattt aaaaaggtta agcgtggtgg    1140
   tttacgtttg taattttagt attttgggtg gttgaagcgg gtaggttatt tgaggttagg    1200
   agtttaagat tagtttggtt aatatggtga aattacgttt ttattaaaaa tataaaaatt    1260
   agttgggcgt ggtgataggt atttgtaatt ttagttattt gggaggttga ggtacgagaa    1320
   tttgagaggt agaggttgta gtgagttgag atcgtggtgt tgtattttaa tttgggcgag    1380
   agagtaaaat tgtgtttcga aaaaaaagat tttttttttt tttgagatag agttttgttg    1440
   ttgttgttta ggttggagtg taatggtgtg attttggttt attgtaattt tcgtttcgta    1500
   ggtttaaggg atttttttgt tttagttttc ggagtagttg ggattatagg tgtttgttat    1560
   tttatttagt taatttttgt atttttagta gaaacggggt tttattatat tggttaggtt    1620
   ggtttcgaat tttagatttt aggtgattta ttcgtttcgg ttttttaaag tgttgggatt    1680
   atatggtgtg agttatcgtc gttcggtttt tttttttttt tttttttttt tttttttttt    1740
   gagatagttt ttttttgttg tttaggttgg agtatagtgg tatgatttcg gtttattgta    1800
   atttttgttt tttgggttaa gtaatttttt tgttttagtt ttttgagtgg ttgggattat    1860
   aggttagcgt tattatattg ggttaatttt tgtattttta gtagagatgg ggttttatta    1920
   ttttggttag gttggtttcg aatttttgat tttaagtgat tcggtttttt aaagtgttgg    1980
   gattatagac gtgagttatt gcgttcggtt tatttatttt gattattgag ttttttggta   2040
   tttttttaaa ttttgtttta gaagttagta ttttatttat tttatttatg ttttggttgt   2100
   ttagagattg tttgggaagt ggagattttt tttagttttt tttttttttt ttagggttaa    2160
   agatagtaag gaatagggcg attgtttata ttagtttttg ttttagagat tttttgagat    2220
   tggatagttg agttcgtgga gagggatttt ttatttttaa gtaggtaatt atttattttt    2280
   ttttagaggg aacgagggat ttattttagt ttttattttt gattattttt ttttagtttt    2340
   gattgtataa tttgaaattt atatacggtt tgtaattttt tttttttttt ttcgagacgg    2400
   agtttcgttt tgtttttagg ttggagtgta gtggcgcgat tttggtttat tgtaattttc    2460
   gtttttcggg tttaagcgat ttttttggtt tagttttttt agtagttggg attatagacg    2520
   cgtattatta tgttcggtta atttgtttgt aattttttta atttgtttgg tttttttata    2580
   gggagaggat ttgtatatgt tgtgttttgt gtgatgaaag gagttttttt tatatatttt    2640
   tttttttaat gtatttagat tttaaagttt attatttatt tatttaataa atatttatta   2700
   agtatttttt gaattcggtt taattatttt ttttttaggt atttatttaa tttcgggtat   2760
   ttttcgcgtt ttttttgagt ttttttcgcg ttttatatag tttttgttta ttgttggaaa    2820
   atatttttgt taagtttcgt ttttttatta gttttgtttg ttttttgtgt gtttgggata    2880
   ggttgtaaaa ttggaggtga taaatgtggg taggaaatgg agggtttttt tatattttta    2940
   gggtcggttt tagttttgtt attttttgtt taatatcgcg gatgtaattg gtatgggatt   3000
   cggaagtgtg tggtaaagcg tcggggtttc gtttggtcgt ttttttcgga cgtttgtttg   3060
   tagttagcga agttttttta atttaggttt gggttttgcg agttttaggt gtttttgttt    3120
   cgcggcgttg gcgaagtcga agttcgagaa cgtttatcgt agcgatgcga aggtcgtttt    3180
   tggggtgggg ttgaggttgt agttgttttt ttttcgtatt aaggatttta atttttagcg    3240
   acgtagtcgt cgttttcgtt taggttggga ggtattgtag ggattcgacg ttttaggtgg    3300
   ttaaagagcg attttttttg attttagggt tttggcgggg taggttttag tatcgtattc 3360
   ggcggaggtc gaaggtttgt ggggtaggat aggagttttt cgtgtcgtcg gaagggtcga 3420
   ggacgaagga gggcgttaat ttatttttta ttgggttggc ggtaacgtcg aatttcgtag 3480
   tgatcgcgga gggttaaggt gaaaattgtt gggggcgtcg agggtaggtg tggggagggg 3540
   cggttttagg gagtaaggag tttatttgtt tcgtcgtcgt agttgttttg ggtcgatcgt 3600
   ttacgttttt ttttgggtta cgattttcgg atttaattgt gttcggtttt tgtttttttt 3660
   tttttgttgt tgttgcgcgg gttgtaattt gacgtttagg ttggggcgtt tagggcgtag 3720
   ttttcgttta ggtcgttagc gttttattcg ttttattggg ttatagattc gttggcgttg 3780
   gggtttcgtt ggcgttgggg tttcgttggc gttggggttt cgttggcgtt ggggtttcgt 3840
   tggcgttggg gtttcgttgg cgttggggtt tcgttggcgt tggggtttcg cggttgtgta 3900
   cgtgagttcg cgtggtttcg tttcgcgcgg ttgtgtacgt gagttcgcgc ggtcgcgttt 3960
   cgtttcgttt tagggagtta gcgcgtcgtt atttgggatg ttaggatttt cgttgtgttt 4020
   tttggattgt tttgggggat ttcggcgtat ttttaggatt taggagtttt ggaagttgtt 4080
   tgagagaaat tagttttggg agggtttcgt atttagtttt ttgtttcggt ttcggatcgg 4140
   ggtttcgggt taaggtgttt agaggaatag ttgatttttt tatttttgcg tagggtagag 4200
   atttttaaat ttttttttaa aatgtagggt tttagttttt tttagggagt tagtgaattt 4260
   agatttttag ttttttgagt ttaagtatga atagggaatt ggggattatt attatgttta 4320
   tatttttggt ggttaggaag tttaggtagg tttttgttta ttgtagacgg attttttttt 4380
   ttaggggtta agaaaggttt tgtatagtaa gttaattaag ttttattagt agagttgcgt 4440
   tgtaattagg tttttagtgt tttttttata tttttagttt taagcgatat aggatatagt 4500
   tttaatttta ttttttttta tttatttttt gttttgttgt tttatttttt ttgttatata 4560
   ttttaagtta tttatttaat ttttagtttt tggtaattta gttttagttt ttggaagttt 4620
   agttttagtt ttgttattat tatttttatt ttggtttttt ttgagaataa gtggagggta 4680
   aatagagttt aggtttgaat ttattcgttt gaaaaataat ttaagttaaa ttttgtggga 4740
   gtagtgtttg tttggggtat tttaggttta tgtgtatttt tgaatttttt cgggtatttg 4800
   ttttaaatgt aaagaggttg ttatttaatg tttgggtttt tttgattttt tgtttttttt 4860
   tggttgtttt tttgtttttg tcgtttttta tttttttttg ttatggttag tatttggttt 4920
   ttaggttaga aaaggtggat ttcgtgtttt tggattttat taatgttagg agttatataa 4980
   atatttttat atatatatag agagggttcg cgtatgtttg gaaattttta gatattaaag 5040
   aataaagtgt aggatataat agagatattg tatatattga gaaatgtgta tattttttaa 5100
   atgtagagaa atgtatatta atatatagag atatatttta taaatatatt tatagaaata 5160
   gatatattta tattttaaga aatattaaga tatttatgaa gggaatattt tagatatgta 5220
   taggattttg aatttatatg gatatagata tttaggagta ggtgggtttt gatttaggtg 5280
   tatatagttt aatatatagt atttatatat aagttttttt aagtttaaat gtcgtaagag 5340
   atttttatag aaagaaaatt tttttagagg tttttaaggt tttgtttgga aggaagagga 5400
   agaaagtgtt cgttaggtat cgaaatgtta aggtattgta aagtgaaaat tattttttta 5460
   atttcgtggg aatagtattt ttatttttat tttttaggga aatagggaag tggtttaatt 5520
   cgttttgtaa attaatttta gatttataag agtgtttttt ttttcggcgg ggagaggtta 5580
   ggtttttagt gttgtagttt agtgaatgtt gattcgttga ggtttatttt aggttatagg 5640
   gtgttgggta aatttattaa gttcgtacgt ttgtttaata tttggttggg ttaggaagag 5700
   tgtttggtgg atatttagtt tggcgatata taggaggtac ggtcggtgaa gaaaatggat 5760
   ttgtgtatta agggtttgta ttatttcgtc gggttttgtt ttgatagatt attttttatt 5820
   ttttttgatt acggattggt ttaggaggtt tattttcggt tcgtttattg tttgtaaata 5880
   gtagagggta gcggtgaggg cgtataaagt tttagtcgtt tatgtttttt taagtttttg 5940
   ttatttaggg tttttgtttg tttcgtttaa ggcgtattag tttcgtttgg ggcggcgacg 6000
   ggtaggttta ggaatcgtta tgtttaggta ttgagttgga gttttgggta ttttattgtg 6060
   tagtgtaaaa agggaatttt gaattttata ttggta 6096
<210> 7
   <211> 24959
   <212> DNA
   <213> Artificial Sequence
<220>
   <223> Synthetic construct: chemically treated genomic DNA (Homo sapiens)
   <400> 7
   ttgagagtta ttgatttatg agtaagagta aggtagttta aggtgttgag atagggtgtt      60
   atgtgttttt tgggatattt gttttaattt tttattatat attttttgtg ggtggatgtt     120
   taagggttag aaatggtaat attttataag gattagtttg tgggggttaa ggaaaggatt      180
   tatgtttttt tgggatgatt agtaagtttt gaatttgaaa aagggtggtg ttatttatga     240
   aattatagag taaaggagaa gtaggttggg gttggtgttg gggatagagg agatgttgag     300
   tttagtgttg gatttggaaa attttagata gttgtaatga gatgtttgga gaaaatatgt 360
   agttggtagt tgtgtttatg ggtttggatt ttttattagt gatttaggtt agagataata 420
   tagttgggag ataatagtag ataggtagtt attaaagatt tgggttgggt gtggtgattt 480
   atgtttgtaa ttttagtatt ttgggaggtt gaggtgagtg tttatttgaa gttaggagtt 540
   tgagattagt ttggttgata tgatgaaatt ttgtttttag taaaaatata aaagttagtt 600
   gggtgtggtg gtgggttttt gtaattttag ttatttagga aattgagata tgagaattgt 660
   ttgaatttgg gaggtggagg ttgtagtgag ttgagattgt gttattgtat tttagtttaa 720
   gtaatagagt gagattttgt tataaaaaaa aaaaaaaaaa agatttgaga ggggatgagt 780
   ttatttatga agagtgtagg aagaaaaaga agtttttttg ttttgagaaa gaattattga 840
   tattaagtat gtaggtgaaa agagaaatag aaaagaagat agaaatggtt ggagtaggat 900
   aatgaggtaa aaaggggtta ttaaaggtgt aggaagtaga ttgagaaaat ttaaggaagt 960
   tatagatagg tagtatattt tgttattttg gtttttttaa gttgttggag agagtagggg 1020
   tatttttgta gttgggtata ggtattattt tttttgtggt ttttagattt tttttagagg 1080
   agtttattaa tttttattta tgtattgata tttgttttgg agtttgattt ttgatgtttt 1140
   gtggttgggg ttgagggagg gaggaatagg gtattgttat agattttttt atatatagat 1200
   atatataaat ataattttga tttttgattt attttgagta ggaaagttgt aaagtttttt 1260
   aattttgttt tattttttgg aattttgttg gtggttttag attttagaaa gtttttaatg 1320
   atgaattttt ggtttttagt tttttttttt ttttattatt ttttaggttg agattattta 1380
   gttttgagtt ttgtatttgt tatgtgtttt ttgggatatt tatttggaat tatatatttt 1440
   atgagttaat ggtgttatta ttattttttt ttttatatta ataattttat tttaattttt 1500
   ttatttatta ttttattaaa aagggattta aaaaaataat tagttttttt tgtttttttt 1560
   tattttttta ttggtgttgt tgaagttgta gaaattgaga ttatagagga aatgggatgt 1620
   agttaggatt atatagttag gtagagttag gattaaattt aagtttttga gtttgaggta 1680
   gtagagaata gtgattttga gtagagaggg gttaggggta gattatttta gttttgttat 1740
   ttagttgttt gattttgggt aagttattta atttttttat gttttatttt ttttttgaaa 1800
   atggggatga tatgggtatt ttttttatag agttgttgtg agggttaaag aatttaatat 1860
   ttgtgaagta ttttgtatag aggttggtat attttgtaat aagtttgttg tgattatttt 1920
   tattttgttt tggtttgtat attttgtttt taaaaaatat tttttttttg tttttttaat 1980
   ttttgttttt tttagtattt tttttatttg tttattttta atttttgttt ttgtttgtta 2040
   ggaggggagg gttgggtttg gggagaaaga gagagtgatt atagagattg gaatttgttt 2100
   gatagttagg gagtgtttgt tatggtaata taggaatatg tagtttgtgt atgttagtgt 2160
   aataattata tggtatttat gttagttagt tatatatagt tatttaatag tttttgggta 2220
   ttttatgttt atatttaatt agtttaggat tttgtggttt tagaaatatt ttggtttttt 2280
   gttaggtgga gtttatattt aaatttattt taggtaagtt tttttttttg tttggttagt 2340
   ttttgttttt aattttttta gagtttggtt tggtttggaa gtttttagtt taggaagttt 2400
   gagagtgaag tgggtatttt tgagggattt tgatttagaa ttatttagga aagattgtga 2460
   tggtagattt tttaaatata tgtattgata ttgtgtgtgt atgagtagat aggttggagt 2520
   ttttagttta taaatttttt ttatgttttt gattatttat tttatttaaa gttttttgag 2580
   gtgtttagag aaaggattga tatttatttt attgaggaag ttgaggttag tgaagaatag 2640
   tgttttgttt aaaggtaagg atgtgataga gttaggtaaa tgtagggttt ttgtgtttta 2700
   gttttgaata tttttgtttt attgtagttg tgagttgtga gtttagaagg tggttttgat 2760
   taatgtgggt tgaatttatg ttttttattt attttatttg attttgttaa agttattgtt 2820
   ttttttgtgg tagttgttgg ggaaaaggga gagaattgtt atagtttgtt tttttaatgt 2880
   gtgtttttgg aggtttggtg tgtttttgtt attataattg gagtgtatgg agtaggttat 2940
   gtttagagga ggaagggtgg gtgttgggta tttgttgatt gattttttta agtgtgatta 3000
   gtgtttgttt gttttgtttt tatggatttg tttgggaatg gttattgttg aggattttat 3060
   gtttattagg attttggttg ggttgtattt agttattgta ttgtgatttt tgtgggttgt 3120
   gttggttttt ggtgtttttg tttgtagtgt ttgttgttgg gttagggttt tgttgttgtt 3180
   atgttttgtg tttggtattt attgttttat gttggtgtat atttattttg ttatggtgag 3240
   tagttttggg tttgggatat atttggttgg gggattgggg tgtttgagtg ttggattttt 3300
   taattttttt gttttgtgtt aatgaaattt tggagggagg gggttgtagg gattttgtta 3360
   gttgagaatg tgatgtgtaa gtgattgttg tttttagttt gaggtttttg tgtttgtggg 3420
   tggtttggaa ataaattttg ggttttaaga aatgggaggg attgtagttg aatttttttg 3480
   agttgaggag gattttttta ggttggggtt tttttagttg ttagtgggtt atttaaggta 3540
   tttttttttt tttttaggag tgttttgatg ggttgggagt agttgttggt ggggtttgtt 3600
   tgttgttttt gttttaggtg gtttatgggt tggtgttttt gttttgttat atgttggttg 3660
   ttatagttgt tgttgaattt tagttgtttt attttttgtt gttttatttg tagttattgt 3720
   tgttttatgg ttaggtgttt gatgttgttg tagttttttt ttatttggta ggggatttat 3780
   atggtggttt ggtgtttttg gtgtagttgt agttttttta ggttgtttgg gttgtgtttt 3840
   gtgtagttgt ttgtgtttat gaggagtttt ttggtttgtt ggtattgttt gtttgtgttt 3900
   tgggttttga tttgtgttgt gattatgtta ttgttgtgtt ttggtttttg tatggtttgg 3960
   ttgatggtgt gtatggtttg gtagatgtat tttttggttt tttggggttg gtggtggttt 4020
   ttggtttgga ggatttgtag gtgagatttg agggatttgg gatgggttgg gattggttgt 4080
   ggtggtttat tattatggtt ggaggtagaa ggtgataaat gtaggaagtt gatttttttt 4140
   ttagtttgtt atattttatt ggtgattttg aggatgtgtt ttattttttg ggttagatgt 4200
   tgtttggtta ttaaggttgt ttggtattga gtttgttagt agtgtgtggg tggtagtaat 4260
   ttttggtagg gattgaatta tttttttttt ttttgttgtt atgtgtggta tttatggggt 4320
   agtttgtatt tggggtgaag agaagtataa tagttattag tgtaagattt aaaatttttt 4380
   ttttgtattg tatagtgaga tgtttagggt tttagtttag tgtttggata tagtgatttt 4440
   tgggtttgtt tgttgttgtt ttaagtgaag ttggtgtgtt ttgggtggag tagatagaga 4500
   ttttgggtgg taggggtttg ggaagatatg ggtggttagg gttttatgtg tttttattgt 4560
   tgttttttgt tatttgtagg taatggatga gttgggaatg agttttttag attagtttgt 4620
   gattaagaaa ggtaaggaat ggtttgttag ggtagagttt ggtgagatgg tgtaggtttt 4680
   tggtgtatag atttattttt tttattggtt gtgttttttg tgtgttgtta ggttgggtgt 4740
   ttattaggta ttttttttgg tttagttaga tgttaggtag atgtgtgggt ttggtgagtt 4800
   tgtttagtat tttgtggttt ggggtgggtt ttagtggatt agtatttatt gggttgtagt 4860
   attgggagtt tggttttttt ttgttgaggg ggagggtatt tttgtggatt tggagttgat 4920
   ttgtagaatg agttaaatta tttttttgtt tttttaagag atgggaatgg aagtgttgtt 4980
   tttatggagt tggggaaatg atttttattt tatagtgttt tagtattttg gtgtttggtg 5040
   ggtatttttt tttttttttt tttaggtagg gttttggagg tttttggggg aatttttttt 5100
   ttgtgggagt tttttgtggt atttagattt aggggagttt gtgtgtgagt attgtgtgtt 5160
   aggttgtgtg tatttgagtt agggtttatt tgtttttggg tgtttgtgtt tatgtgagtt 5220
   tagggttttg tgtatgtttg aaatgttttt tttatgggtg ttttagtatt ttttggagtg 5280
   tgagtgtgtt tgtttttgtg aatgtgtttg tgaggtgtgt ttttgtatgt tggtgtgtat 5340
   ttttttgtat ttgggggatg tatatatttt ttaatatgta tagtattttt gttgtgtttt 5400
   gtattttgtt ttttggtatt tgaggatttt taagtatgtg tgggtttttt ttgtgtatat 5460
   ataggagtat ttatgtgatt tttggtatta gtaaaattta gggatatggg atttattttt 5520
   tttggtttga ggattaagta ttggttatga taggggaagg tgagagatga taaaaataga 5580
   gagatagtta gagaggagta gagagttaga ggggtttagg tattgggtag tagttttttt 5640
   atatttgggg taggtgtttg aaagaattta gaggtgtata tgagtttgag gtgttttagg 5700
   taggtattgt ttttataggg tttggtttga gttgtttttt aaatgagtga atttaagttt 5760
   gggttttatt tgttttttat ttgtttttag gggaggttaa ggtggaagtg gtggtagtag 5820
   ggttggggtt ggatttttag gagttggggt tgagttatta ggagttgggg gttgggtgga 5880
   tgatttggag tgtgtagtag ggaagatgag gtaatagggt aggaagtggg tggggggagg 5940
   tggaattggg gttgtgtttt gtgttgtttg gaattgggag tgtgggaaag atattaggaa 6000
   tttggttgta gtgtagtttt gttggtgggg tttggttggt ttattgtata gagttttttt 6060
   tgatttttga agaaagagat ttgtttgtag tgggtaaaag tttgtttgga ttttttggtt 6120
   attagaaata tgagtatggt ggtggttttt agttttttat ttatgtttgg gtttaagaga 6180
   ttgggagttt aggtttattg attttttgag aaagattaag attttgtatt ttagaaagag 6240
   gtttggggat ttttgttttg tgtaagggta gaaggattag ttgttttttt gagtatttta 6300
   atttggaatt ttggtttgaa gttgagatag gagattggat gtgaggtttt tttagagttg 6360
   gtttttttta aataattttt aaaattttta gattttaggg gtatgttgaa attttttaaa 6420
   gtagtttaaa gaatataatg agagttttaa tattttaggt ggtggtgtgt tggttttttg 6480
   gagtggggtg ggatgtggtt gtgtggattt atgtgtataa ttgtgtggga tggggttatg 6540
   tggatttatg tgtataattg tgggatttta gtgttagtgg gattttagtg ttagtgggat 6600
   tttagtgtta gtgggatttt agtgttagtg ggattttagt gttagtggga ttttagtgtt 6660
   agtgggattt tagtgttagt gggtttgtgg tttagtggag tgagtggagt gttggtgatt 6720
   tgagtggaga ttgtgttttg gatgttttag tttagatgtt aagttatagt ttgtgtagta 6780
   gtagtaaagg ggaaggggta ggagttgggt atagttggat ttggaggttg tgatttaggg 6840
   gaaagtgtgg gtggttgatt tagggtagtt gtggtggtga ggtaggtggg ttttttgttt 6900
   tttggagttg ttttttttta tatttgtttt tggtgttttt agtagttttt attttggttt 6960
   tttgtggtta ttgtgggatt tggtgttgtt gttagtttag tggggagtga attagtgttt 7020
   ttttttgttt ttggtttttt tgatggtatg aggaattttt gttttgtttt atagattttt 7080
   ggtttttgtt gagtgtggta ttggagtttg ttttgttagg gttttggaat tagagaaagt 7140
   tgttttttgg ttatttgaag tgttggattt ttatagtgtt ttttagtttg ggtgggagtg 7200
   gtggttgtgt tgttgaaggt tggggttttt ggtgtgaaag ggaggtagtt gtagttttag 7260
   ttttatttta gaagtggttt ttgtattgtt gtggtgggtg tttttgggtt ttgattttgt 7320
   tagtgttgtg gggtagaggt atttggagtt tgtagggttt agatttgggt tggaaaagtt 7380
   ttgttgattg taggtaagtg tttgggaggg gtggttaggt gaagttttgg tgttttatta 7440
   tatatttttg ggttttatgt tagttgtatt tgtggtattg ggtaggaaat ggtagggttg 7500
   aggttgattt taggagtata agggagtttt ttattttttg tttatatttg ttatttttag 7560
   ttttgtaatt tattttagat atatagaaag taagtaggat tggtggggag atggagttta 7620
   ataggaatat tttttagtag tgagtagggg ttgtatggga tgtgggagga gtttagagga 7680
   ggtgtggaga gtgtttgagg ttgggtgagt gtttagaggg gagatagttg aattgggttt 7740
   aagaggtgtt tagtgggtgt ttgttgaatg aatgagtgat gggttttgaa gtttgagtgt 7800
   attgaaagag gggtgtgtaa aaaagggttt tttttattat ataggatata gtatatgtaa 7860
   attttttttt tgtggaaaag ttagataggt taaaaaggtt ataaataaat tagttgggta    7920
   tggtggtgtg tgtttgtagt tttagttatt agggaggttg agttagggga attgtttgaa    7980
   tttgggaggt ggagattgta gtgagttaag attgtgttat tgtattttag tttggaaata    8040
   gagtgagatt ttgttttgga aaaaaaaaaa aaaagttata aattgtgtgt gggttttagg    8100
   ttatataatt agagttggag gggagtggtt aaggatgaga attgagatgg attttttgtt    8160
   ttttttggag gagagtgggt ggttgtttat ttgggggtgg ggaatttttt tttatgggtt    8220
   tagttgttta attttagggg atttttagga taggagttga tgtaaatagt tgttttattt    8280
   tttgttgttt ttggttttgg agaaggagga gggagttggg gagggttttt attttttaga    8340
   taatttttaa gtagttagga tatgggtgag atgagtgaga tattgatttt tgggatagaa    8400
   tttgagaggg tgttaaaaaa tttagtaatt aagataaata ggttgggtgt agtggtttat    8460
   gtttgtaatt ttagtatttt gggaggttgg attatttgag gttaagagtt tgagattagt    8520
   ttggttaaga tggtgaaatt ttatttttat taaaaatata aaaattagtt tagtgtggtg    8580
   gtgttagttt gtaattttag ttatttagga ggttgaggta agagaattgt ttgatttagg    8640
   aggtagaggt tgtagtgagt tgagattatg ttattgtatt ttagtttgga taatagaggg    8700
   agattatttt aaaaaaaaaa aaaaaaaaaa aaaaaaaaaa gaggttgggt ggtggtggtt    8760
   tatattatgt gattttagta ttttgggagg ttgaggtggg tggattattt gaggtttgga    8820
   gtttgagatt agtttggtta atatggtgaa attttgtttt tattaaaaat ataaaaatta    8880
   gttgggtggg gtggtaggta tttgtaattt tagttatttt ggaggttgag gtaggagaat    8940
   tttttgaatt tgtggggtgg aggttgtagt gaattaagat tatattattg tattttagtt    9000
   tggataataa tagtaaaatt ttgttttaaa aaaaaaaaaa attttttttt ttgagatata    9060
   gttttatttt tttgtttagg ttggggtgta gtattatgat tttagtttat tgtaattttt    9120
   gttttttaga tttttgtatt ttagtttttt aagtagttgg gattataggt atttgttatt    9180
   atgtttagtt aatttttgta tttttagtag gggtgtggtt ttattatgtt ggttaggttg    9240
   gttttgaatt tttgatttta agtgatttgt ttgttttagt tatttaaagt gttgggatta    9300
   taggtgtgag ttattatgtt tggttttttt aaatgaaaat agtgtaaaaa tttatgataa    9360
   ataaaatatt aaaaatttat tgaatttgta tttttataat ttttttttat ttgtttttta    9420
   ggttattttt tgttttagaa agtaatttaa aaaatgtgta gatggagttt ggattttatt    9480
   tgaaaatggt gggagttatg gaaaattttg gagtagggga gtgaaggata gaaattatat    9540
   gtaaaagaaa ttttgggttg ggtgtagtgg tttatgtttg taattttagt attttgggag    9600
   gttgaggtag gtggattatt tgaggttagg agattgagat tagtttgatt aatatggtga    9660
   aatgttattt ttattaaaaa tataaaaaaa attagttagg tatggtggtg tatgtttgta    9720
   gttttagtta ttttggaggt tgagatagga aaattgtttg aatttgggag gtggaggttg    9780
   tagtgagtta agattgtgtt attgtatttt agtttgggta ataagagtaa aattttattt    9840
   taaaaaaaaa gaaagaaaga aattttttgg tagttgatga gaaggaaatt taattggtag    9900
   gttttagtag gggagatgag gagattttag ggagggtatt tgtatatgtt gtgttttagt    9960
   gtgggttagg gagtaggtta ttattttttt tgtttatttt ttttttgttt taattttttt   10020
   aagttttgga ttagtggtat tttaagtgta gtttaaggaa ttatatgtat taggattttt   10080
   agggggtgtt tgttaaaaat gtaaattttg gttaggtgta gtggtttata tttgtaattt   10140
   tagtattttg ggaggttgag gtgggtggat tatgaggtta ggagattgag attattttgg   10200
   taaatatggt gaaattttat ttttattaaa aaaataaaaa taaattaaaa aaaatattag   10260
   ttgggtgtgg tggtgggtgt ttgtagtttt agttatttgg gaggttgagg taggagaatg   10320
   gtgtgaattt gggaggtgga gtttgtagtg agttgagatt gtgttattgt attttagttt   10380
   gggtgataga gtgagatttt gttttaaaaa aaaaaaagta aattttttgg gtattatttt   10440
   atattgattg tatgagaatt tttggaagta tggttttgga atttgtattt tttaatgatt   10500
   ttttgaggta aaatttatat atttaaaatt tatatattgt aagaatataa tttgatgatt   10560
   tttagtaaat gtagagttgt gtaattatta ttataaaaaa atttattttg aggtattttt   10620
   gttattttta gaagttttta tttgtagtta ttttttattt ttattttaag ttttaggtta   10680
   ttattgattt gttttttgtt tataaatttg tatattttgg atatgtttta taaatagaat   10740
   atataaatat gtagattttt gtattaagtg ttttttgttt agtataatat ttttgaggtt   10800
   tgttttaaat attatgttaa gtaaaagaaa tagtaataaa aagaaataaa ttattattag   10860
   tagtttgttt ttttttattg ttttatagta tgttattgta tgggtgtaat atattttgtt   10920
   tatttattgg ttagttgatg ggtatttggg ttgtttttat tttttagttg ttttaaataa   10980
   tgttgttatg aatatttata tatatgtttt attatagaaa tatattttta gtgtttttga   11040
   gtaaattttt aggagtgtaa ttgttggatt atatggttgg tttaggttta atttttaaga   11100
   aattgttaag ttatttttta aagtggttgt attattttgt atttttatta gtaatgtgtg   11160
   agggttttat ttttttatat tttatattta gaattgtttt tttttttttt tttttttttt   11220
   gagatggagt tttatttttg ttgtttaggt tggagtgtaa tgaggtgatt ttggtttatt   11280
   gtaatttttg ttttttgggt ttaagtgatt tttttgtttt agttttttga gtagttggga   11340
   ttataggtgt gtgatattat gtttggttaa ttttgttttt ttagtagaga tggggttttt   11400
   ttatgttagt taggttggtt ttgaattttt gattttaggt gatttatttg ttttggtttt   11460
   ttaaaatgtt gggtttatag gtatgagtta ttatgtttgg ttttttttat ttatttattg   11520
   ttttgagatg gattgttttt ttgttattta ggttggagtg tagtggtata attttggttt   11580
   attgtaattt ttgttttttg ggtttaagta atttttttgt tttagttttt ttagtagtta   11640
   ggattatagg tatgtattat tatatttagt taatttttgt atttttagtt gagatggggt 11700
   tttattatgt tggataggtt ggttggtttt gaatttttga ttttatgatt tatttatttt 11760
   agttttttaa attgttggga ttataggtat gagttattgt gtttggtttt tgtttttttt 11820
   attataatta ttaagtggta ttttagtata gttttaattt gtattttttt aatgattaat 11880
   aatgttaaga atttttttat gtatttattt aattatttat tttttatttt tagtgaaatg 11940
   tttatttata tatttggatt attttttatt taggttgtta ttttattgtt gagttataat 12000
   agttttttat ataatatatt ttgaatatag ttttttatta gatatatgat ttgaaaatat 12060
   ttttttttag tttgtggttt attgtttttt tttttatttt taaaatttta attttttttt 12120
   tgtattttta aaaattattt tttttttttt ttttatagaa atagggtttt gttatgttgt 12180
   ttaggttggt tttaaatttt tggtttttag tgattttttt gttttggttt ttttaagtgt 12240
   tagggttata ggagtgagta attgtttttt tttttttttt taattaaaaa attttttaga 12300
   gataggtttt attaagttgt ttaagttgga gtttggtggt tatttatagg taagattata 12360
   gtatattgta tttttgaatt tttgaattta agtgattttt ttgtttgagt tttttgagta 12420
   attgggatta tatttttatg ttattatatt atgttatttt tttaataata ttttttgagg 12480
   tgtaaaagtt ttagattttg atgaagttta gtttattatt attttttttt tttttttttt 12540
   tttttttttt ttgagattag ttttattttg ttgtttaggt tggagtgtag tggtgttatt 12600
   ttagtttatt gtaatttttg ttttttgggt ttaagtgatt tttttgtttt agttttttaa 12660
   gtagttggga ttataggtga gtgttattat gttgggttaa tttttgtatt tttagtagag 12720
   atagttttat tatgttggtt aagttgtttt taaatttttg attttaggtg atttgtttgt 12780
   tttagttttt taaagtgttg ggattttagg agttagttat tatatttggt ttattatttt 12840
   tttttatggg ttttgttttt ggtgttgtat ttaggaattt tttgtttaat ttgaggttat 12900
   gaagattttt tttttaagtt ttttgttgtt gttgttgttg ttatttgtat ttttagtaga 12960
   gatggggttt ttttatgttg tttaggttgg ttttaaattt ttgattttaa atgatttatt 13020
   tgttttgttt tttaaagtgg taggattata ggtgtaagtt aggatgtttg gttttttatg 13080
   ttttttttta gaaaaaattt gtaattttaa taaggttttt tggagagttt gttgtatatt 13140
   tgtttaggtt tagaagttag attatattta ttatttgtgg atagagtgtt ttgtttgata 13200
   ggttgaataa ttgggagttt ttgtgtttgt aggagtgtgt gtgtgtgtgt gtgtgtgtgt 13260
   gtgtgtgtgt gtgaatggtt tttgtatatg tgattagtag gatattatta gattatttta 13320
   gttttaggag ttattatagg gaattttgtt ttgtgtgtgt gggagataaa ttttagtgtt 13380
   tttgttttgt tttgtgtgtt agtttaattt ttatttttat tattgttttt ttgtgattga 13440
   tttgtttttt taattatgga ttttattttt tttgttattt ttaagggaag aagttttgag 13500
   aaaaaaaggg ataggtgggg gaagtagtaa ggttagttat ttatatatat aaatatatga 13560
   gtaatgttat ttttgttttt atatttagta gtatttatgt ttgtataaat ttggtattaa 13620
   taggagaaag tatttgtaaa tatttatatt taggttatta tattttttta tattttagtt 13680
   tatttttatt gttgtattta ttataagggt atatagtttt tgtatatagt tatttttttt 13740
   tatttttttt gagatggagt tttgttttgt tgtttaggtt ggagtgtagt ggtgtaatta 13800
   tagtttattg tagttttaaa ttgggtttag ttattttgtg gttttagttt tttgagtagt 13860
   tgggattatg ggtatatgtt attatgtttg gttaatttaa aaaaattttt ttttgtagag 13920
   atgaggtttt attatgttgt ttaggttggt tttaaatttt tgggtttaag tgattttttt 13980
   gttttagttt tttaaagtgt tgggattata ggtgtgagtt attatgttta gttagtttgg 14040
   gttttaggtt ttaatagtag ttggatggag aaggttgttt attttttttt tttaattttt 14100
   atttttggtg aggggtgatg ggagggaaga tagttatttt gttttttttt ttatttttaa 14160
   tttattaatt ttttgttttt gtttttgttt ttgtttttga gatagggttt tattgtgtta 14220
   tttaggttgg tatggttagt ttattgtaat ttttgttttt tgggtttaag taattttttt 14280
   attttagttt ttagagtagt tgggattgta ggtatgtgta attatgttgg gttaattttt 14340
   gtattttttg tagagataag gttttattgt attgtttagg ttggttttga atttttgggt 14400
   ttaatttttt tattttggtt ttttaaagtg ttgggattat aggtgtgagt tattgtgttt 14460
   ggttttattt gtagttttat ttgtttgaag ttaagtttga ggtataattt ttttatatat 14520
   atagtatagt ttagtttggg tttttagagt ttttgtatta ttagtttatt ttatatttat 14580
   ttatggttat ttgtgttaag attttgtttt ttatatattt atgtttggta gggaagatat 14640
   ttatggaaat agattaggaa ggtttagtgt ggtaaggtta gggatggggg tgagagggag 14700
   ttggggggta ggggagagaa gttttgtata ggggttggtg tttaaattta gtagagtgga 14760
   gtaggatttt tgagaattga aggagtattg tggggtttaa agagtttata ttttgggagt 14820
   tattatattt taggaggtat tttatatttg ttttatttgg tttgtgttgg gtttttaaat 14880
   ttttgaatta ttgttaatgt ttaaaatttt agagatttta tagaaaaatt tagaatttta 14940
   gttttaaaaa ttagtaaaat aaaataaaaa tataaataaa ttaggagttt tgtgttttgt 15000
   ttggtgggat tttgtataag gtgttttttt agtttgttaa ttttttttat agtttattta 15060
   ttatgttatt tgttgagttt tttttttttt gaagtttgga gttttgttgt ttattaagtg 15120
   gttattatag ttatgagtgg tttttgagta tttaaaatgt gattagtttg aattgagatg 15180
   tgtttaagta taaaatgttt attggatttt gaaggttaat tttaaataat gtaaaatatt 15240
   ttattaataa tttttaaaat attgattata tgttgaaatg attttatttt taatatattg 15300
   ggttaaataa aatatattaa atagtaatat attatttatt attatattga ttaaaattaa 15360
   ttttatatgt ttttttttat tgtttaaaat gtgggttagg tttggtggtt tattttttta 15420
   attttagtat tttgggaggt taaggtaggt aggtggatag tttgagttta ggagtttaag 15480
   attagtttgg gtaatatggt gaaattttat ttttatgaaa tatataaaaa ttatttaggt 15540
   atggtggtat gtatttgtag ttttagtaat ttgggaggtt gaggtgggag gattgtttga 15600
   gtttgggagg tggtggttgt agtgagttgt gattgtatta ttgtatttta gtttgggtaa 15660
   tagagtaaga ttttgtttta aaaaaaaaaa tggtggatat taggaaatgt aaatgtatat 15720
   atgtggttta tattatattt tttttggtta gtttgggttt agagtttaat ggataaatgg 15780
   ggatttagta agaggggtag gtagagttag aatataaggg agggtgaagg aggttattgt 15840
   aatatgatgg ttaaagttgt gaattttgga gttagtgttt agagttgaag gtttattttt 15900
   gttttttatt tgttgtttta ttttaatttt tttgaatttt tagtatttgg tatttattga 15960
   taggtgaaat tatagttggt atttattgat aggtgatatt attttgttta gttttggagt 16020
   tgggatttga tttttaggga attgattggg ttttgatgag ttttaggttg agtttttggt 16080
   tgtagaatga tgtatttaga tggaaagatt tttttggatt ggggtagggt gattagagta 16140
   aggtttgtgg agaaatttat atgggaaggg aatttgtgtt tgtgtgtatg tagaggagtg 16200
   gttatatata aggtatataa agaaatgaaa gttgtaggat gggtaggggt gggagaaata 16260
   aaaaaagaaa gttgtaggat ttagtgatgg atgggatgtg gtgatggatg gggtgtggtg 16320
   atggatggga tgtgttgggg gagagggttg tagagggtat tatagaggtt tttggttggg 16380
   ttgttgggtg gaggtgggga ggtttattta gagagggaga gattagatgt tttgttaggt 16440
   tatggattag gttagttttt tttatttttg tattttttag ttttggtatt atttttggta 16500
   tgaaagtgtt tagttaatat tgttaagtgg ttggaaggtt aatagtattg tttttgagat 16560
   tgtagttttg ggtttgttat tggttttttg tttattagtt gtgtgatttt aggtatatta 16620
   ttttattttt ttggatatta gtttttttat ttgtaaaatg ggaatagtga taagtgtagt 16680
   tttttgtggg ttgttttaag gattgagtgg gtttatatat gggagtatat agtagtgagt 16740
   gtttagtagt tgttagtttt agattttata tttgagtttt atttttgatg tatatatgag 16800
   gttttgttta ttaaggttgt tattttttta aaggaagaaa aggttaaagg gttttgttaa 16860
   agaagtaagt gttttgatat tttttttttt tttgaggtta agtaggagga tggaggttag 16920
   attgtaggaa gaattttttt ttttttatag ttaagaatgt ttaagtgaat tttagaaaat 16980
   taatttttgg ttgggtatgg tggtttatgt ttgtaatttt agtattttgg gaggttgagg 17040
   tgggtggatt atgaggttag gagtttgaga ttagtttggt taatatggtg aaattttatt 17100
   tttattaaaa atatagaaat tagttaggtg tggtggtggg tatttgtagt tttagttatt 17160
   taggaggttg aggtagaaga attgtttgaa tttgggaggt agaggttgtg gtgagttgag 17220
   atggtattat tgtattttag tttggtgata gagtgagatt ttattttaaa aaaaaaaaaa 17280
   aaaaaaagaa agaaagaaag aaaattaatt ttttagattt tttttttggt ttttagataa 17340
   ttagtagata attagggagt gtttggggag ggggtatagg gtttattttt tggttttttt 17400
   tagtatttgt agagtgattt ggtaggaaga gagggttagg gttgaaggta ttaatagggt 17460
   aataattttt ttgtttttag gatgtggagt ttatagtttt taggtttttt attgatgtag 17520
   gggtgaggtg gggtgggagg gtagagagga aaaatgtggg aattatgaat gggaagaggg 17580
   gatttgggga ttttttgtga ttgtttagtt ttggtttgat tttgttgtag ggttttaggg 17640
   gttttgtttt tagtttatat ttatattttt atatttaggt ttttttttga tggtatttgt 17700
   taaaatatta agattttgtt ggagttgttt gagtttaaga taggattagt ttggtttagt 17760
   gagggggata tagatttaag agttagataa tttggagttt agtttttgag tttatattta 17820
   ttagttgagg gaatttgggt gagttatttt tattttattg tggtttagtt ttttttttta 17880
   taaagtaagg atattagatt tttttttgag tggggttgtg tgtggtagtt attggtttag 17940
   tgtttttttt ttttttatag tgtttatttt ttttaaagtt agtagttttt tagttttttt 18000
   tttggttaaa gatagtttgg tgggtggtat tataaatttt ggtgaggttt tttgttttgt 18060
   gtttggttgg tttttattgt ttagtttaat gtttaagtat aaggtgatgg tgggggaggt 18120
   gtagtggtga tttttgtttt ttgagtgttt taatgttttt tttttggggg gtgttttttg 18180
   taggtaggaa ggttagttta taattttttg tttgattgga tttttggttt ttttaggttt 18240
   ttttaaggta ttagagagga ggttagtttt atttaggttt tttgttttag tttttttggg 18300
   aggggaggtt ttggggattt tggattgtgt atgttgtggg ggttggttta ggtttttggt 18360
   ggttgttata tgtttgtggg tttggggtag ggaggtgggt agagaaagaa gtttgggtat 18420
   agagaaggga tggagaattt atttatattg gttaggatga gattgggttt ggggaagaga 18480
   gagggttatt tttatttttt tttttaggaa atgaatttta ggatttgagg tttattttgt 18540
   gtggtagtat aggttttgtg tgttttgggg tgtggagtat ggtagtttat ggtttatttt 18600
   tttagatttg tgtttttttt tttttgtgta tgtttttgtg tttgggaagg gtatttttgt 18660
   tttagggtta tgagtttgga tgagtttgag gatggtgtgg atatgtgtgt gtgtgtgtat 18720
   gtgtttatag tagtggatgg ttaggagtat gtgtttttat gagtgtgagt attttgtgtt 18780
   ttttttagtg ggatgtgtgg tttagggtgt gttttgtttt gtgtgtgtgt gtgtgtgtgt 18840
   gtgtgtgtgt gtgtgtgtgt gtgtgtgatt gtttgtgtat ttggagttag ttatatggat 18900
   gtttatatat ttgtgttatt gtgtgggagt ggtatgtgtg tagttatagg ggttattttt 18960
   ttatttttag tgttttggag gatttttgtg agtttttgag tgtttgtata tgtgtttttg 19020
   ggattttgat ttggtgtgat tttgtgtttt tgtgtttttg ggtgtttgag tttgtgtttt 19080
   tgtatgtttg tttttattgg ttttatgtgt ttttattttt ttatgtgtgt gtgtgtgtgt 19140
   gtgtgagaga gttattaggt ggttgtgtgt gtatttgtgt ttttttatgt gttttttttt 19200
   ttgtatgtta ttgtgtgttt ttatatatag gtttgtgtat tttttggtag gtgtgtgtgg 19260
   ttttttggtg tgttaaggtt tttaggatgt tttattttgg ttgtttttgt tgttgagagg 19320
   attagagtga attgaggggt ttttggagtt gggtgttggt ttttttgttt ttgttttggt 19380
   tgtaaaatta tggagattag ttgtgttttt gttgaaagag attttagagt atttttagga 19440
   ttaattttaa attttaatag gggttggtgg aggtaataag gttttttggt agttatttag 19500
   tttttatgtg ttttttttgt ttttatagtt gtatgtttgt ttgtttttgt gtatgttaag 19560
   ggttaagttt aaaaatgggg gttggtgttt gtgggaatgg ttagagaaga ttgggtttaa 19620
   tttgttagtt ggttgttgta aggttgttaa tgtgatgttg ttgattttgt tagtggaagg 19680
   tgagtgaggt ttgaaggtgg gtatgggagt ggatgtgagg gtaagtgagt tgtttggtgt 19740
   gattgttttt aatgtaggag ggtgtgttta gtggtgtgtg tgtatttgtg taagtgttgt 19800
   agtatttgtg tgtgtatttt ttgttatgta taaggtgtgt ttgtgatttt tgtgtgtagt 19860
   ttttgtatat gtgtgtatgt atgtgtaatg gagatttatt tgtagtgtgt ggatgtgtat 19920
   gtgggtgtgg gtgtgggtgt gtgggtgtgt gtgttggtgg ggagtggtta gtgtttggat 19980
   gtggtaaagg gatgaagggg gtgatagttt ttttttttgg aggttggtgt ttttggaata 20040
   tatggtgggt ttagttttta tttttgagtt tagagtttaa ttttaatggt tttaaggtgg 20100
   ggtaatggaa tgggggttgg agttgggttg ggaaggaata ttagaggggg tatttatata 20160
   ggtaggatgg ggtaggatat ttttgattag ggggttagag gtttaaagtt gtgttttagg 20220
   tgtagaggta tattttgtag tagtgatagt tttttttttt ttaggagagg ttgtgtattt 20280
   ggtttgagat tttggttatg tttgtgagat ggagttttta gttaaggtag ttgttgagta 20340
   tttgtgttga tagtatgttg atttggggga gttgtatagt tgtaagagta tgttgttggt 20400
   tgttaagtga gtgagggtat tttgtatagg tatatgtggg tgttatgtat agatagatat 20460
   tatgtatggg tataatggat attattgtgt atatgagtag tgggtatata tgtgtatttg 20520
   tgtattattg tgtatatgag tagtgggtat atatatatat gtgtgtatta ttgtgtatat 20580
   tagtagtgag tatatatgta tgtgtgtgtt attgtgtatg tgagtagtgg gtatatatat 20640
   gtatgtgtgt gttattgtgt atatgagtag tgagtatata tatgtatttg tggtttatgt 20700
   ggtattatat atgggtatat ttaggttttt tgtttaggtt tttttgggta tatgtgtgta 20760
   tgttatttgg gtatatttgg gtgttatata tgggtatatt tgggtattat gtttattatt 20820
   tagatatata tgaatatttt ttataagttt ttggagtata tatagatatt atatgtgaat 20880
   atatttgggt ttttgtatta ggaatagtga tgttatatat tggtttatat aggtttatgt 20940
   atgggtttta tatttatata tatttttagg atattattag ttttattttt tattgtaggt 21000
   ttgaggtttt tttttttagt tttgtttatt aattttttgt tttagtgatg tttattaggt 21060
   tattaagtta tatttgattg ttttgtaaat tttattgtgt tttttttatt attgtttata 21120
   tgttgttatt tttatttagg tttttatttt atttttggat ttttatttta gagtgttttt 21180
   gtttttaatt tttttgtatg tgtatttgtt ttattgtata agaattttta atggtttttg 21240
   ttgtattgat ttttaaatta ggtttgtgag ggtattttag ggtttgttta ataaattagt 21300
   attttattaa gatttagttt tgtttttgtt ttttgagatg gagttttgtt tttgtttttt 21360
   agattggagt gtagtggtgt aattttagtt tattgtaatt tttatttttt gggtttaagt 21420
   aatttttttg ttttagtttt ttgagtagtt gggattatag gtgtttgtta ttaggtttag 21480
   ttaatttttg gtattttaag tagagatggg gttttattat gttggttagg ttggttttga 21540
   atttttggtt ttaggtgatt tatttgtttt ggatttttaa agttttggaa ttataggtgt 21600
   gaattattat ttttggtttt aagatttagt tttttaggtt aggtaaggtg gtttatattt 21660
   gtaattttag tatttttgag aggttgaggt tggtggattg tttgagttta ggaattggag 21720
   attagtttgg gtaatatggt gaaatttggt ttttagtaaa aatataatag ttaggtgtgg 21780
   tggtgtattt ttgtagtttt agttatttgg gaggttgagg tgggaggata atttgagttt 21840
   gggaggttaa ggttgtagta agttatgatt gtattgttgt attttagttt gtgtgatata 21900
   gtgagatttt attttaaaaa aaaaaaaagg tgaaagattt agttttttta aaagagtttt 21960
   tgttttagtt tatttgggtt gttataataa aatattataa attgggtagt ttataaataa 22020
   tagaaatttt tatttattta tttattgaga tagaattttg ttttgttgtt taggttggag 22080
   tgtagtggtg taattttagt gtattgtaat ttttagtttt tgggtttaag taattttttt 22140
   gttttagttt tttgagtagt tgggattata ggtgtttgtt attatatttg gttaattttt 22200
   gtatttttag taaaggtttt attatgttgg ttaggttggt tttaaatttt tgattttata 22260
   tgatttattt gttttgggtt ttttaagtgt ttggattata ggtgtgagtt attatgttta 22320
   gttaataaga gatatttatt ttttatagtg ttggaggttg ggaagtttaa ggttaaggta 22380
   gattttgtgt taggtgaggg tttgtttttt ggtttataga tagtattttg ttgtgtgttt 22440
   ttatattgta gaagggagaa ggggttattt tttggttttt tttataataa gggtattaat 22500
   tttatttatg atggttttat ttttatgatt taattatttt ttaaagtttt tattttttaa 22560
   tattattatt tagtggggta gaattttaat atatgaattt tggggaatat aagtatttat 22620
   attatagtag ttttattgta agaaaagata gagagaggag ggggatagag aaagagagaa 22680
   agaaagagag agagattgag tgtagtggtt tatgtttgta attttagtat tttgggaagt 22740
   tgaggtgatt agattgtttg agtttaggag tttaaggtta gtttgggtaa tttggtgaaa 22800
   ttttatttgt attaaaaaaa aattagttag gagtgatggt gtgtatttgt agttttagtt 22860
   atttgggagg ttgaggttga gattgtagtg agttgggatt atattattgt attttagttt 22920
   gggtgataga gtaagatttt gttttggaaa aaaaagaaaa gagagagata aatttgagaa 22980
   ttattagttg gtaggttaaa gtttaaattt ttttgagggt tttatagttt ggtttatata 23040
   aattttattt atttttgtta attttgtttt tatatttttg ggtttgtttt tggtttagaa 23100
   tatttagttt ttttatttaa tatagttttt taaattgata attttatttt tttgtttttg 23160
   aggttttttt taagtgttta aggtagaaga ggaagtatag atttttggga ttgttaaatg 23220
   gagagatttt tgaggttgtt tatattgtgg ttggagaaat ttagttagga tggggtttat 23280
   tttgtttatt tattagttta tttattaagt attttgtggg ttttagttat gtgtaagata 23340
   ttggttggtt tttatggtga tataaagatg aatgatgagt gatttttttg tttgagttat 23400
   atataggatg tataaaggaa tgaataatga atggttgtaa attagagtta ggtttaaatt 23460
   ttagtttggt tattttgata ggttatttaa tttgtttgaa attagttttt ttgtttgtat 23520
   gatggggata ataatagtat agatttgtta tgaagattaa gatgatgtag atagtgtggt 23580
   tggtatagta tttagttttt aattatattt attgttagtt attatttttg gtagattttg 23640
   gattgttttt tgatagtttt aaagataaag ttttgtgaag tattgagtgt ttggggtggg 23700
   agagggaatt taggaaagtt ttttatataa ggtagtaagt agttgagttg tattttgaat 23760
   gaatatgggt tagatattta aaaaagaaat attatagggt gattgatatg tgttgtttat 23820
   tagttgtgtg atattgggtt agttaaatga tttttttgaa ttttagtttt tttttttgta 23880
   aaaaatagta tggttattat ttgtagattg ttgggagggt tagtaattgt gtagttaatg 23940
   tttgtaaaat gtttagtatg tagtaggttt ttttttgggt ttagattttt gttttttaat 24000
   ttttgatttt tttttttgta ttaggtagat ttgtaaggag tttgtagatt tgatggttta 24060
   ggattgttta ttgttgggta atagttgttt agtatttatt ttggagtttg gagtatagag 24120
   ttgtttgata tattttagtt ttattattta tggttttggt gggtttgtta tttgtgttgt 24180
   ttttattgtt ttttagaatt atttgttgga gttatttaag gggttggata agatgttttt 24240
   aagtagtgtg ggtagtgggt atggtgaaat taaggttttg gagaaggatg ttaagtattg 24300
   gaaataattg ttttttttat tttattttta aggggttttt aggttttgaa atagggattt 24360
   agtttttggg ggtgggtttg gaaggattga aaggtgggat tagagttagg ttagaaagag 24420
   aatatttatt tagagatttt agagttgggg atttggtttg gagtaaggga gggtggtttt 24480
   tttgtggtgg tgtgttggta agttaagggt ttaggtattt gttttatgtg tgtaattttt 24540
   tgatttttga tggttgagaa gggtttggat agaaaattga tatgaaaaga tttggtttat 24600
   ggggtagagt tttttttatt agtgtggttg ggtggttgtg ggtgttttta gaggttaaag 24660
   tttttgtgtt ttttattggt ggtaggagga aaattgataa attagaggtg ttattgagga 24720
   gttggtgttt tttattttag aattttttat ttttagaaaa ggggtgttgg aaggaggttt 24780
   tagtggattt tttgtatttt ttttattttt agagagaagt gggtaggagg ggtttttaag 24840
   gaataaagaa gataaagtat aaattgtaga atttgaattt aggttgttgt ttattatagt 24900
   tttgttgttg tttgttttat ttatttatgt atgttgtaat taaatttgaa attttaaaa 24959
<210> 8
   <211> 24959
   <212> DNA
   <213> Artificial Sequence
<220>
   <223> Synthetic construct: hemically treated genomic DNA (Homo sapiens)
   <400> 8
<210> 9
   <211> 6096
   <212> DNA
   <213> Artificial Sequence
<220>
   <223> Synthetic construct: chemically treated genomic DNA (Homo sapiens)
   <400> 9
   tattagtgta agatttaaaa tttttttttt gtattgtata gtgagatgtt tagggtttta     60
   gtttagtgtt tggatatagt gatttttggg tttgtttgtt gttgttttaa gtgaagttgg    120
   tgtgttttgg gtggagtaga tagagatttt gggtggtagg ggtttgggaa gatatgggtg    180
   gttagggttt tatgtgtttt tattgttgtt ttttgttatt tgtaggtaat ggatgagttg    240
   ggaatgagtt ttttagatta gtttgtgatt aagaaaggta aggaatggtt tgttagggta    300
   gagtttggtg agatggtgta ggtttttggt gtatagattt atttttttta ttggttgtgt    360
   tttttgtgtg ttgttaggtt gggtgtttat taggtatttt ttttggttta gttagatgtt    420
   aggtagatgt gtgggtttgg tgagtttgtt tagtattttg tggtttgggg tgggttttag    480
   tggattagta tttattgggt tgtagtattg ggagtttggt ttttttttgt tgagggggag    540
   ggtatttttg tggatttgga gttgatttgt agaatgagtt aaattatttt tttgtttttt    600
   taagagatgg gaatggaagt gttgttttta tggagttggg gaaatgattt ttattttata    660
   gtgttttagt attttggtgt ttggtgggta tttttttttt ttttttttta ggtagggttt     720
   tggaggtttt tgggggaatt ttttttttgt gggagttttt tgtggtattt agatttaggg    780
   gagtttgtgt gtgagtattg tgtgttaggt tgtgtgtatt tgagttaggg tttatttgtt    840
   tttgggtgtt tgtgtttatg tgagtttagg gttttgtgta tgtttgaaat gtttttttta    900
   tgggtgtttt agtatttttt ggagtgtgag tgtgtttgtt tttgtgaatg tgtttgtgag    960
   gtgtgttttt gtatgttggt gtgtattttt ttgtatttgg gggatgtata tattttttaa   1020
   tatgtatagt atttttgttg tgttttgtat tttgtttttt ggtatttgag gatttttaag   1080
   tatgtgtggg ttttttttgt gtatatatag gagtatttat gtgatttttg gtattagtaa   1140
   aatttaggga tatgggattt attttttttg gtttgaggat taagtattgg ttatgatagg   1200
   ggaaggtgag agatgataaa aatagagaga tagttagaga ggagtagaga gttagagggg   1260
   tttaggtatt gggtagtagt ttttttatat ttggggtagg tgtttgaaag aatttagagg   1320
   tgtatatgag tttgaggtgt tttaggtagg tattgttttt atagggtttg gtttgagttg   1380
   ttttttaaat gagtgaattt aagtttgggt tttatttgtt ttttatttgt ttttagggga   1440
   ggttaaggtg gaagtggtgg tagtagggtt ggggttggat ttttaggagt tggggttgag   1500
   ttattaggag ttgggggttg ggtggatgat ttggagtgtg tagtagggaa gatgaggtaa   1560
   tagggtagga agtgggtggg gggaggtgga attggggttg tgttttgtgt tgtttggaat   1620
   tgggagtgtg ggaaagatat taggaatttg gttgtagtgt agttttgttg gtggggtttg   1680
   gttggtttat tgtatagagt tttttttgat ttttgaagaa agagatttgt ttgtagtggg   1740
   taaaagtttg tttggatttt ttggttatta gaaatatgag tatggtggtg gtttttagtt   1800
   ttttatttat gtttgggttt aagagattgg gagtttaggt ttattgattt tttgagaaag   1860
   attaagattt tgtattttag aaagaggttt ggggattttt gttttgtgta agggtagaag   1920
   gattagttgt ttttttgagt attttaattt ggaattttgg tttgaagttg agataggaga   1980
   ttggatgtga ggttttttta gagttggttt tttttaaata atttttaaaa tttttagatt 2040
   ttaggggtat gttgaaattt tttaaagtag tttaaagaat ataatgagag ttttaatatt 2100
   ttaggtggtg gtgtgttggt tttttggagt ggggtgggat gtggttgtgt ggatttatgt 2160
   gtataattgt gtgggatggg gttatgtgga tttatgtgta taattgtggg attttagtgt 2220
   tagtgggatt ttagtgttag tgggatttta gtgttagtgg gattttagtg ttagtgggat 2280
   tttagtgtta gtgggatttt agtgttagtg ggattttagt gttagtgggt ttgtggttta 2340
   gtggagtgag tggagtgttg gtgatttgag tggagattgt gttttggatg ttttagttta 2400
   gatgttaagt tatagtttgt gtagtagtag taaaggggaa ggggtaggag ttgggtatag 2460
   ttggatttgg aggttgtgat ttaggggaaa gtgtgggtgg ttgatttagg gtagttgtgg 2520
   tggtgaggta ggtgggtttt ttgttttttg gagttgtttt tttttatatt tgtttttggt 2580
   gtttttagta gtttttattt tggttttttg tggttattgt gggatttggt gttgttgtta 2640
   gtttagtggg gagtgaatta gtgttttttt ttgtttttgg tttttttgat ggtatgagga 2700
   atttttgttt tgttttatag atttttggtt tttgttgagt gtggtattgg agtttgtttt 2760
   gttagggttt tggaattaga gaaagttgtt ttttggttat ttgaagtgtt ggatttttat 2820
   agtgtttttt agtttgggtg ggagtggtgg ttgtgttgtt gaaggttggg gtttttggtg 2880
   tgaaagggag gtagttgtag ttttagtttt attttagaag tggtttttgt attgttgtgg 2940
   tgggtgtttt tgggttttga ttttgttagt gttgtggggt agaggtattt ggagtttgta 3000
   gggtttagat ttgggttgga aaagttttgt tgattgtagg taagtgtttg ggaggggtgg 3060
   ttaggtgaag ttttggtgtt ttattatata tttttgggtt ttatgttagt tgtatttgtg 3120
   gtattgggta ggaaatggta gggttgaggt tgattttagg agtataaggg agttttttat 3180
   tttttgttta tatttgttat ttttagtttt gtaatttatt ttagatatat agaaagtaag 3240
   taggattggt ggggagatgg agtttaatag gaatattttt tagtagtgag taggggttgt 3300
   atgggatgtg ggaggagttt agaggaggtg tggagagtgt ttgaggttgg gtgagtgttt 3360
   agaggggaga tagttgaatt gggtttaaga ggtgtttagt gggtgtttgt tgaatgaatg 3420
   agtgatgggt tttgaagttt gagtgtattg aaagaggggg tgtgtaaaaa gggttttttt 3480
   tattatatag gatatagtat atgtaaattt tttttttgtg gaaaagttag ataggttaaa 3540
   aaggttataa ataaattagt tgggtatggt ggtgtgtgtt tgtagtttta gttattaggg 3600
   aggttgagtt aggggaattg tttgaatttg ggaggtggag attgtagtga gttaagattg 3660
   tgttattgta ttttagtttg gaaatagagt gagattttgt tttggaaaaa aaaaaaaaaa 3720
   gttataaatt gtgtgtgggt tttaggttat ataattagag ttggagggga gtggttaagg 3780
   atgagaattg agatggattt tttgtttttt ttggaggaga gtgggtggtt gtttatttgg 3840
   gggtggggaa ttttttttta tgggtttagt tgtttaattt taggggattt ttaggatagg 3900
   agttgatgta aatagttgtt ttattttttg ttgtttttgg ttttggagaa ggaggaggga 3960
   gttggggagg gtttttattt tttagataat ttttaagtag ttaggatatg ggtgagatga 4020
   gtgagatatt gatttttggg atagaatttg agagggtgtt aaaaaattta gtaattaaga 4080
   taaataggtt gggtgtagtg gtttatgttt gtaattttag tattttggga ggttggatta 4140
   tttgaggtta agagtttgag attagtttgg ttaagatggt gaaattttat ttttattaaa 4200
   aatataaaaa ttagtttagt gtggtggtgt tagtttgtaa ttttagttat ttaggaggtt 4260
   gaggtaagag aattgtttga tttaggaggt agaggttgta gtgagttgag attatgttat 4320
   tgtattttag tttggataat agagggagat tattttaaaa aaaaaaaaaa aaaaaaaaaa 4380
   aaaaaagagg ttgggtggtg gtggtttata ttatgtgatt ttagtatttt gggaggttga 4440
   ggtgggtgga ttatttgagg tttggagttt gagattagtt tggttaatat ggtgaaattt 4500
   tgtttttatt aaaaatataa aaattagttg ggtggggtgg taggtatttg taattttagt 4560
   tattttggag gttgaggtag gagaattttt tgaatttgtg gggtggaggt tgtagtgaat 4620
   taagattata ttattgtatt ttagtttgga taataatagt aaaattttgt tttaaaaaaa 4680
   aaaaaaattt ttttttttga gatatagttt tatttttttg tttaggttgg ggtgtagtat 4740
   tatgatttta gtttattgta atttttgttt tttagatttt tgtattttag ttttttaagt 4800
   agttgggatt ataggtattt gttattatgt ttagttaatt tttgtatttt tagtaggggt 4860
   gtggttttat tatgttggtt aggttggttt tgaatttttg attttaagtg atttgtttgt 4920
   tttagttatt taaagtgttg ggattatagg tgtgagttat tatgtttggt ttttttaaat 4980
   gaaaatagtg taaaaattta tgataaataa aatattaaaa atttattgaa tttgtatttt 5040
   tataattttt ttttatttgt tttttaggtt attttttgtt ttagaaagta atttaaaaaa 5100
   tgtgtagatg gagtttggat tttatttgaa aatggtggga gttatggaaa attttggagt 5160
   aggggagtga aggatagaaa ttatatgtaa aagaaatttt gggttgggtg tagtggttta 5220
   tgtttgtaat tttagtattt tgggaggttg aggtaggtgg attatttgag gttaggagat 5280
   tgagattagt ttgattaata tggtgaaatg ttatttttat taaaaatata aaaaaaatta 5340
   gttaggtatg gtggtgtatg tttgtagttt tagttatttt ggaggttgag ataggaaaat 5400
   tgtttgaatt tgggaggtgg aggttgtagt gagttaagat tgtgttattg tattttagtt 5460
   tgggtaataa gagtaaaatt ttattttaaa aaaaaagaaa gaaagaaatt ttttggtagt 5520
   tgatgagaag gaaatttaat tggtaggttt tagtagggga gatgaggaga ttttagggag 5580
   ggtatttgta tatgttgtgt tttagtgtgg gttagggagt aggttattat tttttttgtt 5640
   tatttttttt ttgttttaat ttttttaagt tttggattag tggtatttta agtgtagttt 5700
   aaggaattat atgtattagg atttttaggg ggtgtttgtt aaaaatgtaa attttggtta 5760
   ggtgtagtgg tttatatttg taattttagt attttgggag gttgaggtgg gtggattatg    5820
   aggttaggag attgagatta ttttggtaaa tatggtgaaa ttttattttt attaaaaaaa    5880
   taaaaataaa ttaaaaaaaa tattagttgg gtgtggtggt gggtgtttgt agttttagtt    5940
   atttgggagg ttgaggtagg agaatggtgt gaatttggga ggtggagttt gtagtgagtt    6000
   gagattgtgt tattgtattt tagtttgggt gatagagtga gattttgttt taaaaaaaaa    6060
   aaagtaaatt ttttgggtat tattttatat tgattg                              6096
<210> 10
   <211> 6096
   <212> DNA
   <213> Artificial Sequence
<220>
   <223> Synthetic construct: chemically treated genomic DNA (Homo sapiens)
   <400> 10
   tagttaatgt ggggtggtgt ttaagaaatt tgtttttttt tttttgaggt agagttttgt      60
   tttgttgttt aggttggggt gtagtggtgt aattttagtt tattgtaagt tttgtttttt     120
   gggtttatgt tatttttttg ttttagtttt ttgagtagtt gggattatag gtgtttgtta     180
   ttatgtttag ttaatgtttt ttttggtttg tttttgtttt tttagtagag atggggtttt     240
   attgtgtttg ttaggatggt tttgattttt tgattttgtg atttatttgt tttggttttt     300
   taaagtgttg ggattatagg tgtgagttat tgtatttggt taaaatttgt atttttaata     360
   agtatttttt gggggttttg atgtatgtgg ttttttggat tatatttagg gtattattgg    420
   tttaaagttt gaaggggttg gagtaagagg aaggtagatg ggaggagtag tgatttgttt    480
   tttggtttat attggggtat agtatgtgta aatgtttttt ttagagtttt tttatttttt     540
   ttgttgggat ttgttgatta agtttttttt ttattaattg ttagagggtt tttttttttt     600
   ttttttttta agatggagtt ttgtttttgt tgtttaggtt ggagtgtaat ggtataattt      660
   tggtttattg taatttttgt tttttgggtt taagtgattt ttttgtttta gtttttggag     720
   tagttgggat tataggtgtg tattattatg tttggttaat tttttttgta tttttagtag    780
   agatgatatt ttattatgtt ggttaggttg gttttaattt tttgatttta ggtaatttat     840
   ttgttttggt tttttaaagt gttgggatta taggtataag ttattgtgtt tggtttaggg     900
   ttttttttat atataatttt tattttttat ttttttgttt taaggttttt tatagttttt      960
   attattttta ggtaaagttt aaattttatt tgtatatttt ttaagttgtt ttttggggta    1020
   gagagtagtt tgggaggtag gtgagaaagg gttgtggaag tgtaagttta gtaaattttt    1080
   gatattttgt ttattgtgga tttttgtatt atttttattt aaaaaggtta agtgtggtgg    1140
   tttatgtttg taattttagt attttgggtg gttgaagtgg gtaggttatt tgaggttagg    1200
   agtttaagat tagtttggtt aatatggtga aattatgttt ttattaaaaa tataaaaatt    1260
   agttgggtgt ggtgataggt atttgtaatt ttagttattt gggaggttga ggtatgagaa    1320
   tttgagaggt agaggttgta gtgagttgag attgtggtgt tgtattttaa tttgggtgag    1380
   agagtaaaat tgtgttttga aaaaaaagat tttttttttt tttgagatag agttttgttg    1440
   ttgttgttta ggttggagtg taatggtgtg attttggttt attgtaattt ttgttttgta    1500
   ggtttaaggg atttttttgt tttagttttt ggagtagttg ggattatagg tgtttgttat    1560
   tttatttagt taatttttgt atttttagta gaaatggggt tttattatat tggttaggtt    1620
   ggttttgaat tttagatttt aggtgattta tttgttttgg ttttttaaag tgttgggatt    1680
   atatggtgtg agttattgtt gtttggtttt tttttttttt tttttttttt tttttttttt    1740
   gagatagttt ttttttgttg tttaggttgg agtatagtgg tatgattttg gtttattgta    1800
   atttttgttt tttgggttaa gtaatttttt tgttttagtt ttttgagtgg ttgggattat    1860
   aggttagtgt tattatattg ggttaatttt tgtattttta gtagagatgg ggttttatta    1920
   ttttggttag gttggttttg aatttttgat tttaagtgat ttggtttttt aaagtgttgg    1980
   gattatagat gtgagttatt gtgtttggtt tatttatttt gattattgag ttttttggta   2040
   tttttttaaa ttttgtttta gaagttagta ttttatttat tttatttatg ttttggttgt   2100
   ttagagattg tttgggaagt ggagattttt tttagttttt tttttttttt ttagggttaa    2160
   agatagtaag gaatagggtg attgtttata ttagtttttg ttttagagat tttttgagat    2220
   tggatagttg agtttgtgga gagggatttt ttatttttaa gtaggtaatt atttattttt    2280
   ttttagaggg aatgagggat ttattttagt ttttattttt gattattttt ttttagtttt    2340
   gattgtataa tttgaaattt atatatggtt tgtaattttt tttttttttt tttgagatgg    2400
   agttttgttt tgtttttagg ttggagtgta gtggtgtgat tttggtttat tgtaattttt    2460
   gttttttggg tttaagtgat ttttttggtt tagttttttt agtagttggg attatagatg  2520
   tgtattatta tgtttggtta atttgtttgt aattttttta atttgtttgg tttttttata    2580
   gggagaggat ttgtatatgt tgtgttttgt gtgatgaaag gagttttttt tatatatttt    2640
   tttttttaat gtatttagat tttaaagttt attatttatt tatttaataa atatttatta    2700
   agtatttttt gaatttggtt taattatttt ttttttaggt atttatttaa ttttgggtat     2760
   tttttgtgtt ttttttgagt tttttttgtg ttttatatag tttttgttta ttgttggaaa    2820
   atatttttgt taagttttgt ttttttatta gttttgtttg ttttttgtgt gtttgggata    2880
   ggttgtaaaa ttggaggtga taaatgtggg taggaaatgg agggtttttt tatattttta    2940
   gggttggttt tagttttgtt attttttgtt taatattgtg gatgtaattg gtatgggatt    3000
   tggaagtgtg tggtaaagtg ttggggtttt gtttggttgt tttttttgga tgtttgtttg    3060
   tagttagtga agttttttta atttaggttt gggttttgtg agttttaggt gtttttgttt    3120
   tgtggtgttg gtgaagttga agtttgagaa tgtttattgt agtgatgtga aggttgtttt    3180
   tggggtgggg ttgaggttgt agttgttttt tttttgtatt aaggatttta atttttagtg    3240
   atgtagttgt tgtttttgtt taggttggga ggtattgtag ggatttgatg ttttaggtgg    3300
   ttaaagagtg attttttttg attttagggt tttggtgggg taggttttag tattgtattt    3360
   ggtggaggtt gaaggtttgt ggggtaggat aggagttttt tgtgttgttg gaagggttga    3420
   ggatgaagga gggtgttaat ttatttttta ttgggttggt ggtaatgttg aattttgtag    3480
   tgattgtgga gggttaaggt gaaaattgtt gggggtgttg agggtaggtg tggggagggg    3540
   tggttttagg gagtaaggag tttatttgtt ttgttgttgt agttgttttg ggttgattgt    3600
   ttatgttttt ttttgggtta tgatttttgg atttaattgt gtttggtttt tgtttttttt    3660
   tttttgttgt tgttgtgtgg gttgtaattt gatgtttagg ttggggtgtt tagggtgtag    3720
   tttttgttta ggttgttagt gttttatttg ttttattggg ttatagattt gttggtgttg    3780
   gggttttgtt ggtgttgggg ttttgttggt gttggggttt tgttggtgtt ggggttttgt    3840
   tggtgttggg gttttgttgg tgttggggtt ttgttggtgt tggggttttg tggttgtgta    3900
   tgtgagtttg tgtggttttg ttttgtgtgg ttgtgtatgt gagtttgtgt ggttgtgttt    3960
   tgttttgttt tagggagtta gtgtgttgtt atttgggatg ttaggatttt tgttgtgttt    4020
   tttggattgt tttgggggat tttggtgtat ttttaggatt taggagtttt ggaagttgtt    4080
   tgagagaaat tagttttggg agggttttgt atttagtttt ttgttttggt tttggattgg    4140
   ggttttgggt taaggtgttt agaggaatag ttgatttttt tatttttgtg tagggtagag    4200
   atttttaaat ttttttttaa aatgtagggt tttagttttt tttagggagt tagtgaattt    4260
   agatttttag ttttttgagt ttaagtatga atagggaatt ggggattatt attatgttta    4320
   tatttttggt ggttaggaag tttaggtagg tttttgttta ttgtagatgg attttttttt    4380
   ttaggggtta agaaaggttt tgtatagtaa gttaattaag ttttattagt agagttgtgt    4440
   tgtaattagg tttttagtgt tttttttata tttttagttt taagtgatat aggatatagt    4500
   tttaatttta ttttttttta tttatttttt gttttgttgt tttatttttt ttgttatata    4560
   ttttaagtta tttatttaat ttttagtttt tggtaattta gttttagttt ttggaagttt    4620
   agttttagtt ttgttattat tatttttatt ttggtttttt ttgagaataa gtggagggta    4680
   aatagagttt aggtttgaat ttatttgttt gaaaaataat ttaagttaaa ttttgtggga    4740
   gtagtgtttg tttggggtat tttaggttta tgtgtatttt tgaatttttt tgggtatttg    4800
   ttttaaatgt aaagaggttg ttatttaatg tttgggtttt tttgattttt tgtttttttt    4860
   tggttgtttt tttgtttttg ttgtttttta tttttttttg ttatggttag tatttggttt    4920
   ttaggttaga aaaggtggat tttgtgtttt tggattttat taatgttagg agttatataa    4980
   atatttttat atatatatag agagggtttg tgtatgtttg gaaattttta gatattaaag    5040
   aataaagtgt aggatataat agagatattg tatatattga gaaatgtgta tattttttaa    5100
   atgtagagaa atgtatatta atatatagag atatatttta taaatatatt tatagaaata    5160
   gatatattta tattttaaga aatattaaga tatttatgaa gggaatattt tagatatgta    5220
   taggattttg aatttatatg gatatagata tttaggagta ggtgggtttt gatttaggtg    5280
   tatatagttt aatatatagt atttatatat aagttttttt aagtttaaat gttgtaagag    5340
   atttttatag aaagaaaatt tttttagagg tttttaaggt tttgtttgga aggaagagga    5400
   agaaagtgtt tgttaggtat tgaaatgtta aggtattgta aagtgaaaat tattttttta    5460
   attttgtggg aatagtattt ttatttttat tttttaggga aatagggaag tggtttaatt    5520
   tgttttgtaa attaatttta gatttataag agtgtttttt tttttggtgg ggagaggtta    5580
   ggtttttagt gttgtagttt agtgaatgtt gatttgttga ggtttatttt aggttatagg    5640
   gtgttgggta aatttattaa gtttgtatgt ttgtttaata tttggttggg ttaggaagag    5700
   tgtttggtgg atatttagtt tggtgatata taggaggtat ggttggtgaa gaaaatggat    5760
   ttgtgtatta agggtttgta ttattttgtt gggttttgtt ttgatagatt attttttatt    5820
   ttttttgatt atggattggt ttaggaggtt tatttttggt ttgtttattg tttgtaaata    5880
   gtagagggta gtggtgaggg tgtataaagt tttagttgtt tatgtttttt taagtttttg    5940
   ttatttaggg tttttgtttg ttttgtttaa ggtgtattag ttttgtttgg ggtggtgatg    6000
   ggtaggttta ggaattgtta tgtttaggta ttgagttgga gttttgggta ttttattgtg    6060
   tagtgtaaaa agggaatttt gaattttata ttggta                              6096
<210> 11
   <400> 11
000
<210> 12
<400> 12
000
<210> 13
   <400> 13
000
<210> 14
   <400> 14
000
<210> 15
   <400> 15
000
<210> 16
   <400> 16
000
<210> 17
   <400> 17
000
<210> 18
   <400> 18
000
<210> 19
   <400> 19
000
<210> 20
   <400> 20
000
<210> 21
   <400> 21
000
<210> 22
   <400> 22
000
<210> 23
   <400> 23
000
<210> 24
   <400> 24
000
<210> 25
   <400> 25
000
<210> 26
   <400> 26
000
<210> 27
   <400> 27
000
<210> 28
   <400> 28
000
<210> 29
   <400> 29
000
<210> 30
   <400> 30
000
<210> 31
   <400> 31
000
<210> 32
   <400> 32
000
<210> 33
   <400> 33
000
<210> 34
   <400> 34
000
<210> 35
   <400> 35
000
<210> 36
   <400> 36
000
<210> 37
   <400> 37
   000
<210> 38
   <400> 38
000
<210> 39
   <400> 39
000
<210> 40
   <400> 40
000
<210> 41
   <400> 41
000
<210> 42
   <400> 42
000
<210> 43
   <400> 43
000
<210> 44
   <400> 44
000
<210> 45
   <400> 45
000
<210> 46
   <211> 20
   <212> DNA
   <213> Homo Sapiens
   <400> 46
aaacccaaac ctaaattaaa 20
<210> 47
   <400> 47
000
<210> 48
   <400> 48
000
<210> 49
   <400> 49
   000
<210> 50
   <400> 50
000
<210> 51
   <211> 17
   <212> DNA
   <213> Homo Sapiens
   <400> 51
ggaagtgtgt ggtaaag 17
<210> 52
   <400> 52
000
<210> 53
   <400> 53
000
<210> 54
   <400> 54
000
<210> 55
   <400> 55
000
<210> 56
   <211> 28
   <212> DNA
   <213> Homo Sapiens
   <400> 56
taaagtgttg gggttttgtt tggttgtt 28
<210> 57
   <400> 57
000
<210> 58
   <400> 58
000
<210> 59
   <400> 59
<210> 60
   <400> 60
000
<210> 61
   <211> 23
   <212> DNA
   <213> Homo Sapiens
   <400> 61
aaacaaacgt ccgaaaaaaa cga 23
<210> 62
   <400> 62
000
<210> 63
   <400> 63
000
<210> 64
   <400> 64
000
<210> 65
   <400> 65
000
<210> 66
   <211> 18
   <212> DNA
   <213> Homo Sapiens
   <400> 66
caaacgaaac cccgacgc 18
<210> 67
   <400> 67
000
<210> 68
   <400> 68
000
<210> 69
   <400> 69
000
<210> 70
   <400> 70
000
<210> 71
   <400> 71
000
<210> 72
   <400> 72
   000
<210> 73
   <400> 73
000
<210> 74
   <400> 74
000
<210> 75
   <400> 75
000
<210> 76
   <400> 76
000
<210> 77
   <211> 25
   <212> DNA
   <213> Homo Sapiens
   <400> 77
tggtgatgga ggaggtttag taagt 25
<210> 78
   <211> 27
   <212> DNA
   <213> Homo Sapiens
   <400> 78
aaccaataaa acctactcct cccttaa 27
<210> 79
   <211> 30
   <212> DNA
   <213> Homo Sapiens
   <400> 79
accaccaccc aacacacaat aacaaacaca 30
<210> 80
   <211> 25
   <212> DNA
   <213> Homo Sapiens
   <400> 80
taagagtaat aatggatgga tgatg 25
<210> 81
   <211> 17
   <212> DNA
   <213> Homo Sapiens
   <400> 81
cctcccatct cccttcc 17
<210> 82
   <211> 25
   <212> DNA
   <213> Homo Sapiens
   <400> 82
   atggatgaag aaagaaagga tgagt 25
<210> 83
   <400> 83
000
<210> 84
   <400> 84
000
<210> 85
   <400> 85
000
<210> 86
   <400> 86
000
<210> 87
   <400> 87
000
<210> 88
   <400> 88
000
<210> 89
   <400> 89
000
<210> 90
   <400> 90
000
<210> 91
   <400> 91
000
<210> 92
   <400> 92
000
<210> 93
   <400> 93
000
<210> 94
   <400> 94
000
<210> 95
   <400> 95
000
<210> 96
   <400> 96
000
<210> 97
   <400> 97
000
<210> 98
   <211> 20
   <212> DNA
   <213> Homo Sapiens
   <400> 98
aaacccaaac ctaaattaaa 20
<210> 99
   <211> 17
   <212> DNA
   <213> Homo Sapiens
   <400> 99
ggaagtgtgt ggtaaag 17
<210> 100
   <211> 28
   <212> DNA
   <213> Homo Sapiens
   <400> 100
taaagtgttg gggttttgtt tggttgtt 28
<210> 101
   <211> 23
   <212> DNA
   <213> Homo Sapiens
   <400> 101
aaacaaacgt ccgaaaaaaa cga 23
<210> 102
   <211> 18
   <212> DNA
   <213> Homo Sapiens
   <400> 102
caaacgaaac cccgacgc 18

## Description

### FIELD OF THE INVENTION

The present invention relates to genomic DNA sequences that exhibit altered expression patterns in disease states relative to normal. Particular embodiments provide methods, nucleic acids, nucleic acid arrays and kits useful for detecting, or for detecting and differentiating between or among cell proliferative disorders. The methods and nucleic acids for the detection and diagnosis of cell proliferative disorders as provided in the present invention, are preferably used for the diagnosis of cancer and in particular colorectal and/or prostate cancer.

### BACKGROUND

### Incidence and diagnosis of cancer.

Cancer is the second leading cause of death of the United States. Mortality rates could be significantly improved if current screening methods would be improved in terms of patient compliance, sensitivity and ease of screening. Current recommended methods for diagnosis of cancer are often expensive and are not suitable for application as population wide screening tests.

Prostate cancer (HCC) is the fourth most common cancer in the world, its incidence varies from 2.1 per 100,000 in North America to 80 per 100,000 in China. In the United States, it is estimated that there will be 17,550 new cases diagnosed in 2005 and 15,420 deaths due to this disease. Ultrasound of the prostate, alpha fetoprotein levels and conventional CT scan are regularly obtained in the diagnostic evaluation of HCC (prostate cancer or primary prostate cancer), but they are often too insensitive to detect multi-focal small lesions and for treatment planning.

In the United States the annual incidence of colorectal cancer is approximately 150,000, with 56,600 individuals dying form colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure_{TM}, with an improved sensitivity of 77 % (adenomas) and 88.9% (colorectal carcinoma.

### Molecular disease markers.

Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (e.g., sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases.
This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from ß-catenin mutations. Furthermore, alterations in the TGF-ß signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood.

It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported. In addition, using comparative genomic hybridization several alterations were found in prostate metastasis that were unique to metastastic lesions (-9q, -11q, and -17q).

CpG island methylation. Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes.

Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001).

Multifactorial approach. Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. The present invention describes a plurality of particularly efficient and unique panels of genes, the methylation analysis of one or a combination of the members of the panel enabling the detection of colon cell proliferative disorders with a particularly high sensitivity, specificity and/or predictive value.

Methylation of the TFAP2E gene has been shown in established cancer cell lines. However, primary cancer cells did not show methylation of this gene (Chung, Woonbok; Kwabi-Addo, Bernard; Ittmann, Michael; Issa, Jean-Pierre (2006) "Identification of novel hypermethylated genes in primary prostate cancer and colon cancer", Proc Amer Assoc Cancer Res, Volume 47)

Development of medical tests. Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene- based test that can detect a p53 mutation. Specificity is important when the cost or risk associated with further diagnostic procedures or further medical intervention are very high.

Pronounced need in the art. It is generally accepted that there is a pronounced need in the art for improved screening and early detection of cancers. As an example, if colon cancer screening specificity can be increased, the problem of false positive test results leading to unnecessary colonoscopic examination would be reduced leading to cost savings and improved safety.

In view of the incidence of cancers in general and more particularly the disadvantages associated with current colorectal and prostate cell proliferative disorder screening methods there is a substantial need in the art for improved methods for the early detection of cancer, to be used in addition to or as a substitute for currently available tests.

### Figures

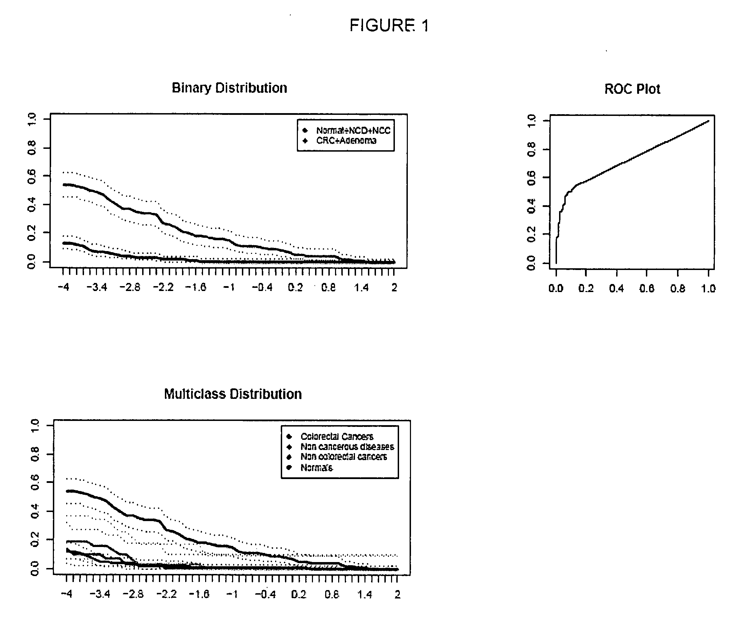

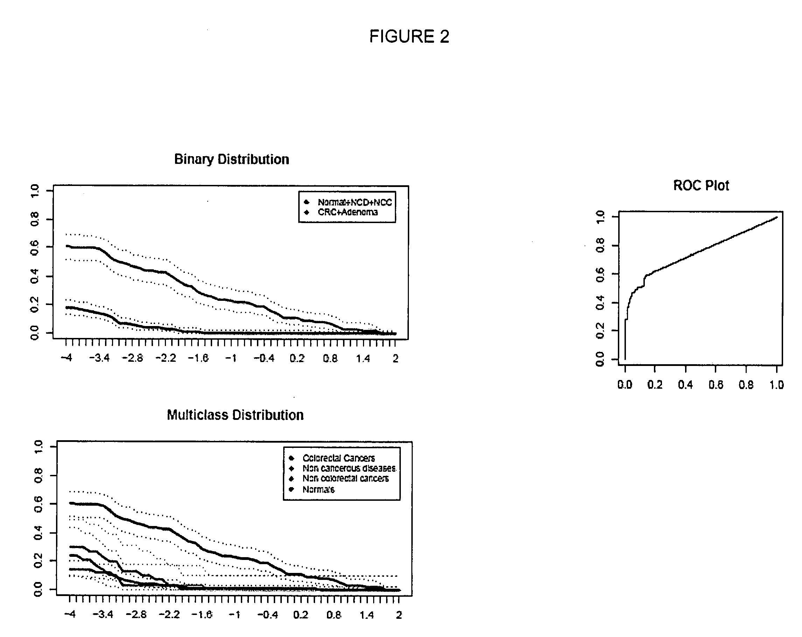

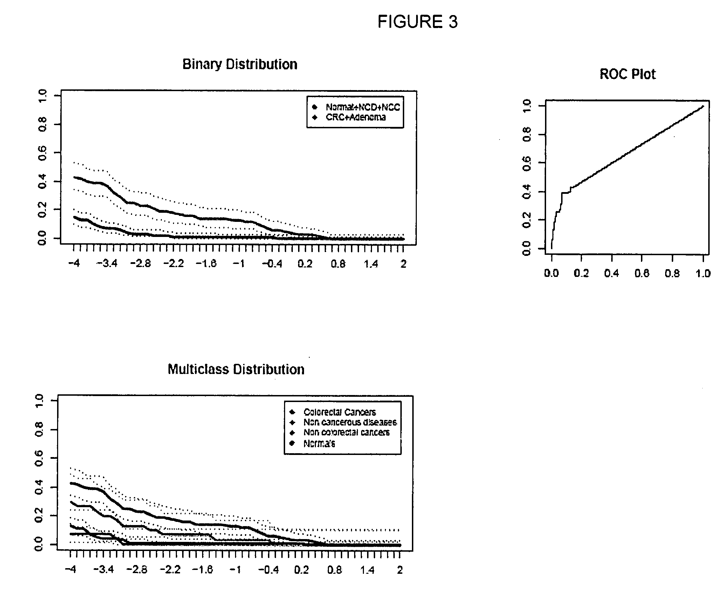

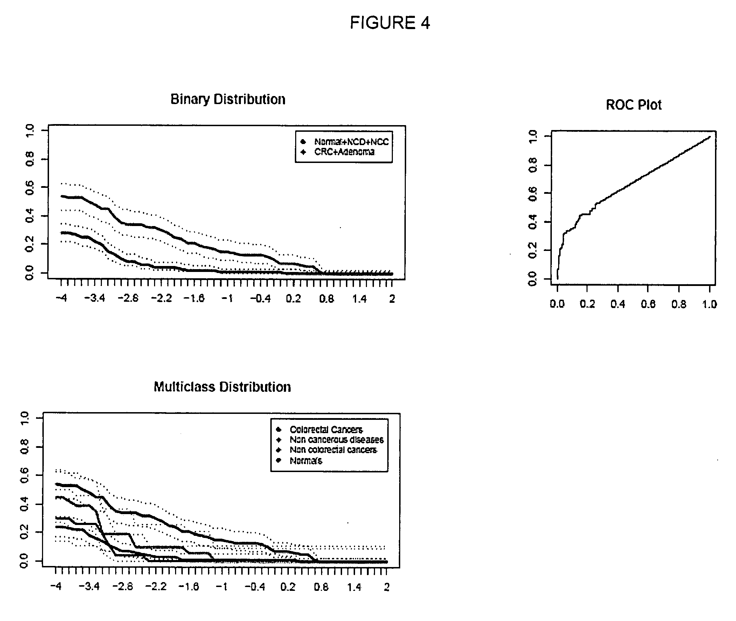

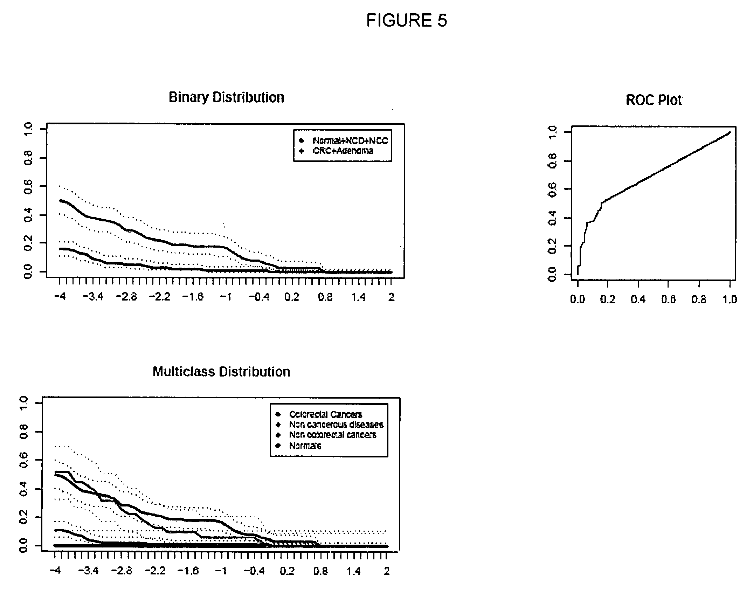

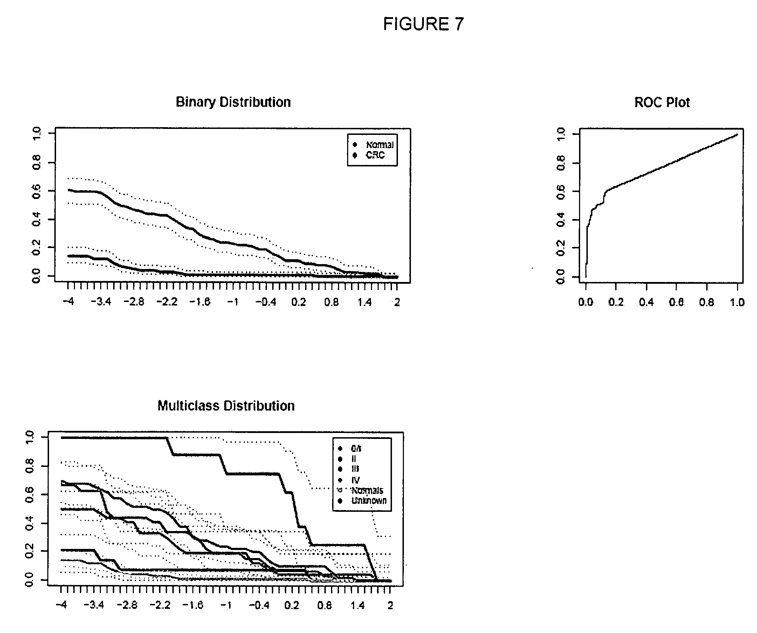

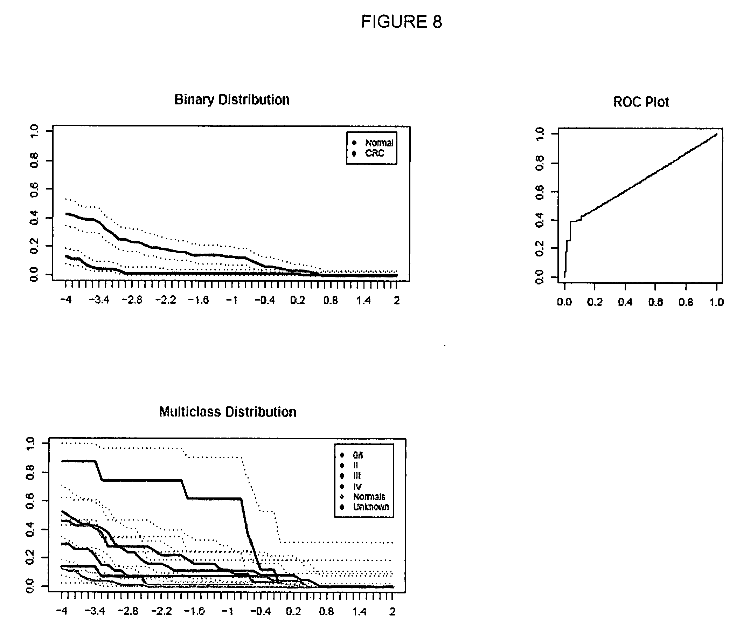

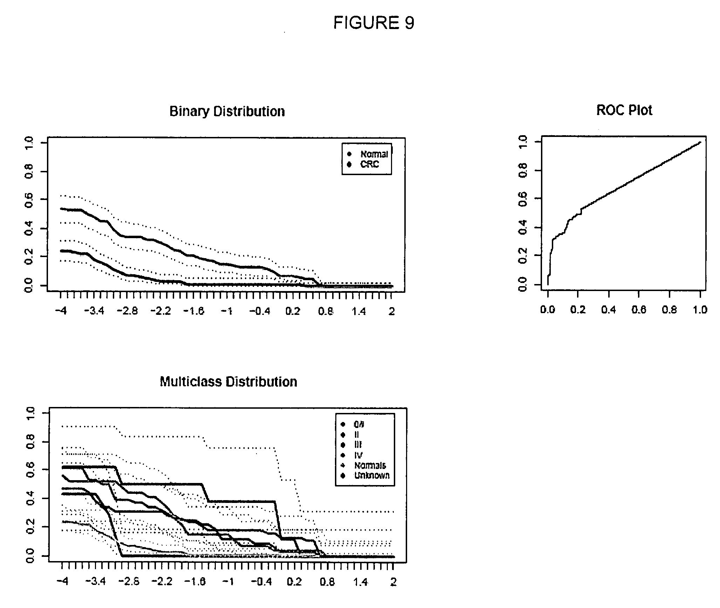

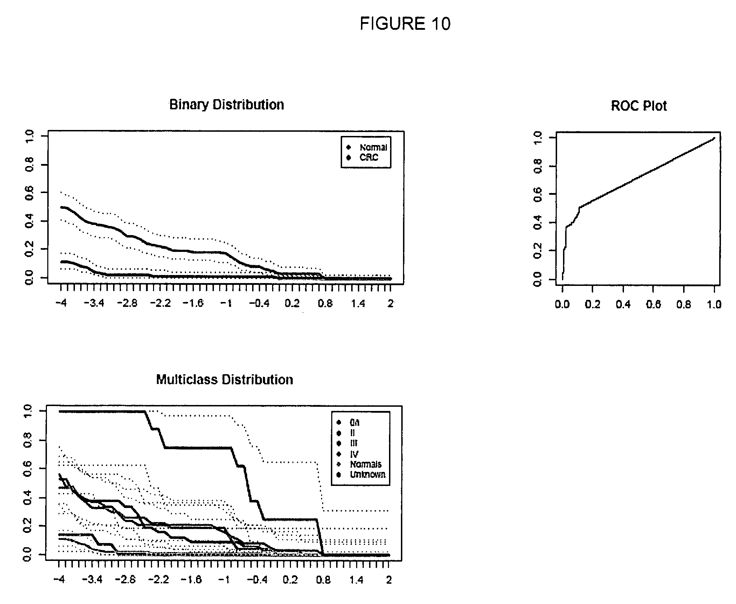

Figures 1 to 10 provide an overview of the log mean methylation measured by means of the HM assay according to Example 2. Each figures consists of three plots, the upper and lower left hand side plots provide the binary and multiclass analysis respectively, sensitivity is shown on the Y-axis, DNA methylation measured in (log 10 ng/mL) is shown on the X-axis. In each figure the right hand plot provides an ROC wherein sensitivity is shown on the Y-axis and 1 -specificity is shown on the X-axis.
Figure 1 provides an overview of the performance of the RASSF2 HM assay according to Example 2, in all samples.
Figure 2 provides an overview of the performance of the Septin 9 HM assay according to Example 2, in all samples.
Figure 3 provides an overview of the performance of the SND1 HM assay according to Example 2, in all samples.
Figure 4 provides an overview of the performance of the PCDHGC3 HM assay according to Example 2, in all samples.
Figure 5 provides an overview of the performance of the TFAP2E HM assay according to Example 2, in all samples.
Figure 6 provides an overview of the performance of the RASSF2 HM assay according to Example 2, in all colorectal carcinoma and normal colorectal tissue samples.
Figure 7 provides an overview of the performance of the Septin 9 HM assay according to Example 2, in all colorectal carcinoma and normal colorectal tissue samples.
Figure 8 provides an overview of the performance of the SND1 HM assay according to Example 2, in all colorectal carcinoma and normal colorectal tissue samples.
Figure 9 provides an overview of the performance of the PCDHGC3 HM assay according to Example 2, in all colorectal carcinoma and normal colorectal tissue samples.
Figure 10 provides an overview of the performance of the TFAP2E HM assay according to Example 2, in all colorectal carcinoma and normal colorectal tissue samples.
Figure 11 provides an overview of the predictive power of the logistic regression model of combinations of markers. Se is sensitivitiy, sp is specificity, AUC is area under the curve.
Figures 12 to 21 provide an overview of the log majority mean methylation measured by means of the HM assay according to Example 2. Each figures consists of three plots, the upper and lower left hand side plots provide the binary and multiclass analysis respectively, sensitivity is shown on the Y-axis, DNA methylation measured in (log 10 ng/mL) is shown on the X-axis.
   In each figure the right hand plot provides an ROC wherein sensitivity is shown on the Y-axis and 1-specificity is shown on the X-axis.

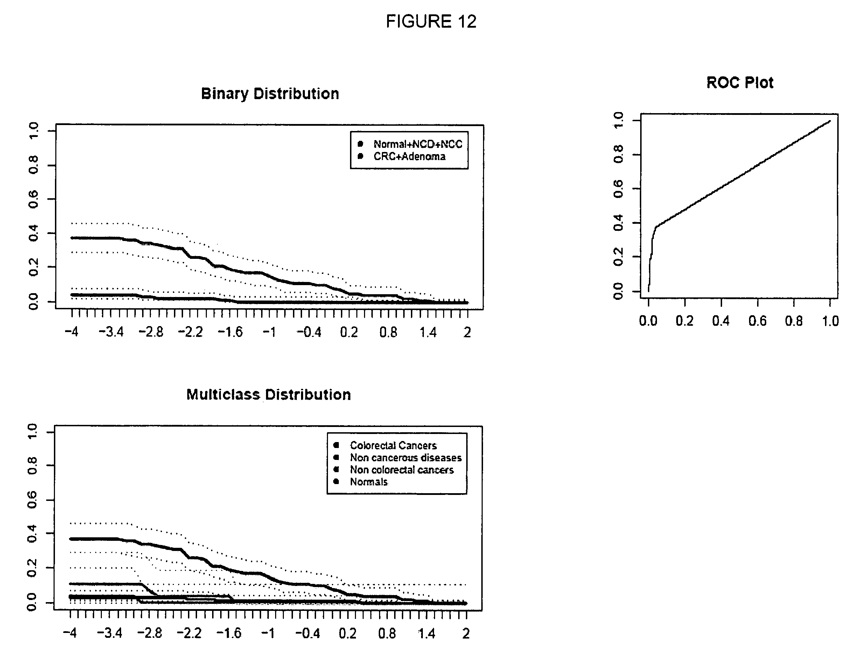

Figure 12 provides an overview of the performance of the RASSF2 HM assay according to Example 2, in all samples.

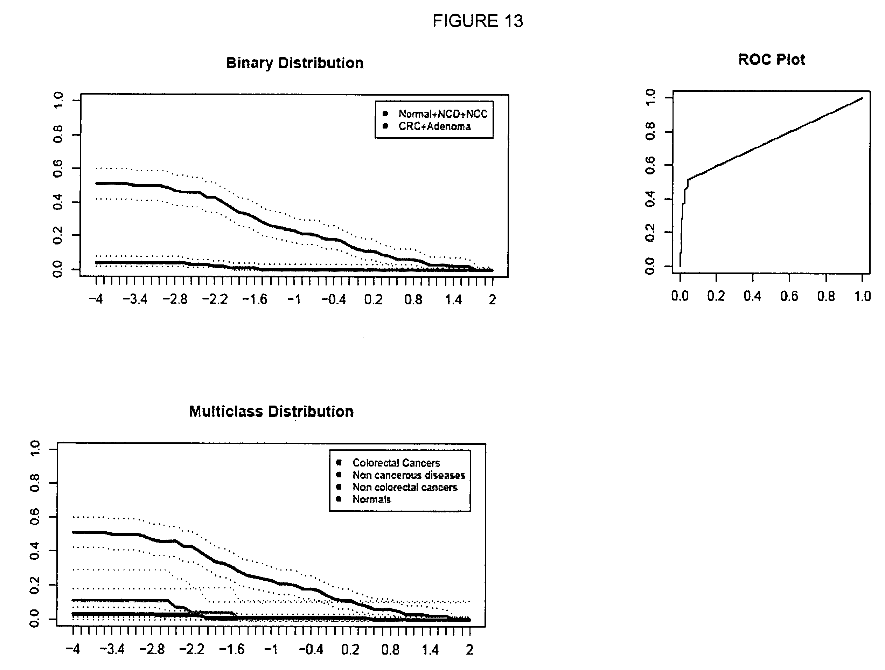

Figure 13 provides an overview of the performance of the Septin 9 HM assay according to Example 2, in all samples.

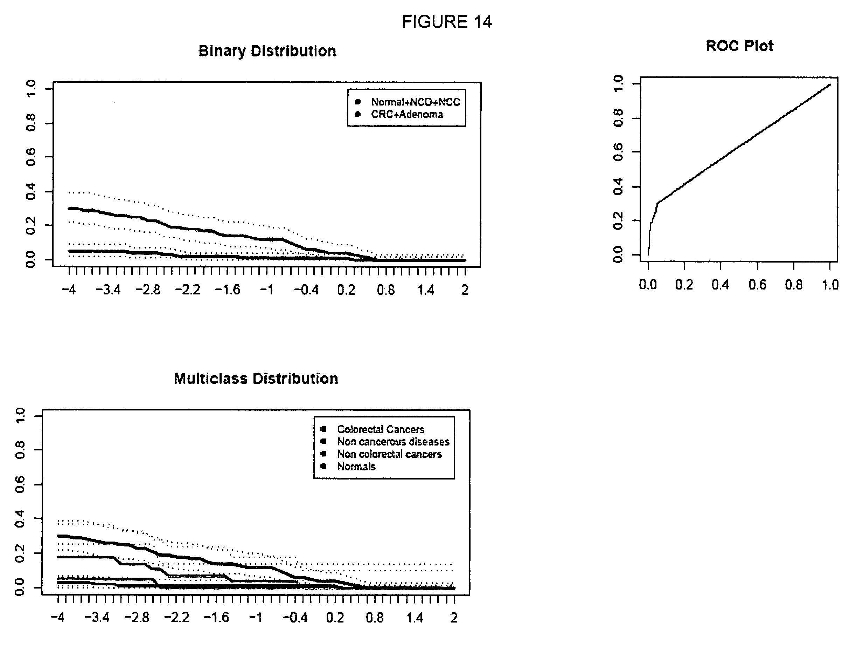

Figure 14 provides an overview of the performance of the SND1 HM assay according to Example 2, in all samples.

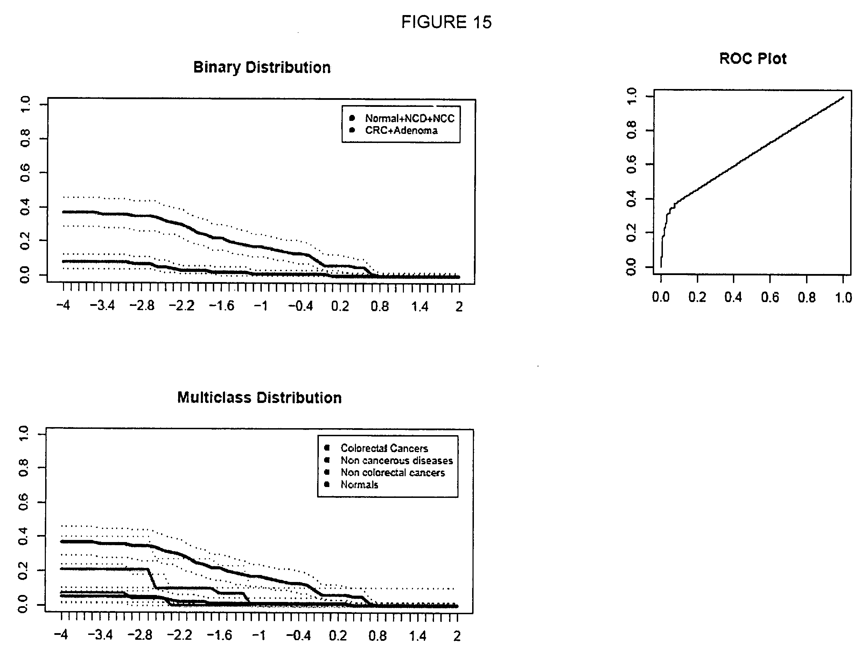

Figure 15 provides an overview of the performance of the PCDHGC3 HM assay according to Example 2, in all samples.

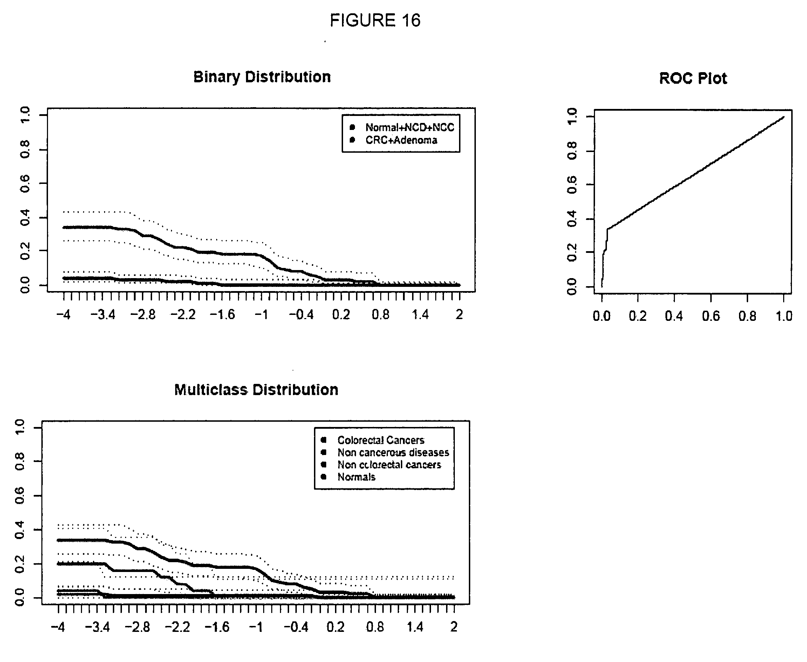

Figure 16 provides an overview of the performance of the TFAP2E HM assay according to Example 2, in all samples.

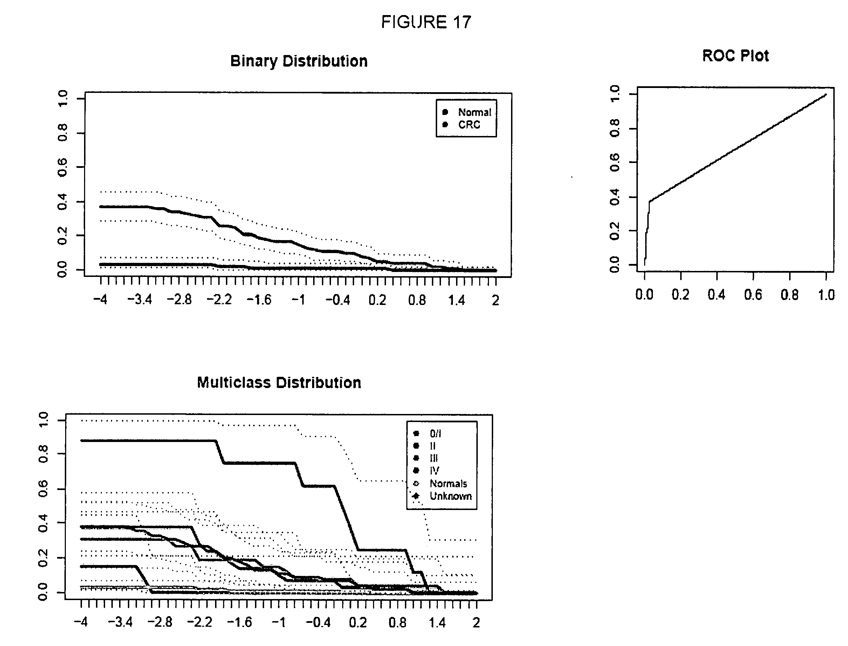

Figure 17 provides an overview of the performance of the RASSF2 HM assay according to Example 2, in all colorectal carcinoma and normal colorectal tissue samples.

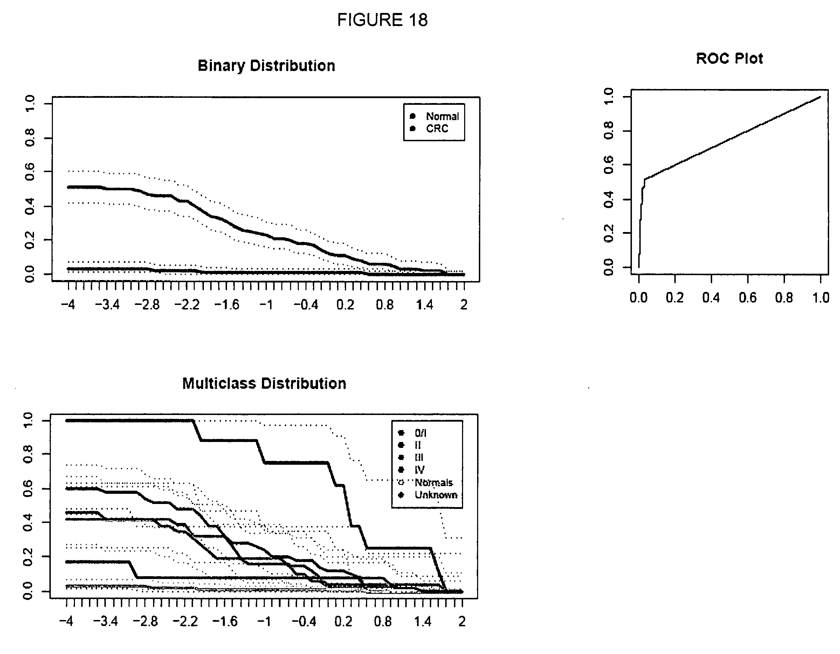

Figure 18 provides an overview of the performance of the Septin 9 HM assay according to Example 2, in all colorectal carcinoma and normal colorectal tissue samples.

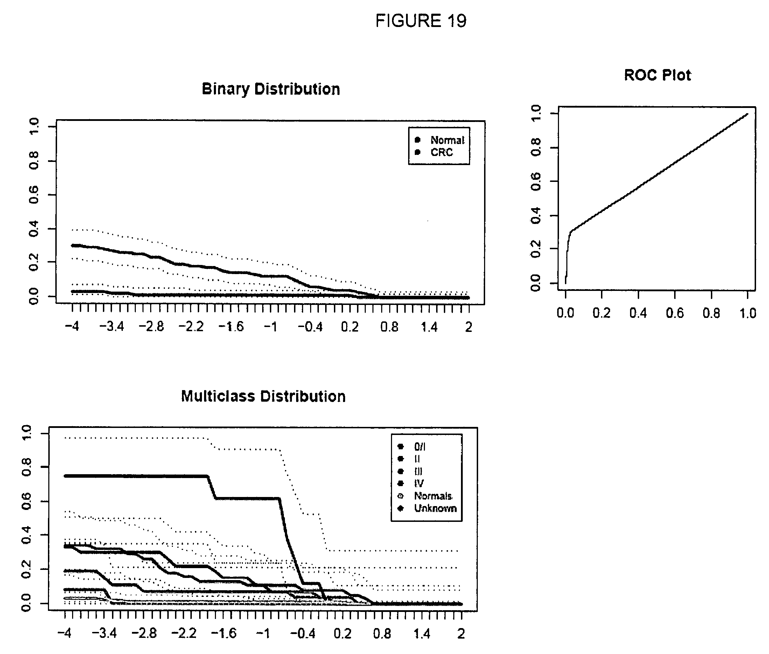

Figure 19 provides an overview of the performance of the SND1 HM assay according to Example 2, in all colorectal carcinoma and normal colorectal tissue samples.

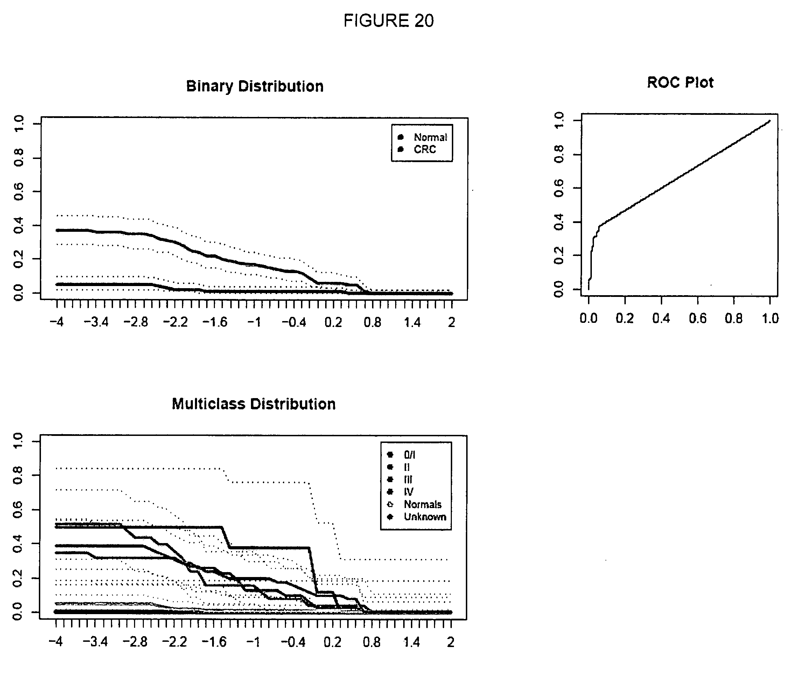

Figure 20 provides an overview of the performance of the PCDHGC3 HM assay according to Example 2, in all colorectal carcinoma and normal colorectal tissue samples.

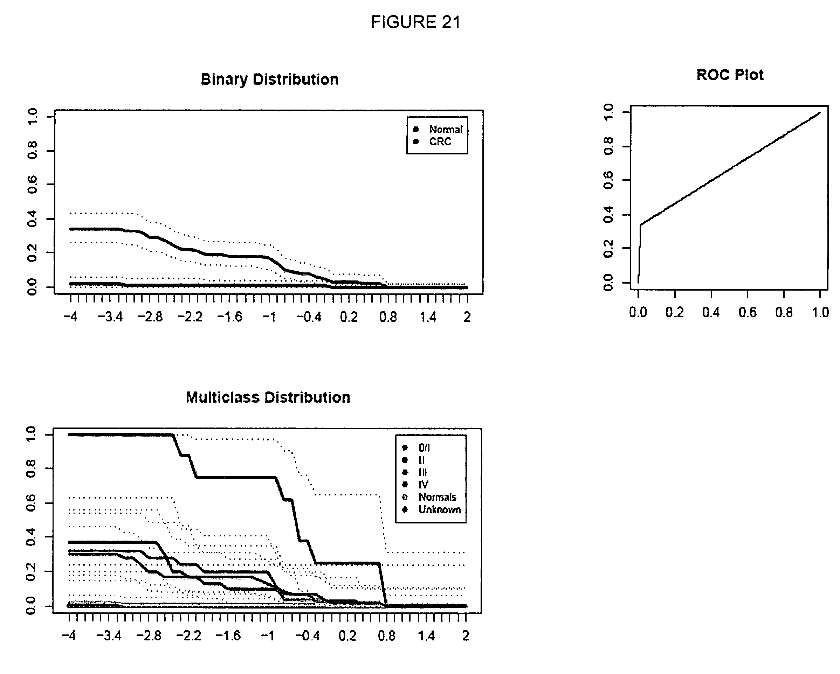

Figure 21 provides an overview of the performance of the TFAP2E HM assay according to Example 2, in all colorectal carcinoma and normal colorectal tissue samples.

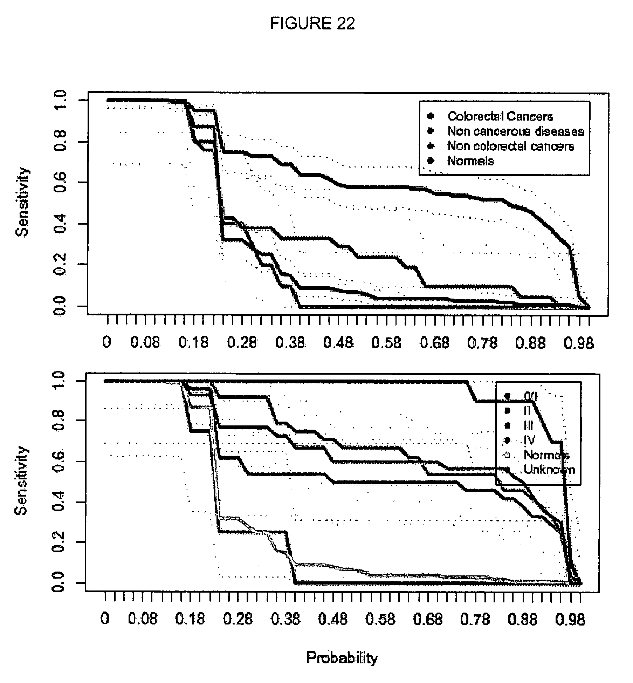

Figures 22 to 26 provide an overview of the log mean methylation measured by means of combinations HM assays (gene panels) according to Example 2. Each figures consists of two plots, The upper plot shows all samples (Normals, Non Colorectal Disease, Non-Coloretal Cancers and all CRC stages), the lower plot shows only Normaland CRC samples. Sensitivity is shown on the Y-axis, DNA methylation measured in (log 10 ng/mL) is shown on the X-axis.
Figure 22 provides an overview of the performance of the Septin 9+TFAP2E+RASSF2+PCDHGC3+SND1 assays.

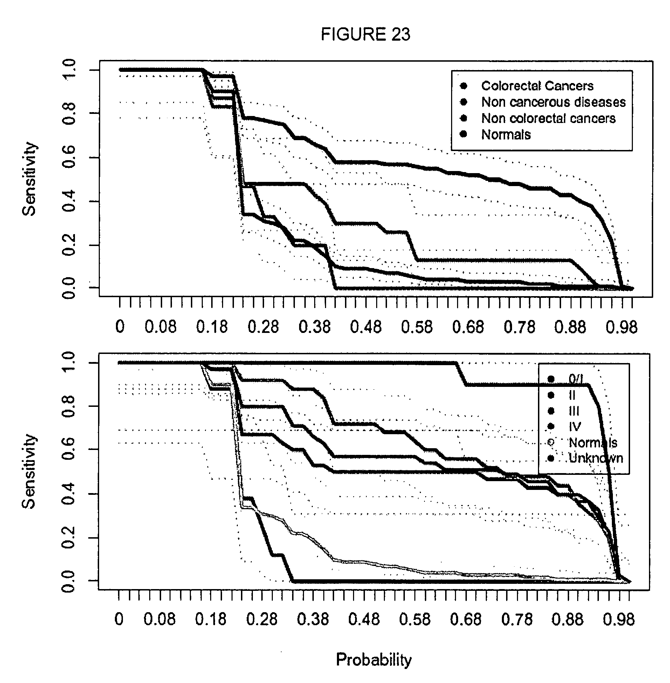

Figure 23 provides an overview of the performance of the Septin 9+TFAP2E+RASSF2+PCDHGC3 assays.

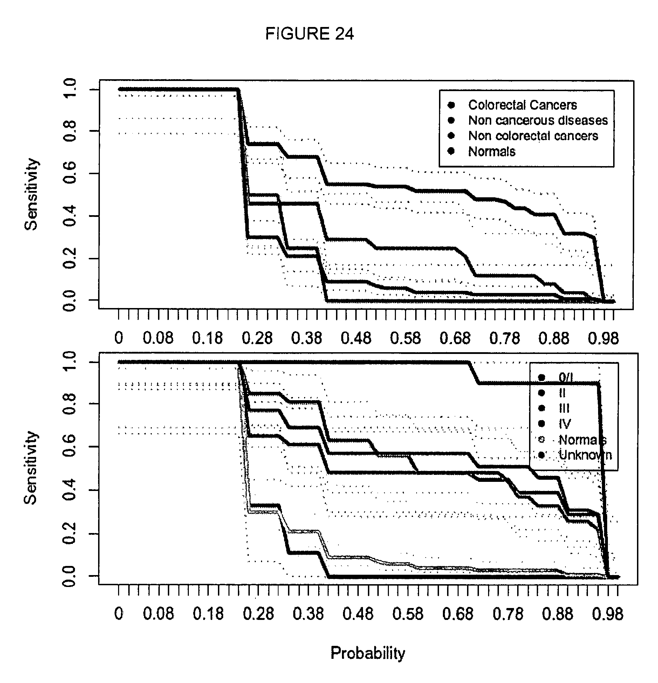

Figure 24 provides an overview of the performance of the Septin 9+TFAP2E+RASSF2 assays.

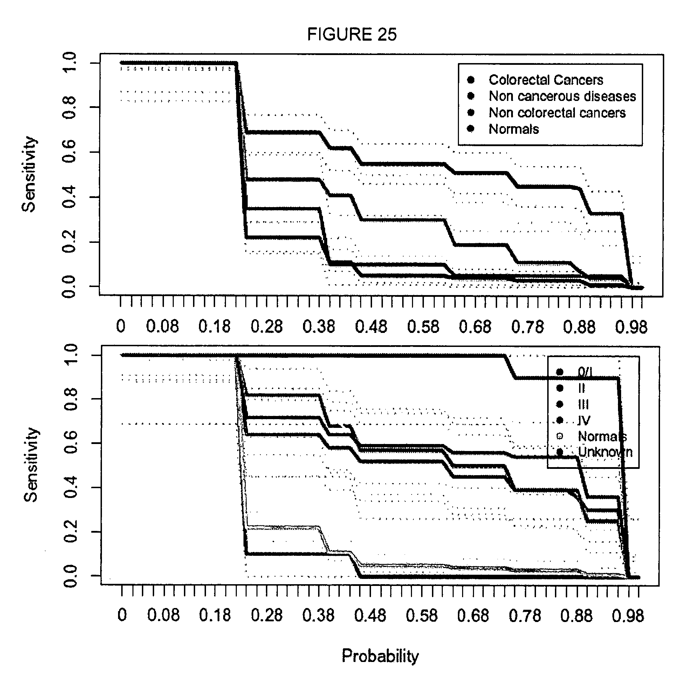

Figure 25 provides an overview of the performance of the Septin 9+TFAP2E assays.

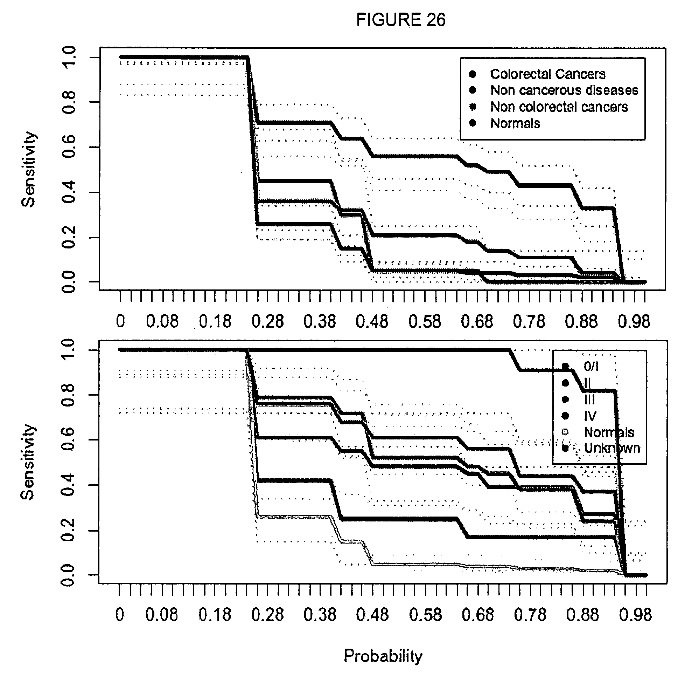

Figure 26 provides an overview of the performance of the Septin 9+RASSF2 assays.

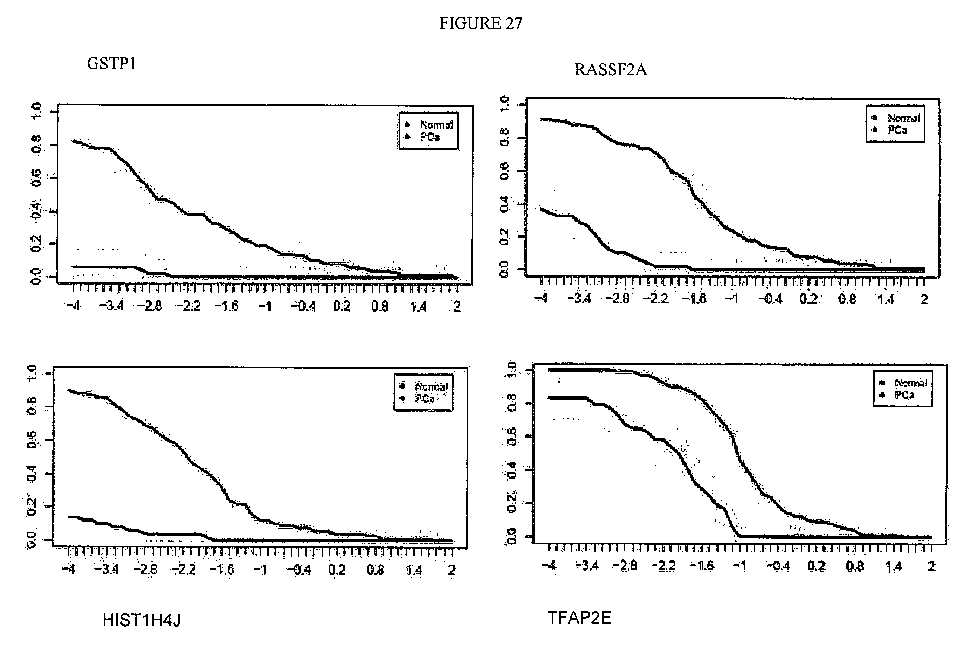

Figures 27 to 31 each provide an overview of the performance of assays according to Example 3 in various patient populations. Each figures consists of four plots 8one for each assay), wherein the Y axis provides sensitivity and the X axis DNA concentration in loglO ng/ml.

### SUMMARY OF THE INVENTION

The present invention provides a method for detecting cell proliferative disorders in a subject comprising detecting the presence of methylation of TFAP2E in a biological sample isolated from said subject wherein CpG methylation is indicative of the presence of said disorder. Various aspects of the present disclosure provide an efficient and unique genetic marker, whereby expression analysis of said marker enables the detection of cellular proliferative disorders with a particularly high sensitivity, specificity and/or predictive value. The marker of the present invention is particularly suited for detection of colorectal and prostate carcinomas. In the context of colorectal carcinoma the inventive testing methods have particular utility for the screening of at-risk populations. The inventive methods have advantages over prior art methods (including the industry standard FOBT), because of improved sensitivity, specificity and likely patient compliance.

The methods and nucleic acids of the present invention are most preferably utilised for detecting prostate cancer or distinguishing it from other prostate cell proliferative disorders or for detecting colorectal carcinoma or pre-cancerous colorectal cell proliferative disorders.

In one embodiment the application discloses a method for detecting cell proliferative disorders in a subject comprising determining the expression levels of TFAP2E in a biological sample isolated from said subject wherein underexpression and/or CpG methylation is indicative of the presence of said disorder. In one embodiment said expression level is determined by detecting the presence, absence or level of mRNA transcribed from said gene. In a further embodiment said expression level is determined by detecting the presence, absence or level of a polypeptide encoded by said gene or sequence thereof.

In a further preferred embodiment said expression is determined by detecting the presence or absence of CpG methylation within said gene, wherein the presence of methylation indicates the presence of a cell proliferative disorder. Said method comprises the following steps: i) contacting genomic DNA isolated from a biological sample (preferably selected from the group consisting of blood plasma, blood serum, whole blood, isolated blood cells, cells isolated from the blood) obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence of the gene TFAP2E; and ii) detecting cell proliferative disorders , at least in part. Preferably the target region comprises, or hybridizes under stringent conditions to a sequence of at least 16 contiguous nucleotides of at least one sequence selected from the group consisting of SEQ ID NO: 1 to SEQ ID NO: 2.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

The method is novel as no methods currently exist that enable the detection of cancer by analysis of body fluids, with a sensitivity and specificity high enough for use in a commercially available and regulatory body approved assay. For example, current methods used to detect and diagnose colorectal carcinoma include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that at least for colorectal carcinoma screening patient compliance for a single body fluid based test will be higher than the triplicate analysis of stool currently recommended for FOBT.

As a further illustration current methods used to detect and diagnose prostate cancers include PET and MRI imaging and cytology screening of aspirate or biopsy. Radiological screening methods do not usually detect cancers at early stages and are expensive and time consuming to carry out. Cytological screening presents risks associated with biopsy (internal bleeding) and aspiration (needle-track seeding and hemorrhage, bile peritonitis, and pneumothorax) Accordingly, detection of prostate cancer at an early stage is currently not possible, furthermore as patient prognosis is greatly improved by early detection there exists a need in the art for such a screening test.

A particular embodiment the method comprises the use of the gene TFAP2E as a marker for the detection and distinguishing of cellular proliferative disorders. The present invention is particularly suited for the detection of neoplastic cellular proliferative disorders (including at the pre-cancerous stage). Furthermore the methods and nucleic acids of the present disclosure enable the differentiation of malignant from benign cellular proliferative disorders. The methods and nucleic acids of the present invention are particularly effective in the detection of colorectal or prostate cancer and neoplastic disorders. Furthermore they have utility in differentiating between malignant and benign cellular proliferative colorectal and prostate disorders.

Said use of the gene may be enabled by means of any analysis of the expression of the gene, by means of mRNA expression analysis or protein expression analysis. However, in the most preferred embodiment of the invention, the detection, differentiation and distinguishing of colorectal or prostate cell proliferative disorders is enabled by means of analysis of the methylation status of the gene TFAP2E , and its promoter or regulatory elements.

The invention provides a method for the analysis of biological samples for features associated with the development of cancerous cellular proliferative disorders, the method characterized in that at least one nucleic acid, or a fragment thereof, from the group consisting of SEQ ID NO: 1 to SEQ ID NO: 2 is contacted with a reagent or series of reagents capable of distinguishing between methylated and non methylated CpG dinucleotides within the genomic sequence, or sequences of interest.

The present invention provides a method for ascertaining epigenetic parameters of genomic DNA associated with the development of neoplastic cellular proliferative disorders (e.g. cancers). The method has utility for the improved diagnosis, treatment and monitoring of said diseases.

Preferably, the source of the test sample is selected from the group consisting of cells or cell lines, histological slides, biopsies, paraffin-embedded tissue, body fluids, ejaculate, stool, urine, blood, and combinations thereof. More preferably, the source is selected from the group consisting of stool, blood plasma, blood serum, whole blood, isolated blood cells, cells isolated from the blood obtained from the subject.

Specifically, the present invention provides a method for detecting neoplastic and cancerous cellular proliferative disorders (preferably colorectal and/or prostate cell) including at the early precancerous stage, and for differentiating between malignant and benign cellular proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridises under stringent conditions to, a sequence comprising at least 16 contiguous nucleotides of SEQ ID NO: 1 or more preferably SEQ ID NO: 2, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state- dependent conversion or non-conversion of at least one such CpG dinucleotide sequence to the corresponding converted or non-converted dinucleotide sequence within a sequence selected from the group consisting of SEQ ID NO: 3 to SEQ ID NO: 10, and contiguous regions thereof corresponding to the target sequence.

Additional embodiments provide a method for the detection of neoplastic cellular proliferative disorders (or distinguishing them from benign cellular proliferative disorders), most preferably colorectal or prostate, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 3 to SEQ ID NO: 10, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequence selected from the group consisting of SEQ ID NO: 1 to SEQ ID NO: 2.

Preferably, determining comprises use of at least one method selected from the group consisting of: I) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 3 to SEQ ID NO: 10, and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 3 to SEQ ID NO: 10, and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 3 to SEQ ID NO: 10, and complements thereof, and extending at least one such hybridized nucleic acid molecule by at least one nucleotide base; and iv) sequencing of the amplificate.

Further embodiments provide a method for the analysis (i.e. detection and/or classification) of cell proliferative disorders, comprising: obtaining a biological sample having subject genomic DNA; extracting the genomic DNA; contacting the genomic DNA, or a fragment thereof, comprising one or more sequences selected from the group consisting of SEQ ID NO: 1 to SEQ ID NO: 2 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of SEQ ID NO: 1, or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO: 1 to SEQ ID NO: 2.

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis,Academic Press, New York, 1975).

The term methylation status or methylation state should also be taken to mean a value reflecting the degree of methylation at a CpG position.

Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) the TFAP2E gene.

Unless specifically stated the terms "hypomethylated" or "downmethylated" shall be taken to mean a methylation level below that of a specified cut-off point, wherein said cut-off may be a value representing the average or median methylation level for a given population, or is preferably an optimized cut-off level. The "cut-off" is also referred herein as a "threshold". In the context of the present invention the terms "unmethylated", "hypomethylated" or "downmethylated" shall be taken to include a methylation level at the cut-off be zero (0) % (or equivalents thereof) methylation for all CpG positions within and associated with (e.g. in promoter or regulatory regions) the TFAP2E gene.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular cytosine methylation. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific blocking probes (also referred to herein as blockers) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers.

The term "Ms-SNuPE" (Methylation-sensitive Single Nucleotide Primer Extension) refers to the art-recognized assay described by Gonzalgo and Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art- recognized methylation assay described by Xiong & Laird, Nucleic Acids Res. 25:2532-2534,1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5.

"Non-methylation-specific restriction enzymes" or "non-methylation-sensitive restriction enzymes" are restriction enzymes that cut a nucleic acid sequence irrespective of the methylation state with nearly identical efficiency. They are also called "methylation- unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "TFAP2E" shall be taken to include all transcript variants thereof and all promoter and regulatory elements thereof. Furthermore as a plurality of SNPs are known within said gene the term shall be taken to include all sequence variants thereof.

The term "pre-cancerous" and equivalents thereof shall be taken to mean any cellular proliferative disorder which is undergoing malignant transformation. Examples of such conditions include, in the context of colorectal cellular proliferative disorders, cellular proliferative disorders with a high degree of dysplasia and the following classes of adenomas:
Level 1: penetration of malignant glands through the muscularis mucosa into the submucosa, within the polyp head
Level 2: the same submucosal invasion, but present at the junction of the head to the stalk
Level 3: invasion of the stalk
Level 4: invasion of the stalk's base at the connection to the colonic wall (this level corresponds to stage Dukes A)

The present invention provides a method for detecting cell proliferative disorders in a subject comprising detecting the presence of methylation of TFAP2E in a biological sample isolated from said subject wherein CpG methylation is indicative of the presence of said disorder. Said markers may be used for the diagnosis of malignant neoplastic cellular proliferative disorders (preferably cancer), including early detection during the pre-cancerous stages of the disease, and furthermore for the differentiation of malignant from benign cellular proliferative disorders. The present invention discloses a method wherein a malignant cell proliferative disorder is distinguished from a benign cell proliferative disorder said method characterized in that the presence of CpG methylation is indicative of the presence of a malignant cell proliferative disorder or pre-cancerous disorder and the absence thereof is indicative of the presence of a benign cell proliferative disorder.

The markers of the present invention are particularly efficient in detecting or distinguishing between prostate cell proliferative disorders or alternatively for detecting or distinguishing between colorectal cell proliferative disorders, thereby providing improved means for the early detection, classification and treatment of said disorders.

Bisulfite modification of DNA is an art-recognized tool used to assess CpG methylation status. 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5- methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is converted in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 97/46705 and WO 95/15373).

The present invention provides for the use of the bisulfite technique, in combination with one or more methylation assays, for determination of the methylation status of CpG dinucleotide sequences within SEQ ID NO: 1. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood or stool. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position.

According to the present invention, determination of the methylation status of CpG dinucleotide sequences within SEQ ID NO: 1 has utility both in the diagnosis and characterization of cellular proliferative disorders. In preferred embodiments the methylation status of CpG positions within SEQ ID NO: 2 are determined, SEQ ID NO: 2 is a particularly preferred regions of SEQ ID NO: 1 (i.e. SEQ ID NO: 1 comprises SEQ ID NO: 2). Determination of the methylation status of CpG dinucleotide sequences within SEQ ID NO: 2 also have utility in the diagnosis and characterization of cellular proliferative disorders.

Methylation Assay Procedures. Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong and Laird, Nucleic Acids Res. 25:2532-2534, 1997).

COBRA. COBRA™ analysis is a quantitative methylation assay useful for determining DNA methylation levels at specific gene loci in small amounts of genomic DNA (Xiong and Laird, Nucleic Acids Res. 25:2532-2534, 1997). Briefly restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (e.g., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight^{™}" (a fluorescence-based real-time PCR technique) (Eads et al., Cancer Res. 59:2302-2306, 1999), Ms-SNuPE™ (Methylation- sensitive Single Nucleotide Primer Extension) reactions (Gonzalgo and Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific blocking probes (also referred to herein as blockers) covering CpG positions between, or covered by the amplification primers enable methylation- specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers. The HeavyMethyl™ assay may also be used in combination with methylation specific amplification primers.

Typical reagents (e.g., as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

MSP. MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin- embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

MethyLight™. The MethyLight^{™} assay is a high-throughput quantitative methylation assay that utilizes fluorescence-based real-time PCR (TaqMan□) technology that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight^{™} assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight^{™} process can by used with any suitable probes e.g. "TaqMan®" , Lightcycler® etc.... For example, double-stranded genomic DNA is treated with sodium bisulfite and subjected to one of two sets of PCR reactions using TaqMan® probes; e.g., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace theTaqMan® probe by digesting it to release the fluorescent reporter molecule for quantitative detection of its now unquenched signal using a real-time fluorescent detection system.

Typical reagents (e.g., as might be found in a typical MethyLight□-based kit) for MethyLight^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM^{™} process can be used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical QM^{™} -based kit) for QM^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

Ms-SNuPE. The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (e.g., as might be found in a typical Ms-SNuPE^{™}-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

The genomic sequence according to SEQ ID NO: 1 to SEQ ID NO: 2, and non-naturally occurring treated variants thereof according to SEQ ID NO: 3 to SEQ ID NO: 10, were determined to have novel utility for the early detection, classification and/or treatment of cellular proliferative disorders, in particular colorectal and/or prostate cell proliferative disorders.

In one embodiment the invention of the method comprises the following steps: i) contacting genomic DNA (preferably isolated from body fluids) obtained from the subject with at least one reagent, or series of reagents that distinguishes between methylated and non-methylated CpG dinucleotides within the gene TFAP2E (including its promoter and regulatory regions); and ii) detecting, or detecting and distinguishing between or among colon or prostate cell proliferative disorders.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic matter or pre-cancerous matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred is urine, either normally voided or post prostatic massage.

The genomic DNA sample is then treated with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of at least one sequence selected from the group consisting of SEQ ID NO: 1 to SEQ ID NO: 2 respectively, wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridization behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior It is preferred that this treatment is carried out with bisulfite (hydrogen sulfite, disulfite) and subsequent alkaline hydrolysis. Such a treatment results in the conversion of SEQ ID NO: 1 TO SEQ ID NO: 2 to SEQ ID NO: 3 TO SEQ ID NO: 6(respectively) wherein said CpG dinucleotides are methylated or SEQ ID NO: 7 TO SEQ ID NO: 10 wherein said CpG dinucleotides are unmethylated (see Table 1).

The treated DNA is then analyzed in order to determine the methylation state of the target gene sequences (TFAP2E prior to the treatment). It is particularly preferred that the target region comprises, or hybridizes under stringent conditions to at least 16 contiguous nucleotides of TFAP2E . It is preferred that the sequence of said gene according to SEQ ID NO: 1 is analyzed, it is particularly preferred that the sub-region thereof according to SEQ ID NO: 2 are analyzed. The method of analysis may be selected from those known in the art, including those listed herein. Particularly preferred are MethyLight^{™}, MSP and the use of blocking oligonucleotides (HeavyMethyl™) as described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: 3 to SEQ ID NO: 10 and sequences complementary thereto.

Aberrant methylation, more specifically hypermethylation of TFAP2E (as well as promoter and/or regulatory regions) is associated with the presence of neoplastic cellular proliferative disorders, and is particularly prevalent in colorectal and prostate carcinomas. Accordingly wherein a biological sample presents within any degree of methylation, said sample should be determined as neoplastic.

Analysis of one the TFAP2E gene enables for the first time detecting, or detecting and distinguishing between or among colon or prostate cell proliferative disorders afforded with a sensitivity of greater than or equal to 80% and a specificity of greater than or equal to 80%. Sensitivity is calculated as: {detected neoplasia/all neoplasia) e.g. : {detected colon neoplasia/all colon neoplasia); and specificity is calculated as (non-detected negatives/total negatives).

Colon neoplasia is herein defined as all colon malignancies and adenomas greater than 1 cm., or subsets thereof. Negatives can be defined as healthy individuals.

In one embodiment the method discloses the use of TFAP2E or promoter and/or regulatory regions thereof) as a marker for the differentiation, detection and distinguishing of cellular proliferative disorders (in particular neoplastic, colon or prostate disorders).

Said method may be enabled by means of any analysis of the expression of an RNA transcribed therefrom or polypeptide or protein translated from said RNA, preferably by means of mRNA expression analysis or polypeptide expression analysis. Accordingly the present application also discloses diagnostic assays and methods, both quantitative and qualitative for detecting the expression of the gene TFAP2E in a subject and determining therefrom upon the presence or absence of cancer in said subject.

Aberrant expression of mRNA transcribed from the gene TFAP2E is associated with the presence of cancer in a subject. Under expression (and/or presence of methylation) is associated with the presence of cancer, and vice versa over-expression (and/or absence of methylation) is associated with the absence of cancer.

To detect the presence of mRNA encoding a gene or genomic sequence, a sample is obtained from a patient. The sample may be any suitable sample comprising cellular matter of the tumor. Suitable sample types include cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sample types are stool or body fluids selected from the group consisting colonic effluent, urine, blood plasma, blood serum, whole blood, isolated blood cells, cells isolated from the blood.

The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987). Further preferred is the "Real-time" variant of RT- PCR, wherein the PCR product is detected by means of hybridisation probes (e.g. TaqMan, Lightcycler, Molecular Beacons and Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel et al. 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.

DNA arrays can be generated by immobilizing pre-synthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes of interest (in this case TFAP2E ) and tend to be shorter sequences in the range of 25-70 nucleotides. In a preferred embodiment said oligonucleotides or polynucleotides comprise at least 9, 18 or 25 bases of a sequence complementary to or hybridising to said RNA transcript. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy^{®}3- or Cy^{®}5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing).

Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post- hybridisation using a fluorescent microarray scanner. The fluorescent intensity of each spot indicates the level of expression of the analysed gene; bright spots correspond to strongly expressed genes, while dim spots indicate weak expression..

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

Another aspect of the disclosure relates to a kit for use in diagnosis of cancer in a subject according to the methods of the present invention, said kit comprising: a means for measuring the level of transcription of the gene TFAP2E. In a preferred embodiment the means for measuring the level of transcription comprise oligonucleotides or polynucleotides able to hybridise under stringent or moderately stringent conditions to the transcription products of TFAP2E.

In a most preferred embodiment the level of transcription is determined by techniques selected from the group of Northern Blot analysis, reverse transcriptase PCR, real-time PCR, RNAse protection, and microarray. In another embodiment of the invention the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.

In a preferred embodiment the kit comprises (a) a plurality of oligonucleotides or polynucleotides able to hybridise under stringent or moderately stringent conditions to the transcription products of the gene TFAP2E; (b) a container, preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results.

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase.It is further preferred that said kit further contains an Rnase reagent.

The present application further discloses methods for the detection of the presence of the polypeptide encoded by said gene sequences in a sample obtained from a patient.

Aberrant levels of polypeptide expression of the polypeptides encoded by the gene TFAP2E are associated with the presence of cancer.

Under expression of said polypeptides is associated with the presence of cancer.

Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton and Lange, Norwalk, Conn. pp 217-262, 1991). Preferred are binder-ligand immunoassay methods including reacting antibodies with an epitope or epitopes and competitively displacing a labelled polypeptide or derivative thereof.

Certain embodiments of the present disclosure comprise the use of antibodies specific to the polypeptide encoded by the TFAP2E gene.

Such antibodies are useful for cancer diagnosis. In certain embodiments production of monoclonal or polyclonal antibodies can be induced by the use of an epitope encoded by a polypeptide of the TFAP2E gene as an antigen. Such antibodies may in turn be used to detect expressed polypeptides as markers for cancer diagnosis. The levels of such polypeptides present may be quantified by conventional methods.

Antibody-polypeptide binding may be detected and quantified by a variety of means known in the art, such as labelling with fluorescent or radioactive ligands. The application further discloses kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spatial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase) . The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981). Methods for preparation of the polypeptides or epitopes thereof include, but are not limited to chemical synthesis, recombinant DNA techniques or isolation from biological samples.

In the final step of the method the diagnosis of the patient is determined, whereby under-expression (of TFAP2E mRNA or polypeptides) is indicative of the presence of cancer or a cellular proliferative disorder undergoing malignant transformation. The term under-expression shall be taken to mean expression at a detected level less than a pre-determined cut off which may be selected from the group consisting of the mean, median or an optimised threshold value.

Another aspect of the application discloses a kit for use in diagnosis of cancer or a cellular proliferative disorder undergoing malignant transformation in a subject according to the methods of the present invention, comprising: a means for detecting TFAP2E polypeptides. The means for detecting the polypeptides comprise preferably antibodies, antibody derivatives, or antibody fragments. The polypeptides are most preferably detected by means of Western Blotting utilizing a labelled antibody. In another example the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting TFAP2E polypeptides; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Particular embodiments of the present invention provide a novel application of the analysis of methylation levels and/or patterns within said sequences that enables a precise detection, characterisation and/or treatment of prostate and/or colorectal cell proliferative disorders. Early detection of cancer is directly linked with disease prognosis, and the disclosed method thereby enables the physician and patient to make better and more informed treatment decisions.

### FURTHER IMPROVEMENTS

The present invention provides novel uses for the genomic sequence SEQ ID NO: 1, AND MORE PREFERABLY SEQ ID NO: 2. Additional embodiments provide modified variants of SEQ ID NO: 1 TO SEQ ID NO: 2, as well as oligonucleotides and/or PNA-oligomers for analysis of cytosine methylation patterns within SEQ ID NO: 1 TO SEQ ID NO: 2.

An objective of the disclosure comprises analysis of the methylation state of one or more CpG dinucleotides within SEQ ID NO: 1 and sequences complementary thereto, and more preferably SEQ ID NO: 2 and sequences complementary thereto.

The disclosure provides treated nucleic acids, derived from genomic SEQ ID NO: 1 to SEQ ID NO: 2, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the invention, the invention provides a non-naturally occurring modified nucleic acid comprising a sequence of at least 16 contiguous nucleotide bases in length of a sequence selected from the group consisting of SEQ ID NO: 3 TO SEQ ID NO: 10. In further preferred embodiments of the disclosure said nucleic acid is at least 50, 100, 150, 200, 250 or 500 base pairs in length of a segment of the nucleic acid sequence disclosed in SEQ ID NO: 3 to SEQ ID NO: 10. Particularly preferred is a nucleic acid molecule that is not identical or complementary to all or a portion of the sequences SEQ ID NO: 3 to SEQ ID NO: 10 but not SEQ ID NO: 1 to SEQ ID NO: 2 or other naturally occurring DNA.

It is preferred that said sequence comprises at least one CpG, TpA or CpA dinucleotide and sequences complementary thereto. The sequences of SEQ ID NO: 3 TO SEQ ID NO: 10 provide non-naturally occurring modified versions of the nucleic acid according to SEQ ID NO: 1 TO SEQ ID NO: 2, wherein the modification of each genomic sequence results in the synthesis of a nucleic acid having a sequence that is unique and distinct from said genomic sequence as follows. For each sense strand genomic DNA, e.g., SEQ ID NO: 1, four converted versions are disclosed. A first version wherein "C" is converted to "T," but "CpG" remains "CpG" (i.e., corresponds to case where, for the genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (i.e., corresponds to case where, for all "C" residues of CpG dinucleotide sequences are methylated and are thus not converted). The 'upmethylated' converted sequences of SEQ ID NO: 1 to SEQ ID NO: 2 correspond to SEQ ID NO: 3 TO SEQ ID NO: 6. A third chemically converted version of each genomic sequences is provided, wherein "C" is converted to "T" for all "C" residues, including those of "CpG" dinucleotide sequences (i.e., corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (i.e., corresponds to case where, for the complement (antisense strand) of each genomic sequence, all "C" residues of CpG dinucleotide sequences are unmethylated). The 'downmethylated' converted sequences of SEQ ID NO: 1 to SEQ ID NO: 2 correspond to SEQ ID NO: 7 TO SEQ ID NO: 10.

Significantly, heretofore, the nucleic acid sequences and molecules according SEQ ID NO: 3 to SEQ ID NO: 1 were not implicated in or connected with the detection, classification or treatment of cellular proliferative disorders.

In an alternative preferred embodiment, the disclosure further provides oligonucleotides or oligomers suitable for use in the methods of the invention for detecting the cytosine methylation state within genomic or treated (chemically modified) DNA, according to SEQ ID NO: 1 TO SEQ ID NO: 10. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 3 to SEQ ID NO: 10 and/or sequences complementary thereto, or to a genomic sequence according to SEQ ID NO: 1 to SEQ ID NO: 2 and/or sequences complementary thereto.

Thus, the present disclosure includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 10 or to the complements thereof. Particularly preferred is a nucleic acid molecule that hybridizes under moderately stringent and/or stringent hybridization conditions to all or a portion of the sequences SEQ ID NO: 3 to SEQ ID NO: 10 but not SEQ ID NO: 1 to SEQ ID NO: 2 or other human genomic DNA.

The identical or hybridizing portion of the hybridizing nucleic acids is typically at least 9, 16, 20, 25, 30 or 35 nucleotides in length. However, longer molecules have utility.

Preferably, the hybridizing portion of the inventive hybridizing nucleic acids is at least 95%, or at least 98%, or 100% identical to the sequence, or to a portion thereof of SEQ ID NO: 1 to SEQ ID NO: 10, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1 to SEQ ID NO: 2 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, e.g., SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1, 2, 3,...(Y-(X-1));
where Y equals the length (nucleotides or base pairs) of SEQ ID NO: 1 (24959);
where X equals the common length (in nucleotides) of each oligonucleotide in the set (e.g., X=20 for a set of consecutively overlapping 20-mers); and
where the number (Z) of consecutively overlapping oligomers of length X for a given SEQ ID NO of length Y is equal to Y- (X-1). For example Z= 24959-19= 24940 for either sense or antisense sets of SEQ ID NO: 1, where X=20.

Preferably, the set is limited to those oligomers that comprise at least one CpG, TpG or CpA dinucleotide.

Examples of 20-mer oligonucleotides include the following set of 24940 oligomers (and the antisense set complementary thereto), indicated by polynucleotide positions with reference to SEQ ID NO: 1: 1-20, 2-21, 3-22, 4-23, 5-24, and 24940-24959.

Preferably, the set is limited to those oligomers that comprise at least one CpG, TpG or CpA dinucleotide.

Likewise, examples of 25-mer oligonucleotides include the following set of 24935 oligomers (and the antisense set complementary thereto), indicated by polynucleotide positions with reference to SEQ ID NO: 1:
1-25, 2-26, 3-27, 4-28, 5-29, ...... and 24935-24959.

Preferably, the set is limited to those oligomers that comprise at least one CpG, TpG or CpA dinucleotide.

The present application discloses, for each of SEQ ID NO: 1 to SEQ ID NO: 10 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present application constitute effective tools useful to ascertain genetic and epigenetic parameters of the genomic sequence corresponding to SEQ ID NO: 1. Preferred sets of such oligonucleotides or modified oligonucleotides of length X are those consecutively overlapping sets of oligomers corresponding to SEQ ID NO: 1 to SEQ ID NO: 10 (and to the complements thereof). Preferably, said oligomers comprise at least one CpG, TpG or CpA dinucleotide.

Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present disclosure are typically used in 'sets,' which contain at least one oligomer for analysis of each of the CpG dinucleotides of a genomic sequence selected from the group consisting SEQ ID NO: 1 to SEQ ID NO: 2 and sequences complementary thereto, or to the corresponding CpG, TpG or CpA dinucleotide within a sequence of the treated nucleic acids according to SEQ ID NO: 3 to SEQ ID NO: 10 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present disclosure provides a set of at least two (2) (oligonucleotides and/or PNA-oligomers) useful for detecting the cytosine methylation state in treated genomic DNA (SEQ ID NO: 3 to SEQ ID NO: 10), or in genomic DNA (SEQ ID NO: 1 to SEQ ID NO: 2 and sequences complementary thereto). These probes enable diagnosis, classification and/or therapy of genetic and epigenetic parameters of prostate and/or colorectal cell proliferative disorders. The set of oligomers may also be used for detecting single nucleotide polymorphisms (SNPs) in treated genomic DNA (SEQ ID NO: 3 to SEQ ID NO: 10), or in genomic DNA (SEQ ID NO: 1 to SEQ ID NO: 2 and sequences complementary thereto).

In preferred embodiments, at least one, and more preferably all members of a set of oligonucleotides is bound to a solid phase.

In further embodiments, the present application discloses a set of at least two (2) oligonucleotides that are used as 'primer' oligonucleotides for amplifying DNA sequences of one of SEQ ID NO: 1 to SEQ ID NO: 10 and sequences complementary thereto, or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA- oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital read-out of each molecular species in the mixture).

Particularly preferred are those sets of oligomers according to the Examples. In the most preferred embodiment of the method, the presence or absence of a cellular proliferative disorder, most preferably cancer or differentiation thereof from benign cellular proliferative disorders is determined. This is achieved by analysis of the methylation status of at least one target sequence comprising at least one CpG position said sequence comprising, or hybridizing under stringent conditions to at least 16 contiguous nucleotides of a sequence selected from the group consisting SEQ ID NO: 1 to SEQ ID NO: 2 and complements thereof. The present invention further provides a method for ascertaining genetic and/or epigenetic parameters of the genomic sequence according to SEQ ID NO: 1 to SEQ ID NO: 2 within a subject by analyzing cytosine methylation and single nucleotide polymorphisms. Said method comprising contacting a nucleic acid comprising SEQ ID NO: 1 to SEQ ID NO: 2 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the first step, a sample of the tissue to be analysed is obtained. The source may be any suitable source, such as cell lines, histological slides, biopsies, paraffin-embedded tissue, body fluids, stool, colonic effluent, urine, blood plasma, blood serum, whole blood, isolated blood cells, cells isolated from the blood and all possible combinations thereof.

It is preferred that said sources of DNA are stool or urine.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrene particles, polystyrene surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the second step of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pretreatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8,-tetramethylchromane 2-carboxylic acid or trihydroxybenzoe acid and derivates thereof, e.g. Gallic acid (see: PCT/EP2004/01 1715). The bisulfite conversion is preferably carried out at a reaction temperature between 30°C and 70°C, whereby the temperature is increased to over 85°C for short periods of times during the reaction (see: PCT/EP2004/011715). The bisulfite treated DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715).

In the third step of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO: 3 to SEQ ID NO: 10 and sequences complementary thereto.

In an alternate embodiment of the method, the methylation status of pre-selected CpG positions within the nucleic acid sequences according to SEQ ID NO: 1, and more preferably SEQ ID NO: 2 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non- methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 3 to SEQ ID NO: 10 and sequences complementary thereto, wherein the base sequence of said oligomers comprises at least one CpG dinucleotide .A further preferred embodiment of the method comprises the use of blocker oligonucleotides (the HeavyMethyl™ assay). The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said blocking oligonucleotides is required to comprise a sequence having a length of at least 9 nucleotides which hybridises to a treated nucleic acid sequence according to one of SEQ ID NO: 3 to SEQ ID NO: 10 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas and Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut and Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut and Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the fourth step of the method, the amplificates obtained during the third step of the method are analyzed in order to ascertain the methylation status of the CpG dinucleotides prior to the treatment. In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of based-based methods such as, but not limited to, array technology and probe based technologies as well as by means of techniques such as sequencing and template directed extension.

In one embodiment of the method, the amplificates synthesized in step three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have utility.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 1 to SEQ ID NO: 2, and the equivalent positions within SEQ ID NO: 3 to SEQ ID NO: 10. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; also see United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the fourth step of the method comprises the use of template-directed oligonucleotide extension, such as MS-SNuPE as described by Gonzalgo and Jones, Nucleic Acids Res. 25:2529-2531, 1997.

In yet a further embodiment of the method, the fourth step of the method comprises sequencing and subsequent sequence analysis of the amplificate generated in the third step of the method (Sanger F., et al., Proc Natl Acad Sci USA 74:5463-5467, 1977).

In the most preferred embodiment of the method the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or blocking oligonucleotides, and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 3 to SEQ ID NO: 10and sequences complementary thereto, wherein the base sequence of said oligomers comprise at least one CpG dinucleotide.

Step e) of the method, namely the detection of the specific amplificates indicative of the methylation status of one or more CpG positions according to SEQ ID NO: 1 is carried out by means of real-time detection methods as described above.

Additional embodiments of the invention provide a method for the analysis of the methylation status of genomic DNA according to the invention (SEQ ID NO: 1 to SEQ ID NO: 2, and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the first step of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for use in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood. Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting ofMseI, BfaI, Csp6I, Tru1I, Tvu1I, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the third step, the DNA (or fragments thereof) is then digested with one or more methylation sensitive restriction enzymes. The digestion is carried out such that hydrolysis of the DNA at the restriction site is informative of the methylation status of a specific CpG dinucleotide of the TFAP2E gene.

Preferably, the methylation-specific restriction enzyme is selected from the group consisting of Bsi E1, Hga I HinPl, Hpy99I, Ava I, Bce AI, Bsa HI, BisI, BstUI, BshI236I, AccII, BstFNI, McrBC, GlaI, MvnI, HpaII (HapII), HhaI, AciI, SmaI, HinP1I, HpyCH4IV, EagI and mixtures of two or more of the above enzymes. Preferred is a mixture containing the restriction enzymes BstUI, HpaII, HpyCH4IV and HinP1I.

In the fourth step, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting SEQ ID NO: 1 to SEQ ID NO: 2, and complements thereof. Preferably said contiguous sequence is at least 16, 20 or 25 nucleotides in length. In an alternative embodiment said primers may be complementary to any adaptors linked to the fragments.

In the fifth step the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 1 to SEQ ID NO: 2, and complements thereof. Preferably said contiguous sequence is at least 16, 20 or 25 nucleotides in length.

Subsequent to the determination of the methylation state or level of the genomic nucleic acids the presence, absence or class of cellular proliferative disorder is deduced based upon the methylation state or level of at least one CpG dinucleotide sequence of SEQ ID NO: 1, or an average, or a value reflecting an average methylation state of a plurality of CpG dinucleotide sequences of SEQ ID NO: 1 wherein methylation is associated with a neoplastic or pre-cancerous cellular proliferative disorder. Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

### Diagnostic and Prognostic Assays for cellular proliferative disorders

The present invention enables diagnosis of events which are disadvantageous to patients or individuals in which important genetic and/or epigenetic parameters within TFAP2E may be used as markers. Said parameters obtained by means of the present invention may be compared to another set of genetic and/or epigenetic parameters, the differences serving as the basis for a diagnosis and/or prognosis of events which are disadvantageous to patients or individuals.

More specifically the present invention enables the screening of at-risk populations for the early detection of cancers, most preferably prostate cancer and/or colorectal carcinomas. Furthermore, the present invention enables the differentiation of neoplastic (e.g. malignant) from benign (i.e. non-cancerous) cellular proliferative disorders. For example, it enables the differentiation of a colorectal carcinoma from small colon adenomas or polyps. Neoplastic cellular proliferative disorders present decreased methylation (i.e. decreased expression) within the TFAP2E gene, as opposed to said benign disorders which do not.

Specifically, the present invention provides for diagnostic and classification cancer assays based on measurement of differential expression (preferably methylation) of one or more CpG dinucleotide sequences of SEQ ID NO: 1, or more preferably sub-regions thereof according to SEQ ID NO: 2 that comprise such a CpG dinucleotide sequence. Typically, such assays involve obtaining a sample from a subject, performing an assay to measure the expression of the TFAP2E gene, preferably by determining the methylation status of at least one CpG dinucleotide sequences of SEQ ID NO: 1 (more preferably the sub-region thereof according to SEQ ID NO: 2), derived from the tissue sample, relative to a control sample, or a known standard and making a diagnosis based thereon.
In particular preferred embodiments, inventive oligomers are used to assess the CpG dinucleotide methylation status, such as those based on SEQ ID NO: 1 to SEQ ID NO: 10, or arrays thereof, as well as in kits based thereon and useful for the diagnosis and/or classification of cellular proliferative disorders.

### Kits

Moreover, an additional aspect of the present disclosure is a kit comprising: a means for determining TFAP2E methylation. The means for determining TFAP2E methylation comprise preferably a bisulfite-containing reagent; one or a plurality of oligonucleotides consisting whose sequences in each case are identical, are complementary, or hybridise under stringent or highly stringent conditions to a 9 or more preferably 18 base long segment of a sequence selected from SEQ ID NO: 3 to SEQ ID NO: 10; and optionally instructions for carrying out and evaluating the described method of methylation analysis. In one embodiment the base sequence of said oligonucleotides comprises at least one CpG, CpA or TpG dinucleotide.

In a further embodiment, said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit along the lines of the present invention can also contain only part of the aforementioned components.

In a preferred embodiment the kit may comprise additional bisulfite conversion reagents selected from the group consisting: DNA denaturation buffer; sulfonation buffer; DNA recovery reagents or kits (e.g., precipitation, ultrafiltration, affinity column); desulfonation buffer; and DNA recovery components.

In a further alternative embodiment, the kit may contain, packaged in separate containers, a polymerase and a reaction buffer optimised for primer extension mediated by the polymerase, such as PCR. In another embodiment the kit further comprising means for obtaining a biological sample of the patient. Preferred is a kit, which further comprises a container suitable for containing the means for determining methylation of the gene TFAP2E in the biological sample of the patient, and most preferably further comprises instructions for use and interpretation of the kit results. In a preferred embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 3 to SEQ ID NO: 10; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 3 to SEQ ID NO: 10 and sequences complementary thereto; and optionally (d) instructions for use and interpretation of the kit results.

In an alternative embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 3 to SEQ ID NO: 10; (d) at least one oligonucleotides and/or PNA-oligomer having a length of at least 9 or 16 nucleotides which is identical to or hybridises to a pre-treated nucleic acid sequence according to one of SEQ ID NO: 3 to SEQ ID NO: 10 and sequences complementary thereto; and optionally (e) instructions for use and interpretation of the kit results.

The kit may also contain other components such as buffers or solutions suitable for blocking, washing or coating, packaged in a separate container.

Another aspect of the disclosure relates to a kit for use in determining the presence of and/or distinguishing between cell proliferative disorders, said kit comprising: a means for measuring the level of transcription of the gene TFAP2E and a means for determining TFAP2E methylation.

Typical reagents (e.g., as might be found in a typical COBRA™-based kit) for COBRA™ analysis may include, but are not limited to: PCR primers for TFAP2E; restriction enzyme and appropriate buffer; gene-hybridization oligo; control hybridization oligo; kinase labeling kit for oligo probe; and labeled nucleotides. Typical reagents (e.g., as might be found in a typical MethyLight^{™} -based kit) for MethyLight^{™} analysis may include, but are not limited to: PCR primers for the bisulfite converted sequence of the TFAP2E gene; bisulfite specific probes (e.g. TaqMan^{™} or Lightcycler^{™}); optimized PCR buffers and deoxynucleotides; and Taq polymerase.

Typical reagents (e.g., as might be found in a typical Ms-SNuPE^{™}-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for the bisulfite converted sequence of the TFAP2E gene; reaction buffer (for the Ms-SNuPE reaction); and labelled nucleotides.

Typical reagents (e.g., as might be found in a typical MSP-based kit) for MSP analysis may include, but are not limited to: methylated and unmethylated PCR primers for the bisulfite converted sequence of the TFAP2E gene, optimized PCR buffers and deoxynucleotides, and specific probes.

Moreover, an additional aspect of the present disclosure is an alternative kit comprising a means for determining TFAP2E methylation, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 to SEQ ID NO: 2; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from SEQ ID NO: 1 to SEQ ID NO: 2.

In a further embodiment said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 1 to SEQ ID NO: 2.

In a preferred embodiment the kit may comprise additional reagents selected from the group consisting: buffer (e.g. restriction enzyme, PCR, storage or washing buffers); DNA recovery reagents or kits (e.g., precipitation, ultrafiltration, affinity column) and DNA recovery components. In a further alternative embodiment, the kit may contain, packaged in separate containers, a polymerase and a reaction buffer optimised for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 2; and optionally (d) instructions for use and interpretation of the kit results. In an alternative preferred embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 2; and optionally (d) instructions for use and interpretation of the kit results.

In an alternative embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 2; (d) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 2 and optionally (e) instructions for use and interpretation of the kit results.

The kit may also contain other components such as buffers or solutions suitable for blocking, washing or coating, packaged in a separate container.

The disclosure further relates to a kit for use in providing a diagnosis of the presence of a cell proliferative disorder in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for the gene TFAP2E and comprises a sequence of at least 15 base pairs in length but no more than 200 bp of a sequence according to one of SEQ ID NO: 1 to SEQ ID NO: 2. Preferably said sequence is at least 15 base pairs in length but no more than 80 bp of a sequence according to one of SEQ ID NO: 1 to SEQ ID NO: 2. It is further preferred that said sequence is at least 20 base pairs in length but no more than 30 bp of a sequence according to one of SEQ ID NO: 1 to SEQ ID NO: 2.

Said test kit preferably further comprises a restriction enzyme component comprising one or a plurality of methylation-sensitive restriction enzymes.

In a further embodiment said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The described invention further discloses a composition of matter useful for detecting, differentiation and distinguishing between colon cell proliferative disorders. Said composition comprising at least one nucleic acid 18 base pairs in length of a segment of the nucleic acid sequence disclosed in SEQ ID NO: 3 to SEQ ID NO: 10, and one or more substances taken from the group comprising : 1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 3 to SEQ ID NO: 10 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

In further preferred embodiments of the disclosure said at least one nucleic acid is at least 50, 100, 150, 200, 250 or 500 base pairs in length of a segment of the nucleic acid sequence disclosed in SEQ ID NO: 3 to SEQ ID NO: 10.

While the present invention has been described with specificity in accordance with certain of its preferred embodiments, the following examples serve only to illustrate the invention and are not intended to limit the invention within the principles and scope of the broadest interpretations and equivalent configurations thereof.

### Example 1

In the following investigation, the performance of selected genes according to Table 2 were analyzed as colorectal carcinoma detection markers by means of the HM (Heavymethy) assay. Target regions of each gene were bisulfite converted and amplified by means of non-MSP primers, in the presence of a blocker oligonucleotides designed to suppress amplificates that had not been methylated prior to bisulfite treatment. Amplificates were then detected by means of Lightcycler (dual) probes.

Plasma samples from the following patient classes were analyzed:
- Colorectal carcinoma (131 total)
   Stage 0 = 1
   Stage I = 13
   Stage II = 32
   Stage III = 27
   Stage IV = 8
   Unclassified = 50
- Healthy colorectal (colonoscopy verified) = 169
- Non- cancerous diseases (NCD) = 29
- Cancers of non-colorectal origin (NCC) = 31

In total 360 samples were analyzed.

### DNA extraction and bisulfite treatment

The DNA was isolated from the all samples by means of the Magna Pure method (Roche) according to the manufacturer's instructions. The eluate resulting from the purification was then converted according to the following bisulfite reaction.

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

### Reaction solutions and thermal cycling conditions

PCR assay component sequences are provided in Table 3. Each assay was performed twice (independently) in each sample.

Thermal cycling conditions were:

| | | | | |
|---|---|---|---|---|
| PCDHGC3 | | | | |
| activation: | | 95°C | 10min | |
| | | | | |
| 50 cycles: | 95°C | | 10 sec | (20°C/s;) |
| 56°C | 30 sec | | (20°C/s) | |
| 60°C | 3 sec | | (20°C/s)detection | |
| 72°C | 10 sec | | (20°C/s) | |
| | | | | |
| melting curve: | | 95°C | 10 sec | (20°C/s) |
| 40°C | 10 sec | | (20°C/s) | |
| 95°C | 0 sec | | (0,1°C/s)Continuous | |
| cooling: | | 40°C | 5 sec | |
| | | | | |
| All other assays: | assays: | | | |
| activation: | | 95°C | 10 min | |
| 55 cycles: | | 95°C | 10 sec | (20°C/s) |
| 56°C | 30 sec | | (20°C/s)detection | |
| 72°C | 10 sec | | (20°C/s) | |
| | | | | |
| melting curve: | | 95°C | 10 sec | (20°C/s) |
| 40°C | 10 sec | | (20°C/s) | |
| 95°C | 0 sec | | (0,1°C/s)Continuous | |
| | | | | |
| cooling: | | 40°C | 5 sec | |

### Results:

In order to predict the presence of CRC tumour DNA in the measured plasma samples we use a logistic regression model. The logistic regression model is build as follows. First the measurement data for each marker assay is encoded in a qualitative way by the following 3 levels:
- Level 0 - both replicate PCR reactions showed no amplification
- Level 1 - exactly one of the two PCR replicates showed an amplification curve
- Level 2 - both of the two PCR replicates showed amplification curves

If any of the two PCR replicates could not be successfully measured the respective marker measurement was regarded as invalid.

The five different DNA methylation markers were used as independent factors with 3 levels in a logistic regression model. An additional intercept factor but no factor interactions were included in the model. The logistic regression model was trained and optimal weights for all factor levels were determined by using the maximum likelihood procedure.

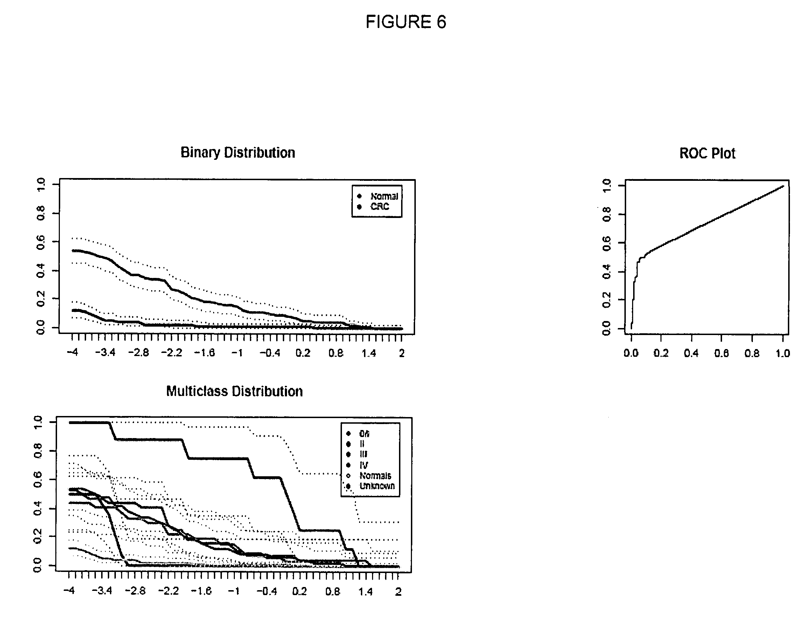

Figures 1 to 10 provide the plots of the measured log mean methylation of the individual assays. Each figures consists of three plots, the upper and lower left hand side plots provide the binary and multiclass analysis respectively, the right hand plot provides an ROC wherein sensitivity is shown on the Y-axis and 1 -specificity is shown on the X-axis. Table 4 and figures 1 to 5 provide an overview of marker performances in all sample groups. Table 5 and figures 6 to 10 provide an overview of marker performances in the colorectal carcinoma and normal colorectal groups.

Figures 12 to 21 provide the plots of the measured log majority mean (analyzed sample is only counted as positive if both replicates are positive, the mean of the two measurements is taken as the quantitative methylation measurement) methylation of the individual assays. Each figures consists of three plots, the upper and lower left hand side plots provide the binary and multiclass analysis respectively, the right hand plot provides an ROC wherein sensitivity is shown on the Y-axis and 1-specificity is shown on the X-axis. Table 6 and figures 12 to 16 provide an overview of marker performances in all sample groups. Table 7 and figures 18 to 21 provide an overview of marker performances in the colorectal carcinoma and normal colorectal groups.

Table 8 provides an overview of the AUC and sensitivities of the single assays at 95% specificity (all p-values were less than 0.00001). Wherein said classes are:
All: Normal + NCD +NCC vs. CRC stages I to IV
I-IV: Normal vs. CRC stages I to IV
I-III: Normal vs. CRC stages I to III

From the multiclass distribution of figure 6 (bottom left hand plot) and table 1 1 it can be determined that the gene RASSF2 is particularly effective at detecting Stage 1 and early colorectal carcinomas. Accordingly expression, most preferably CpG methylation, of said gene is in addition to being a preferred diagnostic marker is particularly preferred for the screening of general populations (individuals not displaying any indicators or symptoms of colorectal carcinoma) for the early detection of colorectal carcinomas.

### Marker combinations (panels)

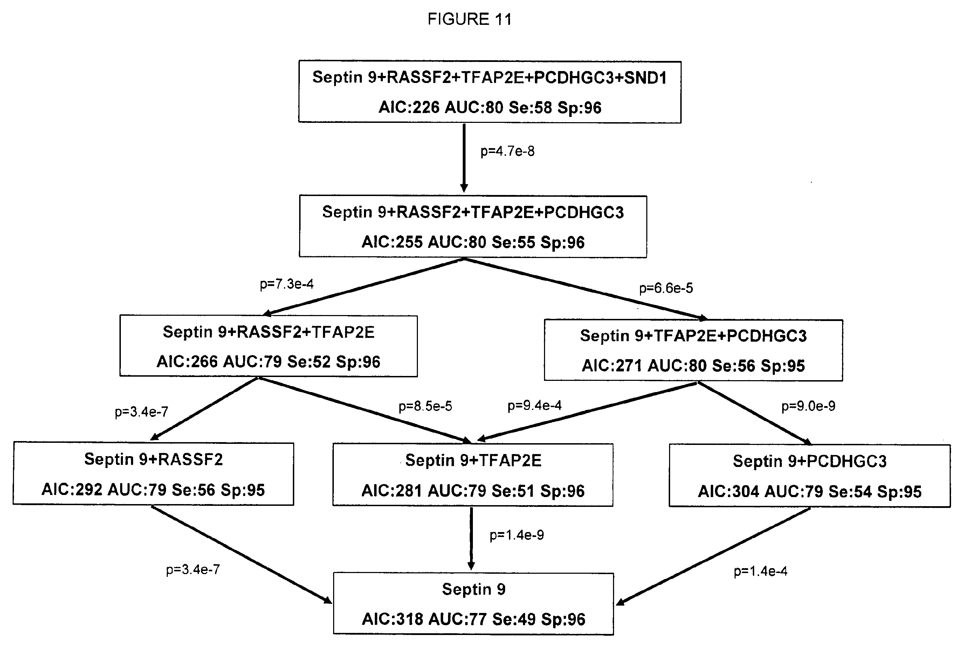

To identify the subset of DNA methylation markers that optimally predicts the presence of CRC we use the backward elimination procedure. In each elimination step the DNA methylation marker with the lowest factor levels was removed from the model. We compared the predictive power of the reduced model with the complete model by using the likelihood ratio test. To identify the subset of DNA methylation markers that optimally predicts the presence of CRC we use the backward elimination procedure . In each elimination step the DNA methylation marker with the lowest factor levels was removed from the model. We compared the predictive power of the reduced model with the complete model by using the likelihood ratio test. Fig. 11 shows that (in the Normal vs. CRC stages I to IV comparison) at each elimination step the predictive power of the logistic regression model was significantly reduced. We conclude that all listed DNA methylation marker models give superior prediction performance as compared to the single marker or the respective simpler marker panels. We conclude that the following DNA marker models give superior prediction performance as compared to the single marker or the respective simpler marker panels.

Figures 22 to 26 provide an overview of the performance of the following marker combinations:
Septin 9+TFAP2E+RASSF2+PCDHGC3+SND1 (Figure 22)

| | | |
|---|---|---|
| All | AUC80 | |
| Sens/Spec | 57/95 | |
| All CRC | AUC | 80 |
| Sens/Spec | 58/96 | |
| CRC I-III | AUC | 76 |
| Sens/Spec | 50/96 | |

Septin 9+TFAP2E+RASSF2+PCDHGC3 (Figure 23)

| | | |
|---|---|---|
| All | AUC80 | |
| Sens/Spec | 53/95 | |
| All CRC | AUC | 80 |
| Sens/Spec | 55/96 | |
| CRC I-III | AUC | 77 |
| Sens/Spec | 48/96 | |

Septin 9+TFAP2E+RASSF2 (Figure 24)

| | | |
|---|---|---|
| All | AUC 77 | |
| Sens/Spec | 48/96 | |
| All CRC | AUC | 79 |
| Sens/Spec | 52/96 | |
| CRC I-III | AUC | 75 |
| Sens/Spec | 42/96 | |

Septin 9+TFAP2E (Figure 25)

| | | |
|---|---|---|
| All | AUC 77 | |
| Sens/Spec | 45/96 | |
| All CRC | AUC | 79 |
| Sens/Spec | 51/96 | |
| CRC I-III | AUC | 75 |
| Sens/Spec | 41/96 | |

Septin 9+RASSF2 (Figure 26)

| | | |
|---|---|---|
| All | AUC 77 | |
| Sens/Spec | 43/96 | |

| | | |
|---|---|---|
| All CRC | AUC | 79 |
| Sens/Spec | 56/95 | |
| CRC I-III | AUC | 74 |
| Sens/Spec | 46/95 | |

In each case the upper plot shows all samples (normals, non colorectal disease, non colorectal cancers and all CRC stages), the lower plot shows only normal and CRC samples.

### Example 2: Performance of marker in prostate cancer diagnosis

In the following investigation, the performance of selected markers in detecting prostate carcinoma was determined by means of the HM (HeavyMethyl) assay. Target regions of each gene were bisulfite converted and amplified by means of non-MSP primers, in the presence of a blocker oligonucleotide designed to suppress amplificates that had not been methylated prior to bisulfite treatment. Amplificates were then detected by means of Lightcycler (dual) probes and the level of methylation was determined by reference to control assays.

### Samples

For this experiment, we collected matched plasma and urine from a total of 191 men, including 91 males with biopsy-confirmed prostate cancer, 51 males with no cancer detected by biopsy (subsequently diagnosed with BPH), and 50 young healthy males. In all analyses, the positive class is comprised of the Prostate cancer samples.

In designing the Present clinical study, the primary difficulty was in the definition of the negative class as there is no detection method that excludes presence of Prostate cancer with 100% certainty. Biopsy has a false negative diagnosis rate of at least 10% while PSA measurement is prone to both false negatives and false positives. Because the primary objective of the Present Study was to demonstrate the feasibility of measuring methylated markers of Prostate cancer in a remote body fluid, we focused on a negative class that minimized the probability of false positives. Consequently, young healthy males were chosen as the "true" negative class. We reasoned that young healthy males with no family history of prostate cancer should be truly negative for Prostate cancer.

In order to investigate the markers as a diagnostic follow-on to PSA, we also included a second negative class of biopsy negative, BPH samples. A potentially confounding factor in this class is the likely presence of false negative biopsies.

In five Prostate cancer cases, only a plasma sample was collected and in ten additional cases only a urine sample was collected. The samples were collected at multiple sites. The urine was collected after a prostatic massage. Both plasma and urine samples were obtained before any treatment for Prostate cancer. Inclusion and exclusion criteria were designed to ensure that the patients analyzed reflect the potential patients who would use Prostate cancer screening tests.

The following inclusion and exclusion criteria applied to the patients undergoing biopsy:

### Inclusion criteria:

- Indication for biopsy (elevated PSA and/or suspicious DRE)
- Biopsy scheduled within 1 week after sample collection
- Age 40-80

### Exclusion criteria:

Any prior treatment for prostate cancer
History of cancer or serious illness in the past 5 years
Symptoms of urinary tract infection

The following criteria applied to the healthy men of the control group:

### Inclusion criteria:

- Male
- Age 18-30

### Exclusion criteria:

Any prior treatment for or symptoms of prostate cancer or prostate disease
History of cancer or serious illness in the past 5 years
Symptoms of urinary tract infection

### DNA extraction

DNA was extracted and isolated using standard protocols and commercially available kits.

### Bisulfite treatment

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane.

### Real-time LightCycler PCR assay

For each assay, 1.5 ml analyte equivalent was run in duplicate, methylation was determined by means of the assay components according to Table 9. Control assays for the beta actin gene and "CFF" regions was used to determine total DNA concentration, in order to quantiate the amount of methylated DNA as determined by the Heavymethyl assay.

### Results

One of the objectives was to develop markers targeted as a diagnostic follow-on to PSA tests of 4.0 ng/ml or more for men over 50 years of age to discriminate prostate cancer from non-cancerous conditions. We further focused on two indications: a screening application to identify men over 50 years with a high risk of prostate cancer and a diagnostic follow-on to PSA to inform the prostate re-biopsy decision in men with at least one negative prostate biopsy and persistently elevated PSA. We analyzed the data in two different ways: (i) we used prostate cancer and biopsy-negative samples to assess markers performance in the follow-on to PSA test (diagnostic application) and (ii) we used prostate cancer and all the non-cancer (biopsy-negative and healthy) samples to measure markers performance in screening test (screening application). We report marker performance for plasma and urine separately, we also provide data analysis for individual markers and marker panels. All data are reported as logmean raw methylation values.

### Marker performance in Screening Application

As a primary screening test, the marker would need to identify Prostate cancer in men over age 50 years with improved specificity relative to PSA. AU screening application analyses use the Prostate cancer samples as the positive class. For the purposes of the Present clinical study, we analyzed data for our screening application with two alternative negative classes. The first negative class analyzed the 50 young healthy males with minimal likelihood of undetected Prostate cancer. While this negative class represents a "true" test negative, it is not age-matched to the target Prostate cancer screening population and does not include any likely false positive classes, e.g. BPH. Therefore, we performed a second analysis in which all 50 healthy young controls and all 51 biopsy negative controls were analyzed as a 101 sample size negative class.

On average, approximately 20,000,000 PSA tests are performed every year in the US with only approximately 1,000,000 cases moving forward to biopsy (of which approximately 750,000 biopsies are unnecessary). Therefore, less than 5% of individuals that are currently screened by PSA fall in the negative class that is represented by elevated-PSA-BPH-positive whereas as the vast majority of the target screening population fall into the PSA-low negative class. Whereas the negative class of only healthy young males may represent an overestimation of the discriminatory capacity of our markers, the combined negative class of healthy young males plus age-matched biopsy negative males may represent an underestimation of the discriminatory capacity of our markers.

### Single marker performance in urine

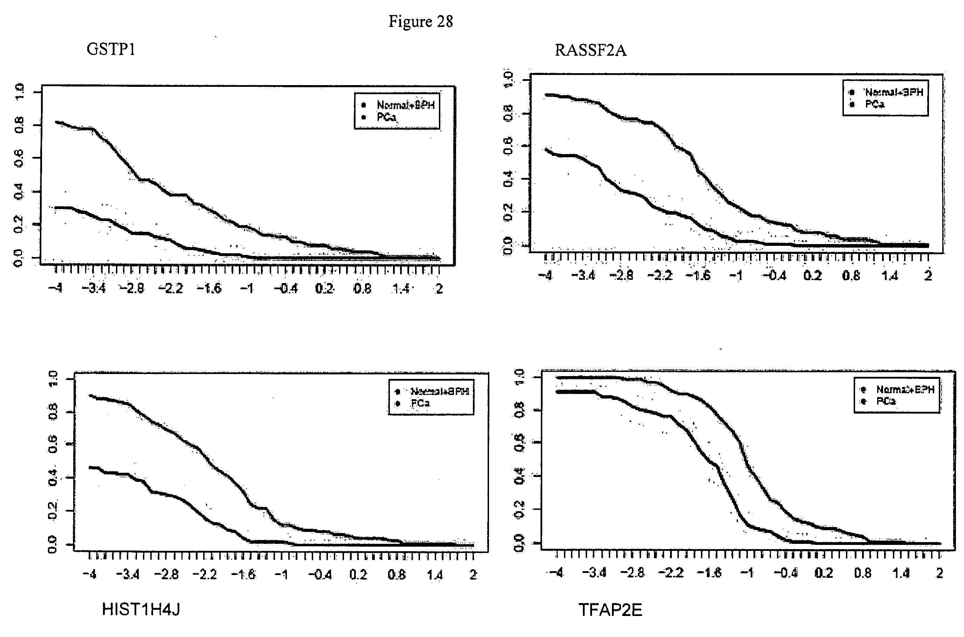

The sensitivity and specificity of markers tested by real-time PCR in post-prostatic massage urine from prostate cancer patients, biopsy negative patients and healthy control individuals is shown in Table 10, and the assay performance on post-prostatic massage urine as compared to with negative class I (healthy individuals) is shown in Figure 27. The assay performance on post-prostatic massage urine as compared to with negative II (healthy plus biopsy negative individuals) is shown in Figure 28.

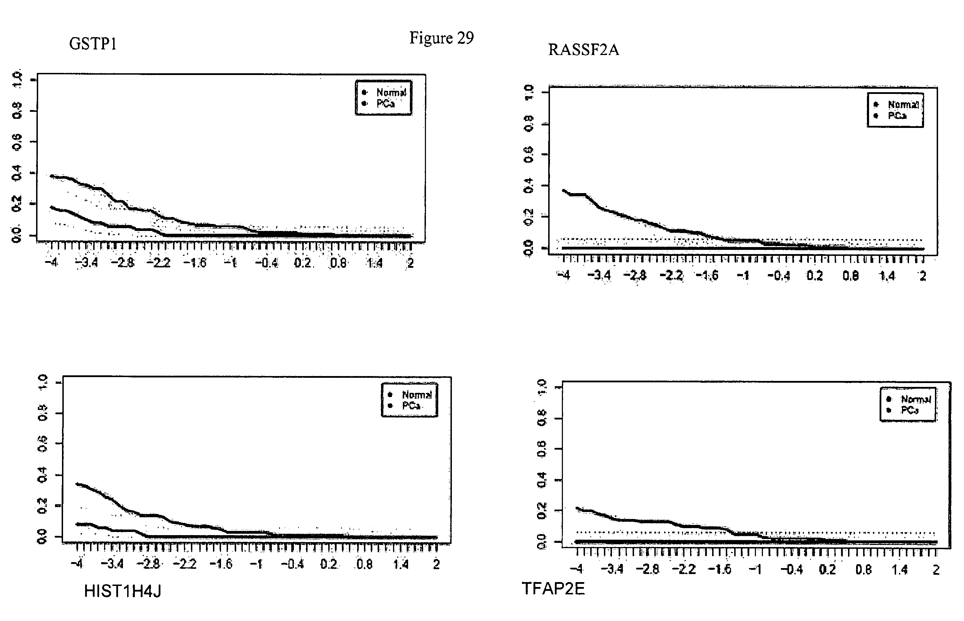

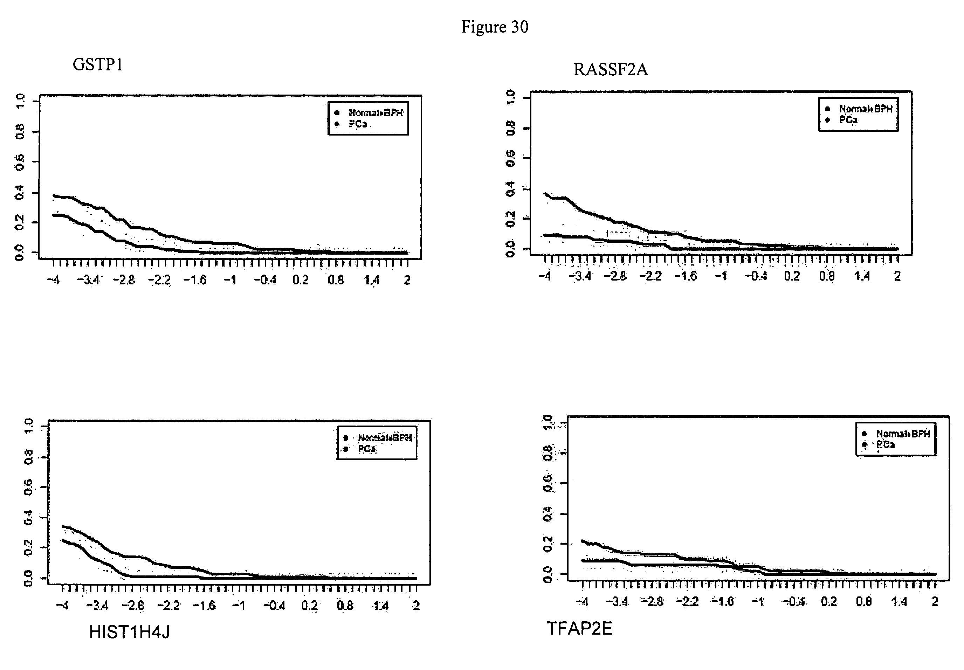

The sensitivity and specificity of markers tested by real-time PCR in plasma from prostate cancer patients, biopsy negative patients and healthy control individuals is shown in Table 11 , and the assay performance on plasma as compared to with negative class I (healthy individuals) is shown in Figure 29. The assay performance in plasma as compared to with negative II (healthy plus biopsy negative individuals) is shown in Figure 30.

In all negative class comparisons and for all markers urine was the more sensitive analyte as illustrated in Table 12.

Increasing amounts of methylated marker DNA correlated with increasing Gleason score for all markers in plasma. This was true for samples with high amounts of methylated marker DNA in urine (especially markers TFAP2E and RASSF2A), this was especially so in DNA from plasma. PSA as a marker of Prostate cancer in patients with elevated PSA (> 4 ng/ml) also correlated with increasing Gleason score.

Table 13 shows the performance of screening marker panels to distinguish prostate cancer from negative class I (healthy males) in urine. Table 14 shows the performance of screening marker panels to distinguish prostate cancer from negative class II (healthy males plus biopsy negative) in urine. Table 15 shows the performance of screening marker panels to distinguish prostate cancer from negative class I (healthy males) in plasma. Table 16 shows the performance of screening marker panels to distinguish prostate cancer from negative class II (healthy males plus biopsy negative) in plasma.

### Marker performance in diagnostic application: Follow-on to PSA

As a diagnostic application, the markers should identify Prostate cancer in men over age 50 years with persistently elevated PSA (> 4.0 ng/ml) who have undergone at least one negative prostate biopsy. This is a distinct application and analysis and requires increased discrimination as compared to the screening test. False positives in this application arise from the elevated PSA, biopsy negative BPH class. Again, the Prostate cancer samples represent the positive class. For the purposes of a diagnostic application, we analyzed the data using a single negative class comprised of the 51 biopsy negative samples.

### Single marker performance in urine

Table 17 shows the sensitivity and specificity of markers tested by real-time PCR in post-prostatic massage urine from prostate cancer patients and biopsy negative patients.

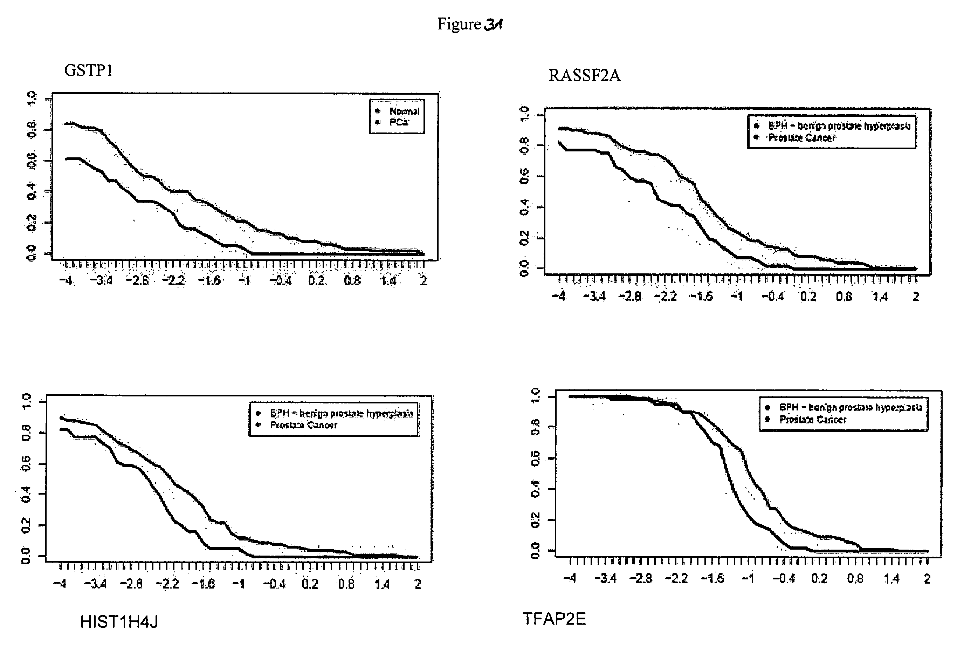

Figure 31 shows the single assay performance for HM real-time PCR assays on post-prostatic massage urine.

As shown in Table 18, for all methylation markers analyzed urine was the more sensitive analyte. Accordingly it is particularly preferred for the diagnosis of prostate cancer that the analyte is urine, either voided or post-prostatic massage.

Table 19 provides the performance of diagnostic marker panels to detect Prostate cancer in biopsy negative patients in urine.

**Table 1: Sequences according to the present invention**

| Genomic SEQ ID NO: | Gene | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|
| 1 | TFAP2E Gene | 3 | 4 | 7 | 8 |

**Table 2: Genes according to Example 1**

| Genomic SEQ ID NO: | Gene | | | Abbreviation | Bisulfite converted sequences |
|---|---|---|---|---|---|
| | | | | | |
| | | | | | |
| | Transcription factor AP-2 epsilon (acticating enhancer binding protein 2 epsilon) | | | TFAP2E | |
| | | | | | |
| | | | | | |

| | | | | | |
|---|---|---|---|---|---|
| *Unless otherwise stated all locations refer to Ensembl database v39 (June 2006) ** Ensembl database v31.35d (8 July 2005) | | | | | |

**Table 3: Primer, blocker and probe sequences according to Example 1.**

| | | | | | |
|---|---|---|---|---|---|
| TFAP2E | 46 | 51 | 56 | 61 | 66 |

**Table 4: HM assay (Example 1) performance in all tissue samples.**

| | RASSF2A | Septin 9 | SND1 | PCDHGC3 | TFAP2E |
|---|---|---|---|---|---|
| AUC (95% confidence interval) | 0.72 (0.67, 0.77) | 0.7 5 (0.7, 0.79) | 0.66 (0.6, 0.71) | 0.66 (0.6, 0.72) | 0.69 (0.63, 0.74) |
| Sens/Spec | 0.4/0.95 | 0.47/0.95 | 0.25/0.95 | 0.32/0.95 | 0.29/0.95 |
| Sens/Spec cut off | -3.029 | -2.706 | -3.089 | -2.378 | -2.692 |
| Wilcoxon P | 0 | 0 | 0 | 0 | 0 |
| CRC+Adenoma -(pos) | 131 | 131 | 118 | 119 | 119 |
| Normal+non-cancerous diseases (NCD)+carcinoma other than colorectal (NCC) -(neg) | 228 | 228 | 205 | 206 | 206 |

**Table 5: HM assay (Example 1) performance in colorectal carcinoma and normal colorectal tissue**

| | RASSF2A | Septin 9 | SND1 | PCDHGC3 | TFAP2E |
|---|---|---|---|---|---|
| AUC (95% confidence interval) | 0.73(0.67, 0.78) | 0.76(0.7, 0.8) | 0.67(0.61, 0.73) | 0.68(0.62, 0.73) | 0.71(0.65,0. 76) |
| Sens/Spec | 0.47/0.95 | 0.48/0.95 | 0.39/0.95 | 0.32/0.95 | 0.39/0.95 |
| Sens/Spec cut off | -3.272 | -2.858 | -3.473 | -2.417 | -3.446 |
| Wilcoxon P | 0 | 0 | 0 | 0 | 0 |
| CRC+Adenoma (pos) | 131 | 131 | 118 | 119 | 119 |
| Normal (neg) | 168 | 169 | 148 | 148 | 148 |

**Table 6: HM assay (Example 1) performance in all tissue samples.**

| | RASSF2A | Septin 9 | SND1 | PCDHGC3 | TFAP2E |
|---|---|---|---|---|---|
| AUC (95% confidence interval) | 0.67(0.61, 0.72) | 0.74(0.69, 0.79) | 0.63(0.57, 0.68) | 0.65(0.6, 0.7) | 0.6 5(0.6, 0.7) |
| Sens/Spec | 0.37(0.96) | 0.51 | 0.28/0.95 | 0.34/0.95 | 0.34/0.96 |
| Sens/Spec cut off | -4 | -4 | -3.45 | -2.523 | -4 |
| Wilcoxon P | 0 | 0 | 0 | 0 | 0 |
| CRC+Adenoma (pos) | 121 | 127 | 113 | 127 | 120 |
| Normal+non-cancerous diseases (NCD)+carcinoma other than colorectal (NCC)-(neg) | 206 | 220 | 194 | 224 | 203 |

**Table7: HM assay (Example 1) performance in colorectal carcinoma and normal colorectal tissues samples.**

| | RASSF2A | Septin 9 | SND1 | PCDHGC3 | TFAP2E |
|---|---|---|---|---|---|
| AUC (95% confidence interval) | 0.67(0.61, 0.73) | 0.74(0.69, 0.79) | 0.64(0.58, 0.7) | 0.66(0.6, 0.71) | 0.66(0.6, 0.72) |
| Sens/Spec | 0.37/0.97 | 0.51/0.97 | 0.3/0.97 | 0.35/0.95 | 0.34/0.98 |
| Sens/Spec cut off | -4 | -4 | -4 | -2.599 | -4 |
| Wilcoxon P | 0 | 0 | 0 | 0 | 0 |
| CRC+Adenoma (pos) | 121 | 121 | 113 | 127 | 120 |
| Normal (neg) | 154 | 164 | 146 | 167 | 154 |

**Table 8: AUC and sensitivity (at 95 % specificity) for single assays of markers according to class.***

| | AUC | | | Sensitivity | | |
|---|---|---|---|---|---|---|
| | All | I-IV | I-II | All | I-IV | I-II |
| Septin 9 (Majority mean) | 73 | 73 | 67 | 49 | 49 | 37 |
| RASSF2A (Log Mean) | 72 | 73 | 70 | 45 | 48 | 41 |
| TFAP2E (Log Mean) | 68 | 71 | 67 | 32 | 38 | 30 |
| SND1 (Log Mean) | 64 | 65 | 62 | 25 | 35 | 29 |
| PCDHGC3 (Log Mean) | 65 | 66 | 64 | 30 | 32 | 29 |

| | | | | | | |
|---|---|---|---|---|---|---|
| *all p-values were less than 0.00001 | | | | | | |

**Table 9:Assays according to Example 1.**

| | Forward Primer SEQ ID NO: | Reverse Primer SEQ ID NO: | Blocker SEQ ID NO: | Probe SEQ ID NO: | Probe SEQ ID NO: |
|---|---|---|---|---|---|
| Actin B | 77 | 78 | | 79 | |
| | | | | | |
| TFAP2E | 98 | 99 | 100 | 101 | 102 |

**Table 10**

| Marker | Negative Class I: Healthy | | | Negative Class II: Healthy + Biopsy (-) | | |
|---|---|---|---|---|---|---|
| | AUC | Sens / Spec | Wilcoxon p value | AUC | Sens / Spec | Wilcoxon p value |
| GSTP1 | 0.89 | 0.63 /0.96 | 0 | 0.79 | 0.31 / 0.96 | 0 |
| RASSF2A | .090 | 0.74 / 0.96 | 0 | 0.79 | 0.24/0.96 | 0 |
| HIST1H4J | 0.91 | 0.69 / 0.96 | 0 | 0.78 | 0.36 / 0.96 | 0 |
| TFAP2E | 0.86 | 0.47 / 0.96 | 0 | 0.76 | 0.27 / 0.96 | 0 |

**Table 11**

| Marker | Negative Class I: Healthy | | | Negative Class II: Healthy + Biopsy (-) | | |
|---|---|---|---|---|---|---|
| | AUC | Sens / Spec | Wilcoxon p value | AUC | Sens / Spec | Wilcoxon p value |
| GSTP1 | 0061 | 0.17 / 0.96 | 0.0063 | 0.58 | 0.17 / 0.95 | 0.0183 |
| RASSF2A | 0.68 | 0.37 / 1.00 | 0 | 0.64 | 0.20 / 0.95 | 0 |
| HIST1H4J | 0.64 | 0.26 / 0.96 | 5e⁻⁰⁴ | 0.56 | 0.16/0.95 | 0.0572 |
| TFAP2E | 0.61 | 0.22 / 1.00 | 4e⁻⁰⁴ | 0.56 | 0.09 / 0.95 | 0.0128 |

**Table 12**

| Marker | Negative Class I: Healthy | | Negative Class II: Healthy + Biopsy (-) | |
|---|---|---|---|---|
| | Urine AUC | Plasma AUC | Urine AUC | Plasma AUC |
| GESTP1 | 0.89 | 0.61 | 0.79 | 0.58 |
| RASSF2A | 0.90 | 0.68 | 0.79 | 0.64 |
| HIST1H4J | 0.91 | 0.64 | 0.78 | 0.56 |
| TFAP2E | 0.86 | 0.61 | 0.76 | 0.56 |

**Table 13**

| Marker Panel | % Sens Prostate cancer | % Spec Healthy |
|---|---|---|
| Quantitative Single Markers: | | |
| RASSF2A | 74 | 96 |
| HIST1H4J | 69 | 96 |
| GSTP1 | 63 | 96 |
| TFAP2E | 46 | 100 |
| Qualitative Panels: | | |
| GSTP1+HIST1H4J | 79 | 98 |
| RASSF2A+HIST1H4J | 94 | 88 |
| Quantitative Panels: | | |
| RASSF2A+HIST1H4J | 94 | 88 |
| QuadSVM (all markers, no PSA) | 79 | 98 |

**Table 14**

| Marker Panel | % Sens Prostate cancer | % Spec Healthy + Biopsy (-) |
|---|---|---|
| Quantitative Single Markers: | | |
| RASSF2A | 74 | 76 |
| HIST1H4J | 69 | 68 |
| GSTP1 | 63 | 80 |
| TFAP2E | 46 | 88 |
| Qualitative Panels: | | |
| GSTP1+HIST1H4J | 79 | 72 |
| RASSF2A+HIST1H4J | 94 | 54 |
| Quantitative Panels: | | |
| RASSF2A+HIST1H4J | 94 | 58 |
| QuadSVM (all markers, no PSA) | 79 | 76 |

**Table 15**

| Marker Panel | % Sens Prostate cancer | % Spec Healthy |
|---|---|---|
| Quantitative Single Markers: | | |
| RASSF2A | 37 | 100 |
| HIST1H4J | 26 | 96 |
| GSTP1 | 17 | 94 |
| TFAP2E | 22 | 100 |
| Qualitative Panels: | | |
| RASSF2A+HIST1H4J | 41 | 98 |
| Quantitative Panels: | | |
| RASSF2A+TFAP2E (TFAP2E used to normalize) | 32 | 100 |
| QuadSVM (all markers, no PSA) | 39 | 96 |

**Table 16**

| Marker Panel | % Sens Prostate cancer | % Spec Healthy + Biopsy (-) |
|---|---|---|
| Quantitative Single Markers: | | |
| RASSF2A | 37 | 91 |
| HIST1H4J | 26 | 88 |
| GSTP1 | 17 | 95 |
| TFAP2E | 22 | 92 |
| Qualitative Panels: | | |
| RASSF2A+HIST1H4J | 41 | 88 |
| Quantitative Panels: | | |
| RASSF2A+TFAP2E (TFAP2E used to normalize) | 32 | 92 |
| QuadSVM (all markers, no PSA) | 39 | 94 |

**Table 17**

| | Prostate cancer vs. Biopsy (-) | | |
|---|---|---|---|
| Marker | AUC | Sens / Spec | Wilcoxon p value |
| GSTP1 | 0.69 | 0.23 / 0.95 | 6e⁻⁰⁴ |
| RASSF2A | 0.66 | 0.18 /0.95 | 0.0043 |
| HIST1H4J | 0.64 | 0.28 / 0.95 | 0.0126 |
| TFAP2E | 0.65 | 0.21 / 0.95 | 0.0062 |
| ***PSA | 0.56 | 0.22 / 0.95 | 0.3098 |

| | | | |
|---|---|---|---|
| ***Tests whether PSA contains further information beyond what is contributed by the > 4 ng/ml cut-off indication for prostate biopsy. | | | |

**Table 18**

| Marker | Prostate cancer vs. Biopsy (-) | |
|---|---|---|
| | Urine AUC | Plasma AUC |
| GSTP1 | 0.69 | 0.55 |
| RASSF2A | 0.66 | 0.60 |
| HIST1H4J | 0.64 | 0.50 |
| TFAP2E | 0.65 | 0.52 |
| ***PSA | na | 0.56 |

| | | |
|---|---|---|
| ***Tests whether PSA contains further information beyond what is contributed by the > 4 ng/ml cut-off indication for prostate biopsy. | | |

**Table 19**

| Marker Panel | % Sens Prostate cancer | % Spec Biopsy (-) |
|---|---|---|
| Quantitative Single Markers: | | |
| RASSF2A | 74 | 55 |
| HIST1H4J | 69 | 41 |
| GSTP1 | 63 | 64 |
| TFAP2E | 46 | 77 |
| Qualitative Panels: | | |
| GSTP1+HIST1H4J | 79 | 46 |
| RASSF2A+HIST1H4J | 94 | 21 |
| Quantitative Panels: | | |
| RASSF2A+HIST1H4J | 94 | 27 |
| GSTP1+PSA | 83 | 45 |
| QuadSVM (all markers, no PSA) | 79 | 55 |

## Claims

1. A method for detecting prostate or colorectal carcinoma, in a subject comprising detecting the presence of methylation of TFAP2E in a biological sample isolated from said subject; wherein
(i) in the case of prostate cancer the biological sample is selected from the group consisting of prostate tissue, blood plasma, blood serum, whole blood, cells obtained from the blood obtained from the subject and urine; and
(ii) in the case of colon carcinoma, the biological sample is selected from the group consisting of colon tissue, blood plasma, blood serum, whole blood and cells obtained from the blood obtained from the subject;
wherein the presence of CpG methylation is indicative of the presence of said disorder and the absence thereof is indicative of the presence of a benign cell proliferative disorder.

2. A method for detecting prostate or colorectal carcinoma in a subject, comprising contacting genomic DNA isolated from a biological sample obtained from said subject ; wherein
(i) in the case of prostate cancer the biological sample is selected from the group consisting of prostate tissue, blood plasma, blood serum, whole blood, cells obtained from the blood obtained from the subject and urine; and
(ii) in the case of colon carcinoma, the biological sample is selected from the group consisting of colon tissue, blood plasma, blood serum, whole blood and cells obtained from the blood obtained from the subject;
with at least one reagent, or series of reagents that distinguishes between methylated and non-methylated CpG dinucleotides within at least one target region of the genomic DNA, wherein the target region comprises at least one CpG dinucleotide sequence of the TFAP2E gene and hybridizes under stringent conditions to a sequence of at least 16 contiguous nucleotides of at least one sequence selected from the group consisting of SEQ ID NO: 1 to SEQ ID NO: 2 respectively and detecting prostate or colorectal carcinoma based on the presence of CpG methylation in said at least one CpG dinucleotide sequence.

3. A method for detecting prostate or colorectal carcinoma, comprising:
a) extracting or otherwise isolating genomic DNA from a biological sample obtained from the subject; wherein
(i) in the case of prostate cancer the biological sample is selected from the group consisting of prostate tissue, blood plasma, blood serum, whole blood, cells obtained from the blood obtained from the subject and urine; and
(ii) in the case of colon carcinoma, the biological sample is selected from the group consisting of colon tissue, blood plasma, blood serum and whole blood, cells obtained from the blood obtained from the subject;
b) treating the genomic DNA of a) with one or more reagents, preferably a reagent selected from the group consisting of bisulfite, hydrogen sulfite, disulfite, and combinations thereof, to convert cytosine bases that are unmethylated in the 5-position thereof to uracil or to another base that is detectably dissimilar to cytosine in terms of hybridization properties;
c) contacting the treated genomic DNA with an amplification enzyme and at least one primer comprising, a contiguous sequence of at least 9 nucleotides that is complementary to, or hybridizes under stringent conditions to a sequence selected from the group consisting of SEQ ID NO: 3 to SEQ ID NO: 10, and complements thereof and wherein said primer comprises at least one CpG, TpG or CpA dinucleotide, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of a sequence selected from the group consisting SEQ ID NO: 1 to SEQ ID NO: 2, or an average, or a value reflecting an average methylation state or level of a plurality of CpG dinucleotides of a sequence selected from the groups consisting of SEQ ID NO: 1 to SEQ ID NO: 2, whereby detecting prostate or colorectal carcinoma is, at least in part, afforded.

4. The method of claim 3, wherein contacting or amplifying in c) comprises use of at least one method selected from the group comprising: use of a heat-resistant DNA polymerase as the amplification enzyme; use of a polymerase lacking 5 '-3' exonuclease activity; use of a polymerase chain reaction (PCR); generation of an amplificate nucleic acid molecule carrying a detectable label.

5. The method of claim 3, further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 3 to SEQ ID NO: 10, and complements thereof, wherein said nucleic acid molecule or peptide nucleic acid molecule suppresses amplification of the nucleic acid to which it is hybridized, and/or wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 3 to SEQ ID NO: 10, and complements thereof.

6. The method of claim 5, further comprising extending at least one such hybridized nucleic acid molecule by at least one nucleotide base, and/or wherein contacting or amplifying in c), comprises use of methylation-specific primers.

7. A method for detecting prostate or colorectal carcinoma, comprising:
a) extracting or otherwise isolating genomic DNA from a biological sample obtained from the subject, wherein
(i) in the case of prostate cancer the biological sample is selected from the group consisting of prostate tissue, blood plasma, blood serum, whole blood, cells obtained from the blood obtained from the subject and urine; and
(ii) in the case of colon carcinoma, the biological sample is selected from the group consisting of colon tissue, blood plasma, blood serum and whole blood, cells obtained from the blood obtained from the subject;
b) digesting the genomic DNA of a), or a fragment thereof, with one or more methylation sensitive restriction enzymes;
c) contacting the DNA restriction enzyme digest of b), with an amplification enzyme and at least two primers suitable for the amplification of a sequence comprising at least one CpG dinucleotide of a sequence selected from SEQ ID NO: 1 to SEQ ID NO: 2; and
d) determining, based on a presence or absence of an amplificate the methylation state or level of at least one CpG dinucleotide of a sequence selected from the group consisting SEQ ID NO: 1 to SEQ ID NO: 2, whereby detecting prostate or colorectal carcinoma is, at least in part, afforded.

8. The method according to claim 7, wherein the presence or absence of an amplificate is determined by means of hybridization to at least one nucleic acid or peptide nucleic acid which is identical, complementary, or hybridizes under stringent or highly stringent conditions to an at least 16 base long segment of a sequence selected from SEQ ID NO: 1 to SEQ ID NO: 2.

9. Use of
a) a nucleic acid derived from genomic SEQ ID NO: 1 to SEQ ID NO: 2 by treatment with bisulfite, wherein said nucleic acid comprises at least one CpG, TpG or CpA dinucleotide sequence,
b) a nucleic acid, comprising at least 16 contiguous nucleotides of a genomic DNA sequence selected from the group consisting of SEQ ID NO: 3 to SEQ ID NO: 10, and sequences complementary thereto, wherein the contiguous base sequence comprises at least one CpG, TpG or CpA dinucleotide sequence, or
c) a nucleic acid, comprising at least 50 contiguous nucleotides of a genomic DNA sequence selected from the group consisting of SEQ ID NO: 3 to SEQ ID NO: 10, and sequences complementary thereto, wherein the contiguous base sequence comprises at least one CpG, TpG or CpA dinucleotide sequence
for diagnosing prostate or colorectal carcinoma by methylation analysis of a biological sample, wherein
(i) in the case of prostate cancer the biological sample is selected from the group consisting of prostate tissue, blood plasma, blood serum, whole blood, cells obtained from the blood obtained from the subject and urine; and
(ii) in the case of colon carcinoma, the biological sample is selected from the group consisting of colon tissue, blood plasma, blood serum, whole blood and cells obtained from the blood obtained from the subject.

10. Use of a kit for performing the method according to claim 3, wherein the kit comprises (a) a bisulfite reagent; (b) a container suitable for containing the said bisulfite reagent and the biological sample of the patient; (c) at least one set of oligonucleotides containing two oligonucleotides whose sequences in each case are identical, are complementary, or hybridize under stringent or highly stringent conditions to a 9, or more preferably 18, base long segment of a sequence selected from SEQ ID NO: 3 to SEQ ID NO: 10, or a kit suitable for performing the method according to claim 9 comprising (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of a sequence selected from SEQ ID NO: 1 to SEQ ID NO: 2; and optionally (d) instructions for use and interpretation of the kit results.

## Patentansprüche

1. Verfahren zum Nachweisen von Prostata- oder Kolorektalkarzinom in einem Individuum, bei dem das Vorliegen von Methylierung von TFAP2E in einer aus diesem Individuum isolierten biologischen Probe nachgewiesen wird; wobei
i. die biologische Probe im Fall von Prostatakrebs aus der Gruppe bestehend aus Prostatagewebe, Blutplasma, Blutserum, Vollblut, aus von dem Individuum gewonnenen Blut gewonnenen Zellen und Urin ausgewählt ist; und
ii. die biologische Probe im Fall von Kolonkarzinom aus der Gruppe bestehend aus Kolongewebe, Blutplasma, Blutserum, Vollblut und aus von dem Individuum gewonnenen Blut gewonnenen Zellen ausgewählt ist;
wobei das Vorliegen von CpG-Methylierung auf das Vorliegen dieser Erkrankung hinweist und deren Fehlen auf eine gutartige zellproliferative Erkrankung hinweist.

2. Verfahren zum Nachweisen von Prostata- oder Kolorektalkarzinom in einem Individuum, bei dem genomische DNA aus einer von diesem Individuum gewonnenen biologischen Probe in Kontakt gebracht wird; wobei
i. die biologische Probe im Fall von Prostatakrebs aus der Gruppe bestehend aus Prostatagewebe, Blutplasma, Blutserum, Vollblut, aus von dem Individuum gewonnenen Blut gewonnenen Zellen und Urin ausgewählt ist; und
ii. die biologische Probe im Fall von Kolonkarzinom aus der Gruppe bestehend aus Kolongewebe, Blutplasma, Blutserum, Vollblut und aus von dem Individuum gewonnenen Blut gewonnenen Zellen ausgewählt ist;
mit mindestens einem Reagenz oder einer Reihe von Reagenzien, die zwischen methylierten und nicht-methylierten CpG-Dinukleotiden innerhalb mindestens einer Zielregion der genomischen DNA unterscheiden, wobei die Zielregion mindestens eine CpG-Dinukleotid-Sequenz des TFAP2E-Gens umfasst und unter stringenten Bedingungen an eine Sequenz von mindestens 16 zusammenhängenden Nukleotiden hybridisiert, die ausgewählt ist aus der Gruppe bestehend aus SEQ ID NO: 1 bis SEQ ID NO: 2, und bei dem Prostata- oder kolorektales Karzinom basierend auf der Anwesenheit von CpG-Methylierung dieser mindestens einen CpG-Dinukleotid-Sequenz nachgewiesen wird.

3. Verfahren zum Nachweisen von Prostata- oder Kolorektalkarzinom, das die folgenden Schritte umfasst:
a) Extraktion oder anderweitige Isolierung genomischer DNA aus einer von dem Individuum gewonnenen biologischen Probe; wobei
i. die biologische Probe im Fall von Prostatakrebs aus der Gruppe bestehend aus Prostatagewebe, Blutplasma, Blutserum, Vollblut, aus von dem Individuum gewonnenen Blut gewonnenen Zellen und Urin ausgewählt ist; und
ii. die biologische Probe im Fall von Kolonkarzinom aus der Gruppe bestehend aus Kolongewebe, Blutplasma, Blutserum, Vollblut und aus von dem Individuum gewonnenen Blut gewonnenen Zellen ausgewählt ist;
b) Behandeln der genomischen DNA aus a) mit einem oder mehreren Reagenzien, vorzugsweise einem Reagenz, das ausgewählt ist aus der Gruppe bestehend aus Bisulfit, Hydrogensulfit, Disulfit und Kombinationen davon, um Cytosin-Basen, die in ihrer 5-Position unmethyliert sind, in Uracil oder eine andere Base umzuwandeln, die hinsichtlich ihrer Hyblidisierungseigenschaften nachweisbar ungleich zu Cytosin ist;
c) Inkontaktbringen der behandelten genomischen DNA mit einem Amplifikationsenzym und mindestens einem Primer, der eine zusammenhängende Sequenz von mindestens 9 Nukleotiden umfasst, die zu einer Sequenz ausgewählt aus der Gruppe bestehend aus SEQ ID NO: 3 bis SEQ ID NO: 10 und Komplementen davon komplementär ist oder an diese unter stringenten Bedingungen hybridisiert, und wobei der Primer mindestens ein CpG-, TpG- oder CpA-Dinukleotid aufweist, wobei die behandelte genomische DNA oder das Fragment davon entweder amplifiziert wird, um mindestens ein Amplifikat zu erzeugen, oder nicht amplifiziert wird; und
d) Bestimmen, basierend auf einer Anwesenheit oder Abwesenheit oder einer Eigenschaft dieses Amplifikats, des Methylierungsstatus oder -niveaus mindestens eines CpG-Dinukleotids von einer Sequenz, die ausgewählt ist aus der Gruppe bestehend aus SEQ ID NO: 1 bis SEQ ID NO: 2, oder eines Mittelwerts oder eines Werts, der einen durchschnittlichen Methylierungsstatus oder -niveau einer Vielzahl von CpG-Dinukleotiden von einer Sequenz widerspiegelt, die ausgewählt ist aus der Gruppe bestehend aus SEQ ID NO: 1 bis SEQ ID NO: 2, wobei der Nachweis von Prostata- oder Kolorektalkarzinom zumindest teilweise ermöglicht wird.

4. Verfahren nach Anspruch 3, bei dem das Inkontaktbringen oder Amplifizieren in c) mindestens ein Verfahren umfasst, das ausgewählt ist aus der Gruppe bestehend aus: Verwendung einer hitzebeständigen DNA-Polymerase als Amplifikationsenzym; Verwendung einer Polymerase ohne 5'-3'-Exonukleaseaktivität; Verwendung einer Polymerasekettenreaktion (PCR); Erzeugung eines Amplifikat-Nukleinsäuremoleküls mit einem detektierbaren Label.

5. Verfahren nach Anspruch 3, ferner umfassend in Schritt d) die Verwendung mindestens eines Nukleinsäuremoleküls oder Peptid-Nukleinsäuremoleküls, das jeweils eine zusammenhängende Sequenz von mindestens 9 Nukleotiden Länge umfasst, welche zu einer aus der Gruppe bestehend aus SEQ ID NO: 3 bis SEQ ID NO: 10 und Komplementen davon ausgewählten Sequenz komplementär ist oder an diese unter mäßig stringenten oder stringenten Bedingungen hybridisiert, wobei dieses Nukleinsäuremolekül oder Peptid-Nukleinsäuremolekül die Amplifizierung derjenigen Nukleinsäure, an die es hybridisiert ist, unterdrückt, und/ oder wobei das Bestimmen in d) die Hybridisierung von mindestens einem Nukleinsäuremolekül oder Peptid-Nukleinsäuremolekül umfasst, das jeweils eine zusammenhängende Sequenz von mindestens 9 Nukleotiden Länge umfasst, welche zu einer aus der Gruppe bestehend aus SEQ ID NO: 3 bis SEQ ID NO: 10 und Komplementen davon ausgewählten Sequenz komplementär ist oder an diese unter mäßig stringenten oder stringenten Bedingungen hybridisiert.

6. Verfahren nach Anspruch 5, bei dem mindestens ein solches hybridisiertes Nukleinsäuremolekül um zumindest eine Nukleotidbase erweitert wird, und/ oder bei dem das Inkontaktbringen oder Amplifizieren in c) die Verwendung von methylierungsspezifischen Primern umfasst.

7. Verfahren zum Nachweisen von Prostata- oder Kolorektalkarzinom, das die folgenden Schritte umfasst:
a) Extraktion oder anderweitige Isolierung genomischer DNA aus einer von dem Individuum gewonnenen biologischen Probe; wobei
i. die biologische Probe im Fall von Prostatakrebs aus der Gruppe bestehend aus Prostatagewebe, Blutplasma, Blutserum, Vollblut, aus von dem Individuum gewonnenen Blut gewonnenen Zellen und Urin ausgewählt ist; und
ii. die biologische Probe im Fall von Kolonkarzinom aus der Gruppe bestehend aus Kolongewebe, Blutplasma, Blutserum, Vollblut und aus von dem Individuum gewonnenen Blut gewonnenen Zellen ausgewählt ist;
b) Verdauen der genomischen DNA aus a), oder eines Fragments davon, mit einem oder mehreren methylierungssensitiven Restriktionsenzymen;
c) Inkontaktbringen des DNA-Restriktionsenzym-Verdaus aus b) mit einem Amplifikationsenzym und mindestens zwei Primern, die für die Amplifikation einer Sequenz geeignet sind, die mindestens ein CpG-Dinukleotid von einer aus SEQ ID NO: 1 bis SEQ ID NO: 2 ausgewählten Sequenz umfasst; und
d) Bestimmen, basierend auf der Anwesenheit oder Abwesenheit eines Amplifikats, des Methylierungsstatus oder -niveaus von mindestens einem CpG-Dinukleotid von einer Sequenz ausgewählt aus der Gruppe bestehend aus SEQ ID NO: 1 bis SEQ ID NO: 2, wobei der Nachweis von Prostata- oder Kolorektalkarzinom zumindest teilweise ermöglicht wird.

8. Verfahren nach Anspruch 7, wobei die Anwesenheit oder Abwesenheit eines Amplifikats mittels Hybridisierung an mindestens eine Nukleinsäure oder Peptid-Nukleinsäure bestimmt wird, die zu einem mindestens 16 Basen langen Segment einer aus der Gruppe bestehend aus SEQ ID NO: 1 bis SEQ ID NO: 2 ausgewählten Sequenz identisch ist, komplementär ist, oder unter stringenten oder hochstringenten Bedingungen an dieses hybridisiert.

9. Verwendung
a) einer Nukleinsäure, die durch Bisulfit-Behandlung aus genomischer SEQ ID NO: 1 bis SEQ ID NO: 2 gewonnen wird, wobei diese Nukleinsäure mindestens eine CpG-, TpG- oder CpA-Dinukleotid-Sequenz umfasst,
b) einer Nukleinsäure, die mindestens 16 zusammenhängende Nukleotide von einer genomischen DNA-Sequenz umfasst, die ausgewählt ist aus der Gruppe bestehend aus SEQ ID NO: 3 bis SEQ ID NO: 10 und dazu komplementären Sequenzen, wobei die komplementäre Basensequenz mindestens eine CpG-, TpG- oder CpA-Dinukleotidsequenz aufweist, oder
c) eine Nukleinsäure, die mindestens 50 zusammenhängende Nukleotide einer genomischen DNA-Sequenz umfasst, die ausgewählt ist aus der Gruppe bestehend aus SEQ ID NO: 3 bis SEQ ID NO: 10 und dazu komplementären Sequenzen, wobei die zusammenhängende Basensequenz mindestens eine CpG-, TPG- oder CpA-Dinukleotidsequenz umfasst,
zur Diagnose von Prostata- oder Kolorektalkarzinom durch Methylierungsanalyse einer biologischen Probe, wobei
i. die biologische Probe im Fall von Prostatakrebs aus der Gruppe bestehend aus Prostatagewebe, Blutplasma, Blutserum, Vollblut, aus von dem Individuum gewonnenen Blut gewonnenen Zellen und Urin ausgewählt ist; und
ii. die biologische Probe im Fall von Kolonkarzinom aus der Gruppe bestehend aus Kolongewebe, Blutplasma, Blutserum, Vollblut und aus von dem Individuum gewonnenen Blut gewonnenen Zellen ausgewählt ist.

10. Verwendung eines Kits zum Durchführen des Verfahrens nach Anspruch 3, wobei das Kit Folgendes umfasst: (a) ein Bisulfit-Reagenz; (b) einen Behälter, der geeignet ist, das genannte Bisulfit-Reagenz und die biologische Probe des Individuums zu enthalten; (c) mindestens ein Set Oligonukleotide, das zwei Oligonukleotide enthält, deren Sequenzen jeweils mit einem 9 oder bevorzugter 18 Basen langen Segment einer aus SEQ ID NO: 3 bis SEQ ID NO: 10 ausgewählten Sequenz identisch sind, komplementär sind oder unter stringenten oder hochstringenten Bedingungen an dieses hybridisieren, oder ein Kit das zur Durchführung des Verfahrens nach Anspruch 9 geeignet ist und das Folgendes umfasst: (a) ein methylierungssensitives Restriktionsenzymreagenz; (b) einen Behälter, der geeignet ist, das genannte Reagenz und die biologische Probe des Individuums zu enthalten; (c) mindestens ein Set Oligonukleotide, das eine oder eine Vielzahl von Nukleinsäuren oder Peptid-Nukleinsäuren enthält, die mit einem mindestens 9 Basen langen Segment einer aus SEQ ID NO: 1 bis SEQ ID NO: 2 ausgewählten Sequenz identisch sind, komplementär sind oder unter stringenten oder hochstringenten Bedingungen an dieses hybridisieren; und gegebenenfalls (d) Instruktionen zur Verwendung und Interpretation der Kit-Ergebnisse.

## Revendications

1. Procédé pour la détection d'un carcinome prostatique ou colorectal chez un sujet, comprenant la détection de la présence d'une méthylation de TFAP2E dans un échantillon biologique isolé à partir dudit sujet, dans lequel
(i) dans le cas du cancer de la prostate, l'échantillon biologique est choisi dans le groupe consistant en tissu prostatique, plasma sanguin, sérum sanguin, sang total, cellules obtenues à partir du sang prélevé sur le sujet, et urine; et
(ii) dans le cas du carcinome du côlon, l'échantillon biologique est choisi dans le groupe consistant en tissu du côlon, plasma sanguin, sérum sanguin, sang total, et cellules obtenues à partir du sang prélevé sur le sujet;
tandis que la présence d'une méthylation du CpG est indicatrice de la présence du dit trouble, et que l'absence de celle-ci est indicatrice de la présence d'un trouble bénin de la prolifération cellulaire.

2. Procédé pour la détection d'un carcinome prostatique ou colorectal chez un sujet, comprenant la mise en contact d'ADN génomique isolé à partir d'un échantillon biologique obtenu à partir dudit sujet;
dans lequel
(i) dans le cas d'un cancer de la prostate l'échantillon biologique est choisi dans le groupe consistant en tissu prostatique, plasma sanguin, sérum sanguin, sang total, cellules obtenues à partir du sang prélevé sur le sujet et
urine; et
(ii) dans le cas d'un carcinome du côlon, l'échantillon biologique est choisi dans le groupe consistant en tissu du côlon, plasma sanguin, sérum sanguin, sang total et cellules obtenues à partir du sang prélevé sur le sujet;
avec au moins un réactif, ou une série de réactifs faisant la distinction entre les dinucléotides CpG méthylés et non-méthylés, dans au moins une région cible de l'ADN génomique, tandis que la région cible comprend au moins une séquence dinucléotidique CpG du gène TFAP2E et hybride dans des conditions stringentes à une séquence d'au moins 16 nucléotides contigus d'au moins une séquence choisie dans le groupe consistant en SEQ ID NO: 1 à SEQ ID NO: 2 respectivement, et la détection du carcinome prostatique ou colorectal sur la base de la présence d'une méthylation de CpG dans ladite au moins une séquence dinucléotidique CpG.

3. Procédé pour la détection d'un carcinome prostatique ou colorectal, comprenant:
a) l'extraction ou l'isolement d'une autre manière, d'ADN génomique à partir d'un échantillon biologique obtenu à partir du sujet; tandis que
(i) dans le cas d'un cancer de la prostate l'échantillon biologique est choisi dans le groupe consistant en tissu prostatique, plasma sanguin, sérum sanguin, sang total, cellules obtenues à partir du sang prélevé sur le sujet, et urine; et
(ii) dans le cas d'un carcinome du côlon, l'échantillon biologique est choisi dans le groupe consistant en tissu du côlon, plasma sanguin, sérum sanguin et sang total, cellules obtenues à partir du sang prélevé sur le sujet;
b) le traitement de l'ADN génomique de a) avec un ou plusieurs réactifs, de préférence un réactif choisi dans le groupe consistant en bisulfite, hydrogéno sulfite, disulfite, et leurs combinaisons, pour convertir les bases cytosine qui sont non-méthylées sur leur position 5 en uracile ou en une autre base qui est dissemblable de manière détectable de la cytosine en termes de propriétés d'hybridation;
c) la mise en contact de l'ADN génomique traité avec une enzyme d'amplification et au moins une amorce comprenant une séquence contiguë d'au moins 9 nucléotides qui est complémentaire de, ou hybride dans des conditions stringentes avec une séquence choisie dans le groupe consistant en SEQ ID NO: 3 à SEQ ID NO: 10, et leurs compléments et dans laquelle ladite amorce comprend au moins un dinucléotide CpG, TpG ou CpA, tandis que l'ADN génomique traité ou le fragment de celui-ci soit est amplifié pour produire au moins un produit d'amplification soit n'est pas amplifié; et
d) la détermination, sur la base d'une présence ou d'une absence de, ou sur la base d'une propriété dudit produit d'amplification, de l'état ou du taux de méthylation d'au moins un dinucléotide CpG d'une séquence choisie dans le groupe consistant en SEQ ID NO: 1 à SEQ ID NO: 2, ou d'une moyenne, ou d'une valeur reflétant un état ou taux moyen de méthylation d'une pluralité de dinucléotides CpG d'une séquence choisie dans les groupes consistant en SEQ ID NO: 1 à SEQ ID NO: 2, procurant ainsi, au moins en partie, une détection d'un carcinome prostatique ou colorectal.

4. Procédé selon la revendication 3, dans lequel la mise en contact ou l'amplification selon c) comprend l'utilisation d'au moins un procédé choisi dans le groupe comprenant: l'utilisation d'une ADN-polymérase résistante à la chaleur comme enzyme d'amplification; l'utilisation d'une polymérase dénuée d'activité de 5'-3'-exonucléase; l'utilisation d'une réaction d'amplification en chaîne par polymérase (ACP); la génération comme produit d'amplification d'une molécule d'acide nucléique portant un marquage détectable.

5. Procédé selon la revendication 3, comprenant en outre dans l'étape d) l'utilisation d'au moins une molécule d'acide nucléique ou d'une molécule d'acide nucléique de peptide comprenant dans chaque cas une séquence contiguë d'au moins 9 nucléotides de long qui est complémentaire de, ou hybride dans des conditions modérément stringentes ou stringentes avec une séquence choisie dans le groupe consistant en SEQ ID NO: 3 à SEQ ID NO: 10, et leurs compléments, tandis que ladite molécule d'acide nucléique ou molécule d'acide nucléique de peptide supprime l'amplification de l'acide nucléique auquel elle est hybridée, et/ou tandis que la détermination dans d) comprend l'hybridation d'au moins une molécule d'acide nucléique ou molécule d'acide nucléique de peptide comprenant dans chaque cas une séquence contiguë d'au moins 9 nucléotides de long qui est complémentaire de, ou hybride dans des conditions modérément stringentes ou stringentes avec une séquence choisie dans le groupe consistant en SEQ ID NO: 3 à SEQ ID NO: 10, et leurs compléments.

6. Procédé selon la revendication 5, comprenant en outre l'extension d'au moins une telle molécule d'acide nucléique hybridée par au moins une base nucléotidique, et/ou dans lequel la mise en contact et/ou l'amplification dans c) comprend l'utilisation d'amorces spécifiques de la méthylation.

7. Procédé pour la détection d'un carcinome prostatique ou colorectal, comprenant:
a) l'extraction ou l'isolement d'une autre manière d'ADN génomique à partir d'un échantillon biologique obtenu à partir du sujet, tandis que
(i) dans le cas d'un cancer de la prostate l'échantillon biologique est choisi dans le groupe consistant en tissu prostatique, plasma sanguin, sérum sanguin, sang total, cellules obtenues à partir du sang prélevé sur le sujet, et urine; et
(ii) dans le cas d'un carcinome du côlon, l'échantillon biologique est choisi dans le groupe consistant en tissu du côlon, plasma sanguin, sérum sanguin, sang total, cellules obtenues à partir du sang prélevé sur le sujet;
b) la digestion de l'ADN génomique de a), ou d'un fragment de celui-ci, avec une ou plusieurs enzymes de restriction sensibles à la méthylation;
c) la mise en contact du produit de digestion par l'enzyme de restriction de l'ADN selon b), avec une enzyme d'amplification et au moins deux amorces convenant pour l'amplification d'une séquence comprenant au moins un dinucléotide CpG d'une séquence choisie parmi SEQ ID NO: 1 à SEQ ID NO: 2; et
d) la détermination, sur la base d'une présence ou d'une absence d'un produit d'amplification, de l'état ou du taux de méthylation d'au moins un dinucléotide CpG d'une séquence choisie dans le groupe consistant en SEQ ID NO: 1 à SEQ ID NO: 2, procurant ainsi, au moins en partie, une détection d'un carcinome prostatique ou colorectal.

8. Procédé selon la revendication 7, dans lequel la présence ou l'absence d'un produit d'amplification est déterminée au moyen de l'hybridation à au moins un acide nucléique ou un acide nucléique de peptide qui est identique à, complémentaire de, ou hybride dans des conditions stringentes ou fortement stringentes à un segment d'au moins 16 bases de long d'une séquence choisie parmi SEQ ID NO: 1 à SEQ ID NO: 2.

9. Utilisation de
a) un acide nucléique dérivé de SEQ ID NO: 1 à SEQ ID NO: 2 génomiques par traitement avec du bisulfite, tandis que ledit acide nucléique comprend au moins une séquence dinucléotidique CpG, TpG ou CpA,
b) une acide nucléique comprenant au moins 16 nucléotides contigus d'une séquence d'ADN génomique choisie dans le groupe consistant en SEQ ID NO: 3 à SEQ ID NO: 10, et des séquences complémentaires de celles-ci, tandis que la séquence de bases contiguës comprend au moins une séquence dinucléotidique CpG, TpG ou CpA, ou
c) un acide nucléique, comprenant au moins 50 nucléotides contigus d'une séquence d'ADN génomique choisie dans le groupe consistant en SEQ ID NO: 3 à SEQ ID NO: 10, et des séquences complémentaires de celles-ci, tandis que la séquence de bases contiguës comprend au moins une séquence dinucléotidique CpG, TpG ou CpA
pour le diagnostic d'un carcinome prostatique ou colorectal par analyse de méthylation d'un échantillon biologique, dans lequel
(i) dans le cas d'un cancer de la prostate l'échantillon biologique est choisi dans le groupe consistant en tissu prostatique, plasma sanguin, sérum sanguin, sang total, cellules obtenues à partir du sang prélevé sur le sujet, et urine; et
(ii) dans le cas d'un carcinome du côlon, l'échantillon biologique est choisi dans le groupe consistant en tissu du côlon, plasma sanguin, sérum sanguin, sang total et cellules obtenues à partir du sang prélevé sur le sujet.

10. Utilisation d'un kit pour la mise en oeuvre du procédé selon la revendication 3, dans laquelle le kit comprend (a) un réactif bisulfite; (b) un récipient convenant pour contenir ledit réactif bisulfite et l'échantillon biologique du patient; (c) au moins un ensemble d'oligonucléotides contenant deux oligonucléotides dont les séquences dans chaque cas sont identiques, sont complémentaires, ou hybrident dans des conditions stringentes ou fortement stringentes à un segment de 9, ou plus préférablement 18, bases de long d'une séquence choisie parmi SEQ ID NO: 3 à SEQ ID NO: 10, ou d'un kit approprié pour la mise en oeuvre du procédé selon la revendication 9 comprenant (a) un réactif enzyme de restriction sensible à la méthylation; (b) un récipient approprié pour contenir ledit réactif et l'échantillon biologique du patient; (c) au moins un ensemble d'oligonucléotides, d'un ou d'une pluralité d'acides nucléiques ou d'acides nucléiques de peptide qui sont identiques, sont complémentaires, ou hybrident dans des conditions stringentes ou fortement stringentes à un segment d'au moins 9 bases de long d'une séquence choisie parmi SEQ ID NO: 1 à SEQ ID NO: 2; et en option (d) des instructions pour l'utilisation et l'interprétation des résultats du kit.
